(19) Europäisches Patentamt
European Patent Office
Office européen des brevets

(11) EP 4 786 461 A1

(12) EUROPEAN PATENT APPLICATION
published in accordance with Art. 153(4) EPC

(43) Date of publication:
05.08.2026 Bulletin 2026/32

(21) Application number: 24870951.1

(22) Date of filing: 27.09.2024

(51) International Patent Classification (IPC):
*C07D 401/04* (2006.01) *A61K 31/496* (2006.01)
*A61P 9/10* (2006.01)

(52) Cooperative Patent Classification (CPC):
A61K 31/496; A61P 9/10; C07D 401/04

(86) International application number:
PCT/CN2024/121712

(87) International publication number:
WO 2025/067414 (03.04.2025 Gazette 2025/14)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 27.09.2023 CN 202311260783
29.02.2024 CN 202410228783
29.04.2024 CN 202410528937

(71) Applicant: Haisco Pharmaceutical Group Co., Ltd.
Shannan, Xizang 856099 (CN)

(72) Inventors:
- ZHANG, Chen
Chengdu, Sichuan 611130 (CN)
- WANG, Jianmin
Chengdu, Sichuan 611130 (CN)
- CHEN, Tengfei
Chengdu, Sichuan 611130 (CN)
- HUANG, Longbin
Chengdu, Sichuan 611130 (CN)
- CHEN, Quanlong
Chengdu, Sichuan 611130 (CN)
- QIAN, Meilin
Chengdu, Sichuan 611130 (CN)
- TANG, Pingming
Chengdu, Sichuan 611130 (CN)
- LI, Yao
Chengdu, Sichuan 611130 (CN)
- YAN, Pangke
Chengdu, Sichuan 611130 (CN)

(74) Representative: AWA Denmark A/S
Strandgade 56
1401 Copenhagen K (DK)

# (54) PYRAZOLE CARBOXYLIC ACID DERIVATIVE AND USE THEREOF IN MEDICINE

(57) A pyrazole carboxylic acid derivative and a use thereof in medicine, in particular, a compound as represented by formula (I) or a stereoisomer, tautomer, deuterated substance, solvate, prodrug, metabolite, pharmaceutically acceptable salt or eutectic crystal thereof, and a use thereof in the preparation of a drug for treating diseases related to sGC.

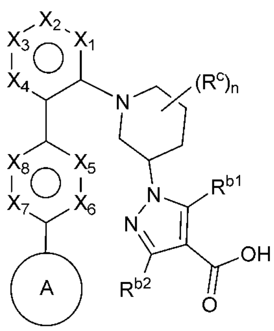

(I)



Processed by Luminess, 75001 PARIS (FR)

## Description

### Technical Field

The present invention belongs to the field of medical chemistry, and relates to a pyrazole carboxylic acid derivative and the use thereof in medicine, in particular to providing a pyrazole carboxylic acid derivative and the use thereof in medicine, in particular to a compound represented by formula (I), or a stereoisomer, tautomer, deuterated compound, solvate, prodrug, metabolite, pharmaceutically acceptable salt, or co-crystal thereof, and the use thereof in the preparation of a medicament for treating a disease associated with sGC.

### Background Art

Nitric oxide (NO) signaling has pleiotropic effects in biology, and plays a critical role in cardiovascular homeostasis. An increase in NO secretion occurs under the influence of mediators, such as norepinephrine (NA), angiotensin, adenosine triphosphate, or bradykinin. NO synthesis is also affected by many physical stimuli.

The mechanism of NO's intracellular action is primarily through the stimulation of the activity of soluble guanylyl cyclase (sGC). sGC is a heme-containing enzyme, which may cause an increase in the level of cyclic 3'-5'-guanosine monophosphate (cGMP) in smooth muscle, leading to vasodilation. The NO/sGC/cGMP regulatory pathway plays an important role in the homeostasis of the cardiovascular and respiratory systems and organs (such as kidneys, brain, and liver). In addition to smooth muscle cells, cGMP influences the functions of fibroblasts, cardiomyocytes, platelets, neurons, and immune cells, and regulates the processes of fibrosis, inflammatory response, and neurotransmission.

sGC modulators and sGC agonists are a class of drugs that can stimulate cGMP formation, which provide tools for studying the regulatory mechanism of sGC and its role in pathological mechanisms. The development of sGC modulators or agonists has made it possible to develop drugs that directly target diseased blood vessels, myocardium, kidneys, and other organs. The development of novel sGC modulators or agonists, and the exploration of the therapeutic value and wider therapeutic potential of sGC targets in different indications have good application prospects.

### Summary of the Invention

The objective of the present invention is to provide a compound having a novel structure and represented by general formula (I), or a stereoisomer, deuterated compound, solvate, prodrug, metabolite, pharmaceutically acceptable salt, or co-crystal thereof, an intermediate thereof, a preparation method therefor, and the use thereof in the preparation of a medicament for treating a disease associated with sGC.

The compound of the present invention exhibits favorable pharmacokinetic properties, improved stability in liver microsomes, oral bioavailability, and good safety profile.

The present invention provides a compound represented by general formula (I), or a stereoisomer, tautomer, deuterated compound, solvate, prodrug, metabolite, pharmaceutically acceptable salt, or co-crystal thereof,

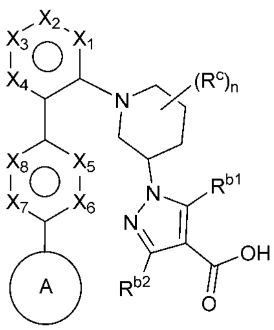

(I).

In some embodiments, the compound represented by general formula (I) is selected from a compound represented by general formula (II-a), (II-b), (II-c), (II-d), (II-e), (II-f), (II-g), (II-h), or (II-i),

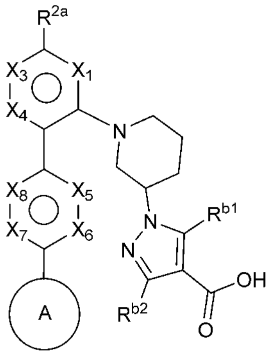
(II-a),

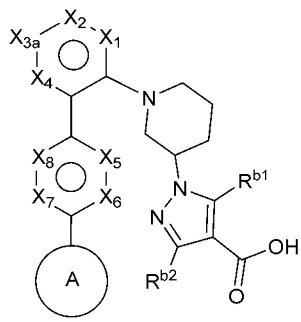
(II-b),

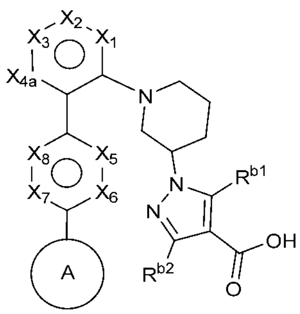
(II-c),

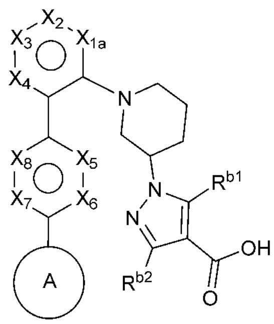
(II-d),

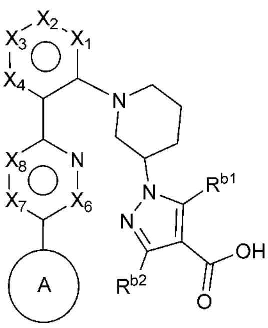
(II-e),

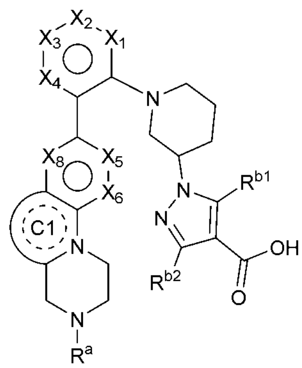
(II-f),

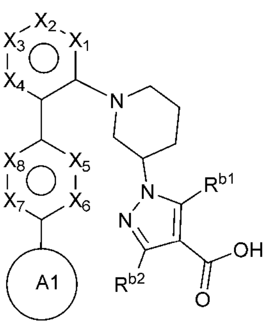
(II-g),

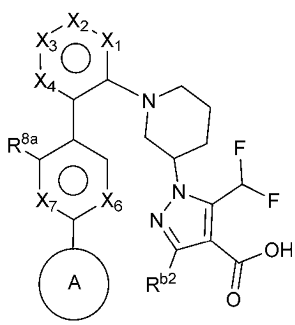
(II-h),

or

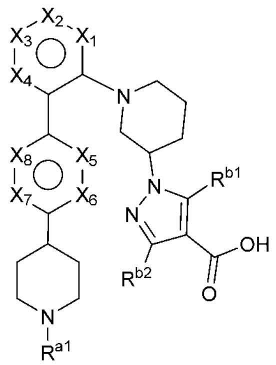

(II-i).

In some embodiments, 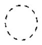 represents an aromatic ring or a non-aromatic ring.

In some embodiments, C1 is a 5- to 6-membered heterocycle.

In some embodiments,

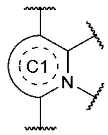

is selected from

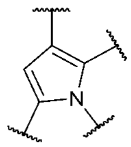

,

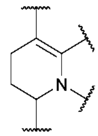

,

or

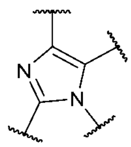

.

In some embodiments, $X_1$ is selected from $CR^1$ or N.

In some embodiments, $X_2$ is selected from $CR^2$ or N.

In some embodiments, $X_3$ is selected from $CR^3$ or N.

In some embodiments, $X_4$ is selected from $CR^4$ or N.

In some embodiments, $X_5$ is selected from $CR^5$ or N.

In some embodiments, $X_6$ is selected from $CR^6$ or N.

In some embodiments, $X_7$ is selected from $CR^7$ or N.

In some embodiments, $X_8$ is selected from $CR^8$ or N.

In some embodiments, $X_1$ is selected from $CR^1$ or N, $X_2$ is selected from $CR^2$ or N, $X_3$ is selected from $CR^3$ or N, $X_4$ is selected from $CR^4$ or N, $X_5$ is selected from $CR^5$ or N, $X_6$ is selected from $CR^6$ or N, $X_7$ is selected from $CR^7$ or N, and $X_8$ is selected from $CR^8$ or N.

In some embodiments,

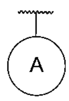

is selected from $C_{3-12}$ carbocyclyl or 4- to 12-membered heterocyclyl, wherein the carbocyclyl or heterocyclyl is optionally substituted with 1 to 4 $R^a$.

In some embodiments,

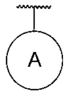

is selected from the following groups optionally substituted with 1 to 4 $R^a$: $C_{3-6}$ monocycloalkyl, $C_{3-6}$ monocycloalkenyl, $C_{5-11}$ spirocycloalkyl, $C_{4-10}$ fused cycloalkyl, $C_{5-10}$ bridged cycloalkyl, 4- to 6-membered monocyclic heterocycloalkyl, 4- to 6-membered monocyclic heterocycloalkenyl, $C_{5-11}$ spiroheterocycloalkyl, $C_{4-10}$ fused heterocycloalkyl, or $C_{5-10}$ bridged heterocycloalkyl.

In some embodiments,

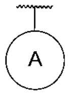

is selected from the following groups optionally substituted with 1 to 4 $R^a$: cyclopropyl, cyclobutyl, cyclopentyl, cyclohexyl, cyclohexenyl, azetidinyl, pyrrolidinyl, piperidinyl, azacyclohexenyl, piperazinyl, morpholinyl, oxetanyl, tetrahydrofuranyl, oxanyl,

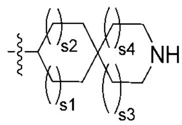, 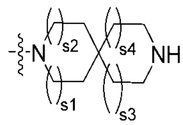,

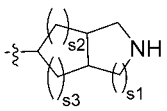, 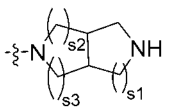, 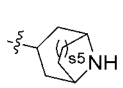, 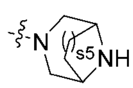, 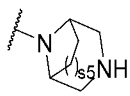, 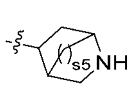,

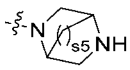, 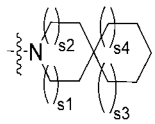, 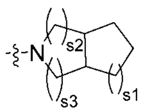, 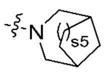, 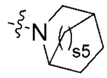, 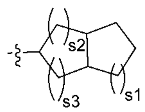, 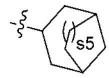,

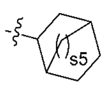, 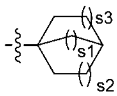, 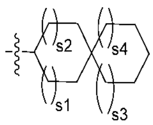, 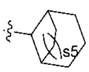, 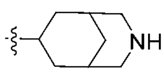, 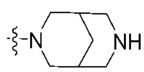, 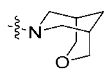,

or

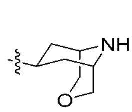,

wherein s1, s2, s3, s4, and s5 are each independently selected from 0 or 1.

In some embodiments,

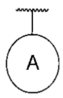

is selected from the following groups optionally substituted with 1 to 4 $R^a$: cyclopropyl, cyclobutyl, cyclopentyl, cyclohexyl,

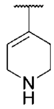,

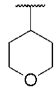, 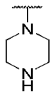, 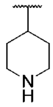, 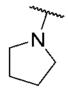, 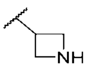, 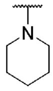, 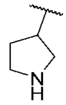, 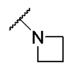, 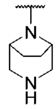, 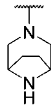, 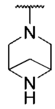, 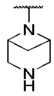, 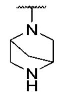, 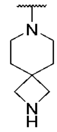,

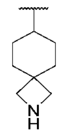, 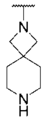, 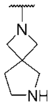, 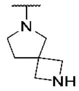, 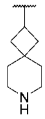, 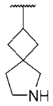, 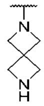, or 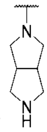.

In some embodiments, $R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, - O-$C_{3-6}$ carbocyclyl, -O-3- to 7-membered heterocyclyl, -NH-$C_{3-6}$ carbocyclyl, - NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl, or heterocyclyl is optionally substituted with 1 to 4 $R^k$.

In some embodiments, $R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, - O-$C_{3-6}$ carbocyclyl, -O-3- to 6-membered heterocyclyl, -NH-$C_{3-6}$ carbocyclyl, - NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl, or heterocyclyl is optionally substituted with 1 to 4 $R^k$.

In some embodiments, $R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, F, Cl, Br, I, OH, CN, $NH_2$, $NHCH_3$, $NHCH_3$,

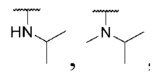

or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl.

In some embodiments, $R^1$, $R^2$, $R^3$, $R^4$, $R^5$, $R^6$, $R^7$, and $R^8$ are each independently selected from H, deuterium, F, Cl, Br, I, OH, CN, $NH_2$, $NHCH_3$, $NHCH_3$,

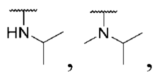

$CH_2F$, $CHF_2$, $CF_3$, methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl.

In some embodiments, $R^a$ is selected from H, deuterium, F, Cl, Br, I, OH, CN, $NH_2$, $NHCH_3$, $NHCH_3$,

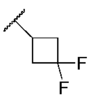, 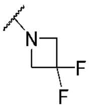, 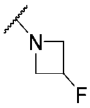, 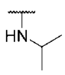, 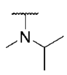, 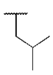, 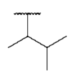,

methyl, methyl, ethyl, propyl, isopropyl, butyl, cyclopropyl, or $-CH_2$-cyclopropyl.

In some embodiments, $R^a$ is selected from H, deuterium, F, Cl, Br, I, OH, CN, $NH_2$, $NHCH_3$, $NHCH_3$,

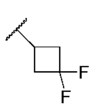, 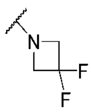, 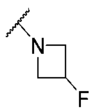, 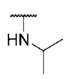, 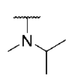, 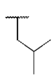, 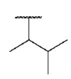,

methyl, methyl, ethyl, propyl, isopropyl, butyl, or cyclopropyl.

In some embodiments, $R^7$ and $R^a$, together with the atoms to which they are attached, form a 4- to 8-membered carbocycle or a 4- to 8-membered heterocycle, wherein the carbocycle or heterocycle is optionally substituted with 1 to 4 $R^k$.

In some embodiments, $R^7$ and $R^a$, together with the atoms to which they are attached, form a 4- to 6-membered carbocycle or a 4- to 6-membered heterocycle, wherein the carbocycle or heterocycle is optionally substituted with 1 to 4 $R^k$.

In some embodiments, $R^7$ and $R^a$, together with the atoms to which they are attached, form a 5- to 6-membered carbocycle or a 5- to 6-membered heterocycle, wherein the carbocycle or heterocycle is optionally substituted with 1 to 4 $R^k$.

In some embodiments, $R^7$ and $R^a$, together with the atoms to which they are attached, form 5- to 6-membered heterocyclyl.

In some embodiments, each $R^k$ is independently selected from deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}\ alkyl)_2$, $-O-C_{3-6}$ carbocyclyl, -O-3-to 7-membered heterocyclyl, $-NH-C_{3-6}$ carbocyclyl, -NH-3- to 7-membered heterocyclyl, $-C_{0-4}$ alkylene-$C_{3-6}$ carbocyclyl, or $-C_{0-4}$ alkylene-3- to 7-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$ alkoxy.

In some embodiments, each $R^k$ is independently selected from deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}\ alkyl)_2$, $-O-C_{3-6}$ carbocyclyl, -O-3-to 6-membered heterocyclyl, $-NH-C_{3-6}$ carbocyclyl, -NH-3- to 6-membered heterocyclyl, $-C_{0-2}$ alkylene-$C_{3-6}$ carbocyclyl, or $-C_{0-2}$ alkylene-3- to 6-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$ alkoxy.

In some embodiments, each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy.

In some embodiments, $R^{2a}$ is selected from H, deuterium, F, Br, $CHF_2$, methyl, ethyl, isopropyl, or cyclopropyl.

In some embodiments, $X^{1a}$ is selected from N or $CR^{1a}$.

In some embodiments, $X^{3a}$ is selected from N or $CR^{3a}$.

In some embodiments, $X^{4a}$ is selected from N or $CR^{4a}$.

In some embodiments, $R^{1a}$, $R^{3a}$, and $R^{4a}$ are each independently selected from deuterium, F, Cl, Br, $CHF_2$, methyl, ethyl, isopropyl, or cyclopropyl.

In some embodiments,

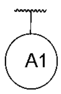

is selected from the following groups optionally substituted with 1 to 4 $R^a$:

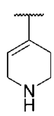, 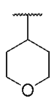, 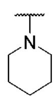, 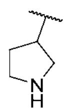, 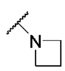, 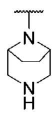, 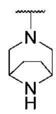, 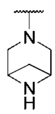, 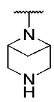, 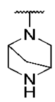,

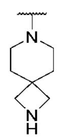, 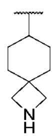, 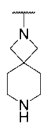, 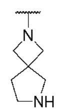, 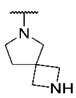, 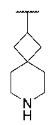, 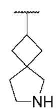, 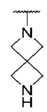, or 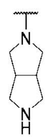.

In some embodiments, $R^{8a}$ is selected from deuterium, F, Br, $CF_3$, $CHF_2$, or cyclopropyl.

In some embodiments, each $R^{b1}$ is independently selected from H, deuterium, F, Cl, Br, $CH_2F$, $CHF_2$, $CF_3$, methyl, ethyl, propyl, isopropyl, or cyclopropyl.

In some embodiments, $R^{b1}$ or $R^{b2}$ is selected from H, deuterium, F, $CH_2F$, $CF_3$, or $CHF_2$.

In some embodiments, $R^{b1}$ is selected from $CF_3$ or $CHF_2$.

In some embodiments, $R^{b2}$ is H.

In some embodiments, n is selected from 0, 1, 2, 3, or 4.

Optionally, 1) $R^2$ is not Cl or $CF_3$;

or 2) at least one of $X_1$, $X_2$, $X_3$, $X_4$, $X_5$, and $X_8$ is N;
or 3) at least one of $R^1$, $R^3$, and $R^4$ is not H;
or 4)

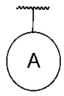

is not selected from

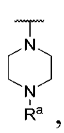 , 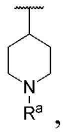 , 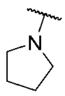 , 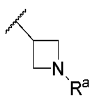 , or 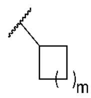 ,

wherein m is selected from 0, 1, 2, 3, or 4;
or 5) $R^7$ and $R^a$, together with the atoms to which they are attached, form a 4-to 8-membered carbocycle or a 4- to 8-membered heterocycle, wherein the carbocycle or heterocycle is optionally substituted with 1 to 4 $R^k$;
or 6) when $R^{b1}$ is $CHF_2$, $R^{b2}$ is H,

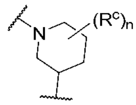

is

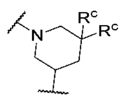 ,

each $R^c$ is independently selected from H or halogen,

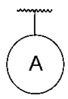

is selected from

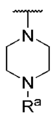 , 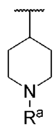 , 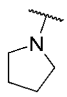 ,

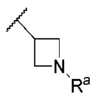 , or 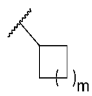 ,

wherein m is selected from 0, 1, 2, 3, or 4, $X_1$ is CH, $X_3$ is CH, $X_4$ is CH, $X_2$ is $CR^2$, wherein $R^2$ is selected from Cl or $CF_3$, $X_6$ or $X_7$ is selected from CH, CF, or N, $X_5$ is $CR^5$, and $X_8$ is $CR^8$, at least one of $R^5$ and $R^8$ is not H or $C_{1-4}$ alkyl.

Optionally, 1) $R^2$ is not Cl or $CF_3$;

or 2) at least one of $X_1$, $X_2$, $X_3$, $X_4$, $X_5$, and $X_8$ is N;
or 3) at least one of $R^1$, $R^3$, and $R^4$ is not H;
or 4)

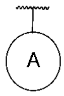

is not selected from

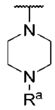, 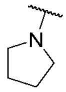, 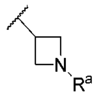, or 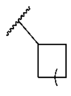,

wherein m is selected from 0, 1, 2, 3, or 4;
or 5) $R^7$ and $R^a$, together with the atoms to which they are attached, form a 4-to 8-membered carbocycle or a 4- to 8-membered heterocycle, wherein the carbocycle or heterocycle is optionally substituted with 1 to 4 $R^k$;
or 6) when $R^{b1}$ is $CHF_2$, $R^{b2}$ is H,

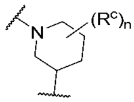

is

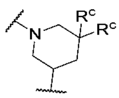,

each $R^c$ is independently selected from H or halogen,

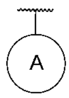

is selected from

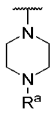, 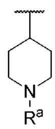, 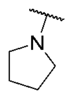,

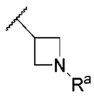, or 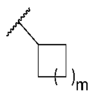,

wherein m is selected from 0, 1, 2, 3, or 4, $X_1$ is CH, $X_3$ is CH, $X_4$ is CH, $X_2$ is $CR^2$, wherein $R^2$ is selected from Cl or $CF_3$, $X_6$ or $X_7$ is selected from CH, CF, or N, $X_5$ is $CR^5$, and $X_8$ is $CR^8$, at least one of $R^5$ and $R^8$ is not H or $C_{1-4}$ alkyl;
or 7) when

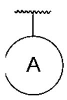

is

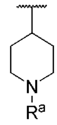 ,

$R^a$ is not $C_{1-4}$ alkylcarbonyl or optionally substituted $C_{3-6}$ cycloalkyl.

As a first embodiment of the present invention, in the compound represented by general formula (I) described above, or the stereoisomer, tautomer, deuterated compound, solvate, prodrug, metabolite, pharmaceutically acceptable salt, or co-crystal thereof,

$X_1$ is selected from $CR^1$ or N, $X_2$ is selected from $CR^2$ or N, $X_3$ is selected from $CR^3$ or N, $X_4$ is selected from $CR^4$ or N, $X_5$ is selected from $CR^5$ or N, $X_6$ is selected from $CR^6$ or N, $X_7$ is selected from $CR^7$ or N, and $X_8$ is selected from $CR^8$ or N;

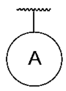

is selected from $C_{3-12}$ carbocyclyl or 4- to 12-membered heterocyclyl, wherein the carbocyclyl or heterocyclyl is optionally substituted with 1 to 4 $R^a$;

$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl, or heterocyclyl is optionally substituted with 1 to 4 $R^k$;

or $R^7$ and $R^a$, together with the atoms to which they are attached, form a 4- to 8-membered carbocycle or a 4- to 8-membered heterocycle, wherein the carbocycle or heterocycle is optionally substituted with 1 to 4 $R^k$;

each $R^k$ is independently selected from deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}$ alkyl$)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$ alkoxy; and

n is selected from 0, 1, 2, 3, or 4;

provided that

1) $R^2$ is not Cl or $CF_3$;

or 2) at least one of $X_1$, $X_2$, $X_3$, $X_4$, $X_5$, and $X_8$ is N;

or 3) at least one of $R^1$, $R^3$, and $R^4$ is not H;

or 4)

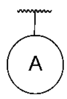

is not selected from

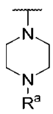 , 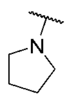 , 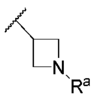 , or 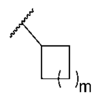 ,

wherein m is selected from 0, 1, 2, 3, or 4;

or 5) $R^7$ and $R^a$, together with the atoms to which they are attached, form a 4-to 8-membered carbocycle or a 4- to 8-membered heterocycle, wherein the carbocycle or heterocycle is optionally substituted with 1 to 4 $R^k$;

or 6) when $R^{b1}$ is $CHF_2$, $R^{b2}$ is H,

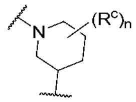

is

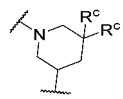,

each $R^c$ is independently selected from H or halogen, is selected from

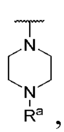, 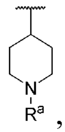, 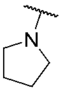,

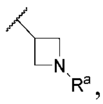, or 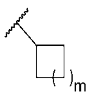,

wherein m is selected from 0, 1, 2, 3, or 4, $X_1$ is CH, $X_3$ is CH, $X_4$ is CH, $X_2$ is $CR^2$, wherein $R^2$ is selected from Cl or $CF_3$, $X_6$ or $X_7$ is selected from CH, CF, or N, $X_5$ is $CR^5$, and $X_8$ is $CR^8$, at least one of $R^5$ and $R^8$ is not H or $C_{1-4}$ alkyl; or 7) when

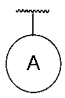 is 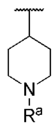,

$R^a$ is not $C_{1-4}$ alkylcarbonyl or optionally substituted $C_{3-6}$ cycloalkyl.

As a second embodiment of the present invention, in the compound represented by general formula (I) described above, or the stereoisomer, tautomer, deuterated compound, solvate, prodrug, metabolite, pharmaceutically acceptable salt, or co-crystal thereof,

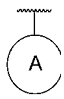

is selected from the following groups optionally substituted with 1 to 4 $R^a$: $C_{3-6}$ monocycloalkyl, $C_{3-6}$ monocycloalkenyl, $C_{5-11}$ spirocycloalkyl, $C_{4-10}$ fused cycloalkyl, $C_{5-10}$ bridged cycloalkyl, 4- to 6-membered monocyclic heterocycloalkyl, 4- to 6-membered monocyclic heterocycloalkenyl, $C_{5-11}$ spiroheterocycloalkyl, $C_{4-10}$ fused heterocycloalkyl, or $C_{5-10}$ bridged heterocycloalkyl;

$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl, or heterocyclyl is optionally substituted with 1 to 4 $R^k$;

or $R^7$ and $R^a$, together with the atoms to which they are attached, form a 4- to 6-membered carbocycle or a 4- to 6-membered heterocycle, wherein the carbocycle or heterocycle is optionally substituted with 1 to 4 $R^k$;

each $R^k$ is independently selected from deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$

alkoxy; and
the remaining definitions are the same as those in the first embodiment of the present invention.

As a third embodiment of the present invention, in the compound represented by general formula (I) described above, or the stereoisomer, tautomer, deuterated compound, solvate, prodrug, metabolite, pharmaceutically acceptable salt, or co-crystal thereof,

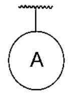

is selected from the following groups optionally substituted with 1 to 4 $R^a$: cyclopropyl, cyclobutyl, cyclopentyl, cyclohexyl, cyclohexenyl, azetidinyl, pyrrolidinyl, piperidinyl, azacyclohexenyl, piperazinyl, morpholinyl, oxetanyl, tetrahydrofuranyl, oxanyl,

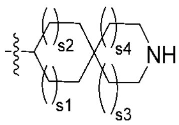, 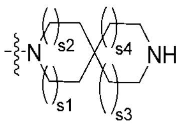, 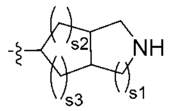,

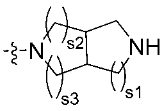, 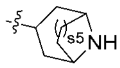, 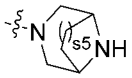, 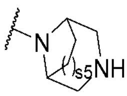, 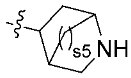, 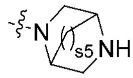,

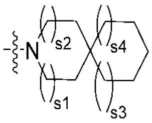, 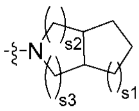, 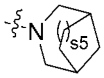, 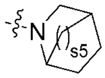, 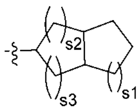, 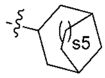, 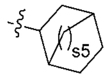,

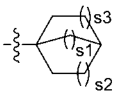, 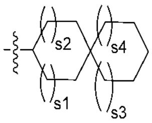, 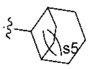, 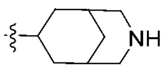, 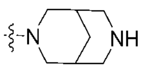, 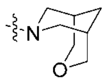,

or

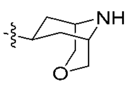;

s1, s2, s3, s4, and s5 are each independently selected from 0 or 1;
$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, F, Cl, Br, I, OH, CN, $NH_2$, $NHCH_3$, $NHCH_3$,

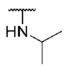,

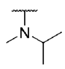,

or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;
or $R^7$ and $R^a$, together with the atoms to which they are attached, form a 5- to 6-membered carbocycle or a 5- to 6-membered heterocycle, wherein the carbocycle or heterocycle is optionally substituted with 1 to 4 $R^k$;
each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy; and
the remaining definitions are the same as those in the first or second embodiment of the present invention.

As a fourth embodiment of the present invention, in the compound represented by general formula (I) described above, or the stereoisomer, tautomer, deuterated compound, solvate, prodrug, metabolite, pharmaceutically acceptable salt, or co-crystal thereof,

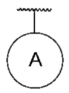

is selected from the following groups optionally substituted with 1 to 4 $R^a$: cyclopropyl, cyclobutyl, cyclopentyl, cyclohexyl,

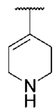, 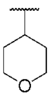, 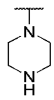, 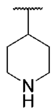, 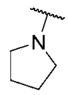,

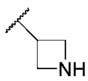, 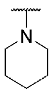, 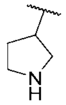, 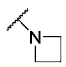, 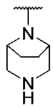, 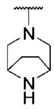, 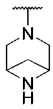, 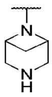, 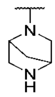, 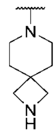, 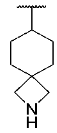, 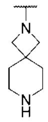, 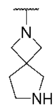, 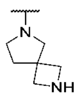,

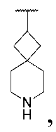, 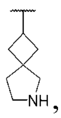, 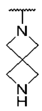, or 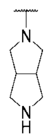;

or $R^7$ and $R^a$, together with the atoms to which they are attached, form 5- to 6-membered heterocyclyl; and
the remaining definitions are the same as those in the first, second, or third embodiment of the present invention.

As a fifth embodiment of the present invention, in the compound represented by general formula (II-a), (II-b), (II-c), (II-d), (II-e), (II-f), (II-g), (II-h), or (II-i) described below, or the stereoisomer, tautomer, deuterated compound, solvate, prodrug, metabolite, pharmaceutically acceptable salt, or co-crystal thereof,

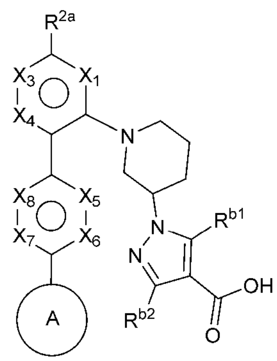

(II-a),

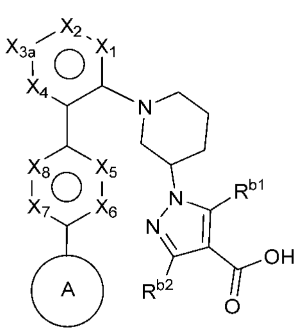

(II-b),

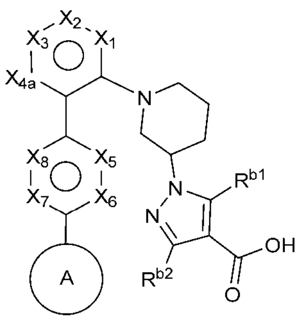

(II-c),

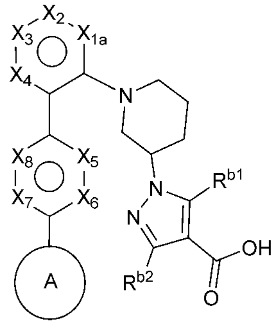

(II-d),

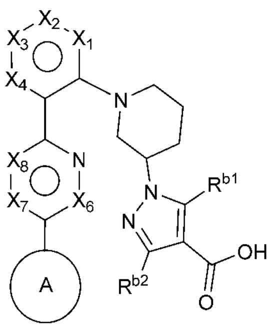

(II-e),

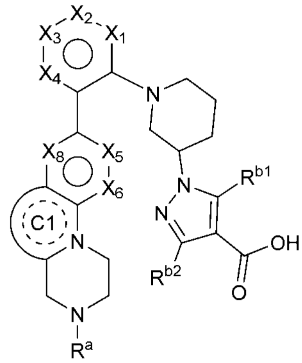

(II-f),

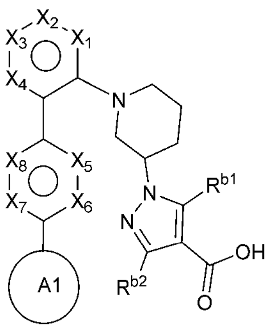

(II-g),

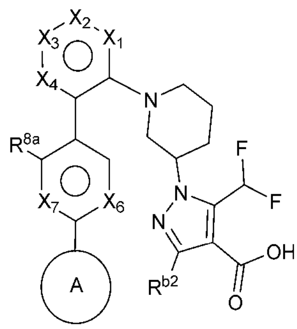

(II-h),

or

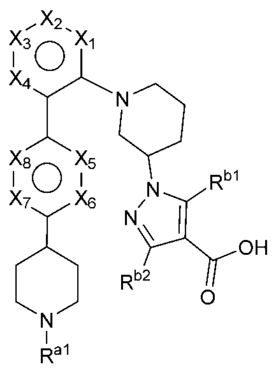

(II-i);

$R^{2a}$ is selected from H, deuterium, F, Br, $CHF_2$, methyl, ethyl, isopropyl, or cyclopropyl;
$X^{1a}$ is selected from N or $CR^{1a}$;
$X^{3a}$ is selected from N or $CR^{3a}$;
$X^{4a}$ is selected from N or $CR^{4a}$;
$R^{1a}$, $R^{3a}$, and $R^{4a}$ are each independently selected from deuterium, F, Cl, Br, $CHF_2$, methyl, ethyl, isopropyl, or

cyclopropyl;

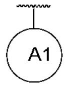

is selected from the following groups optionally substituted with 1 to 4 $R^a$:

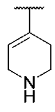, 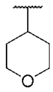, 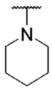, 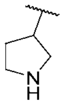, 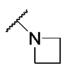, 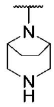, 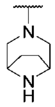, 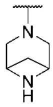, 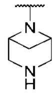, 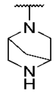, 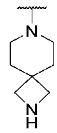, 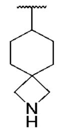, 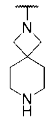, 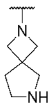,

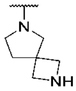, 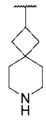, 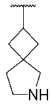, 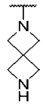, or 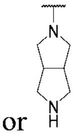;

$R^a$ is selected from H, deuterium, F, Cl, Br, I, OH, CN, $NH_2$, $NHCH_3$, $NHCH_3$,

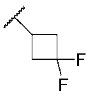, 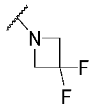, 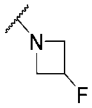, 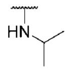, 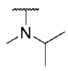, 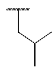, 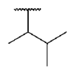,

methyl, methyl, ethyl, propyl, isopropyl, butyl, cyclopropyl, or -$CH_2$-cyclopropyl;

$R^{a1}$ is selected from H, deuterium, F, Cl, Br, I, OH, CN, $NH_2$, $NHCH_3$, $NHCH_3$,

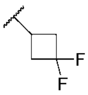, 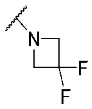, 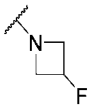, 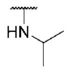, 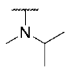, 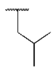, 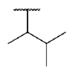,

methyl, methyl, ethyl, propyl, isopropyl, butyl, or cyclopropyl;

$R^{8a}$ is selected from deuterium, F, Br, $CF_3$, $CHF_2$, or cyclopropyl;

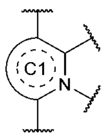

is selected from

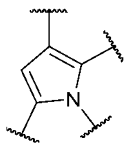, 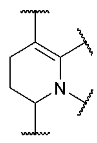, or 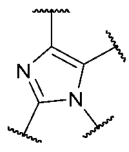;

and

the remaining definitions are the same as those in the first, second, third, or fourth embodiment of the present invention.

As a sixth embodiment of the present invention, in the compound represented by general formula (III-a) or (III-f) described below, or the stereoisomer, tautomer, deuterated compound, solvate, prodrug, metabolite, pharmaceutically acceptable salt, or co-crystal thereof,

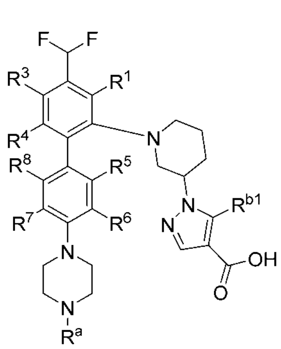

(III-a) or

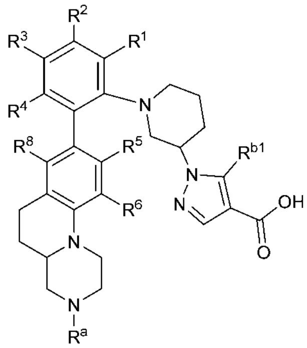

(III-f);

$R^1$, $R^2$, $R^3$, $R^4$, $R^5$, $R^6$, $R^7$, and $R^8$ are each independently selected from H, deuterium, F, Cl, Br, I, OH, CN, $NH_2$, $NHCH_3$, $NHCH_3$,

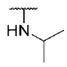, 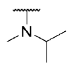,

, $CH_2F$, $CHF_2$, $CF_3$, methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;

$R^a$ is selected from H, deuterium, F, Cl, Br, I, OH, CN, $NH_2$, $NHCH_3$, $NHCH_3$,

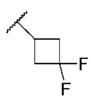, 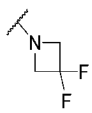, 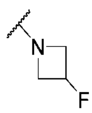, 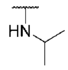, 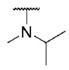, 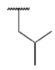, 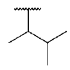,

methyl, methyl, ethyl, propyl, isopropyl, butyl, cyclopropyl, or $-CH_2$-cyclopropyl; and

each $R^{b1}$ is independently selected from H, deuterium, F, Cl, Br, $CH_2F$, $CHF_2$, $CF_3$, methyl, ethyl, propyl, isopropyl, or cyclopropyl.

The present invention relates to a compound as described below, or a stereoisomer, tautomer, deuterated compound, solvate, prodrug, metabolite, pharmaceutically acceptable salt, or co-crystal thereof, wherein the compound is selected from one of the structures in Table E-1 below:

Table E-1

| | | | | | |
|---|---|---|---|---|---|
| 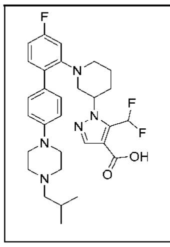 | 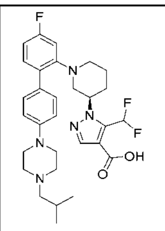 | 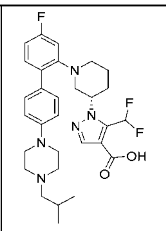 | 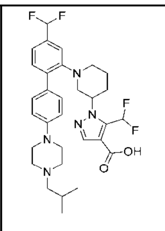 | 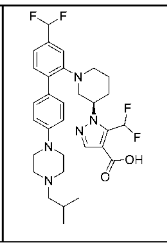 | 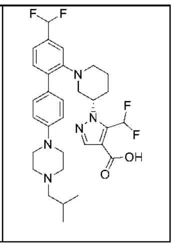 |

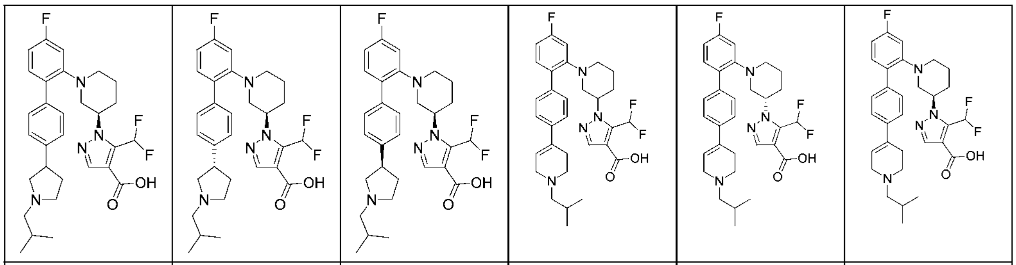

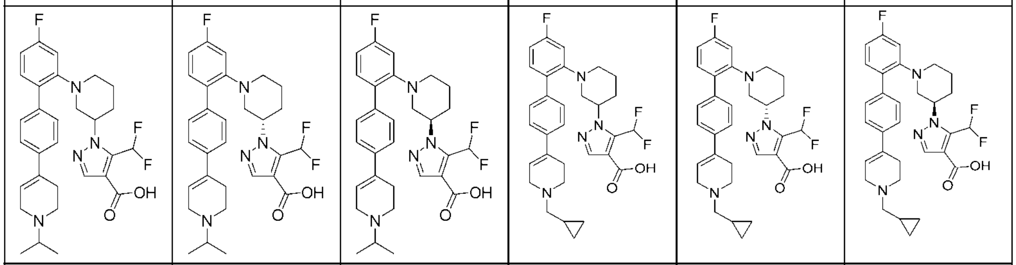

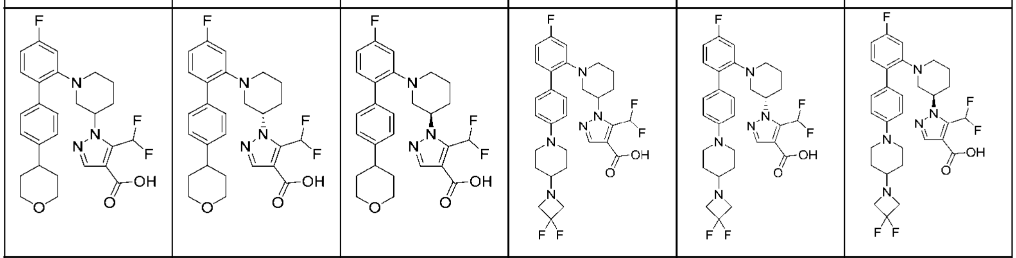

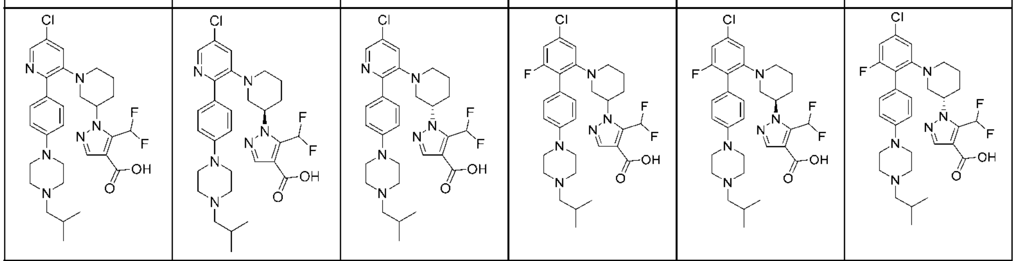

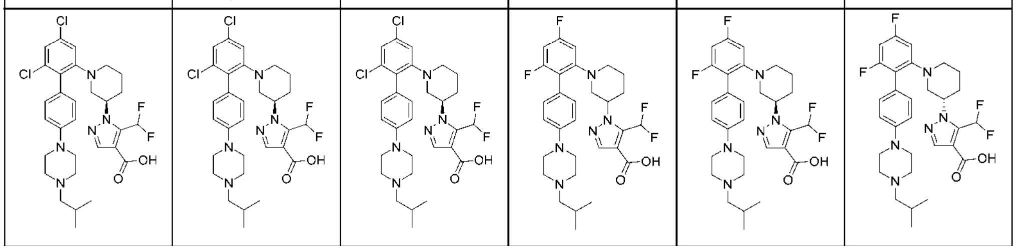

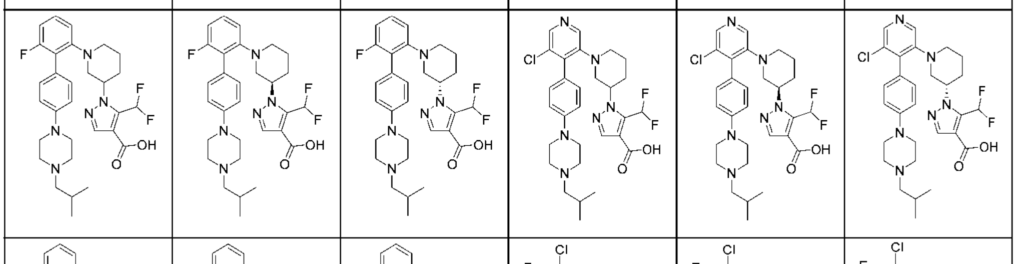

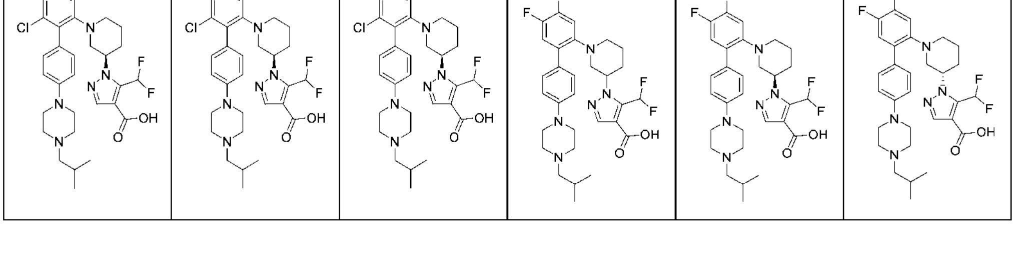

| | | | | | |
|---|---|---|---|---|---|
| 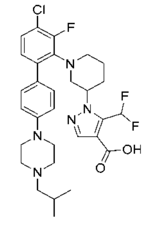 | 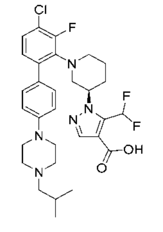 | 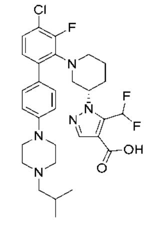 | 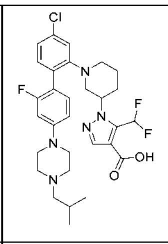 | 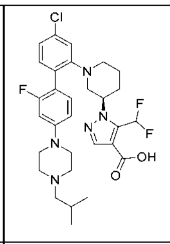 | 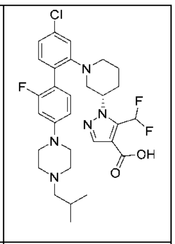 |
| 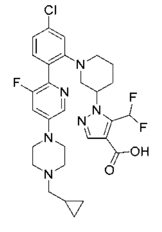 | 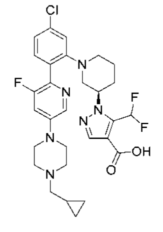 | 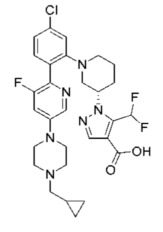 | 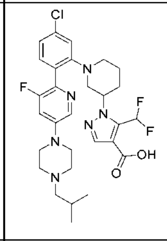 | 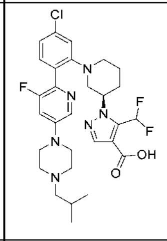 | 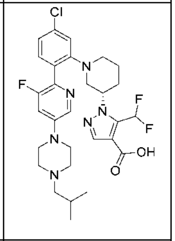 |
| 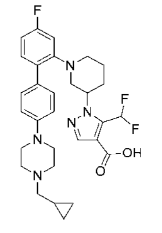 | 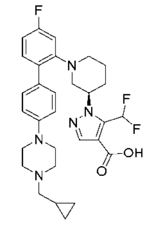 | 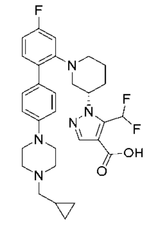 | 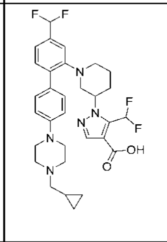 | 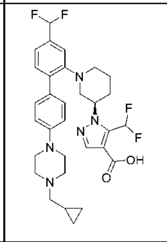 | 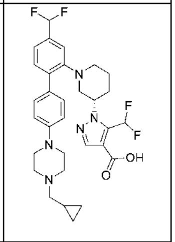 |
| 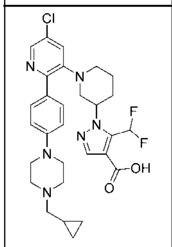 | 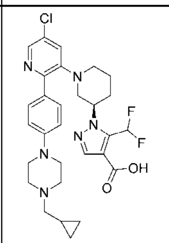 | 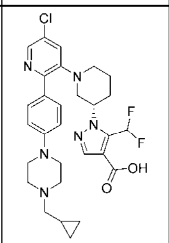 | 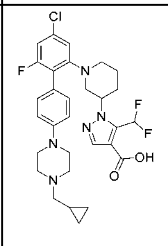 | 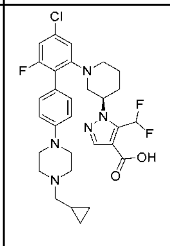 | 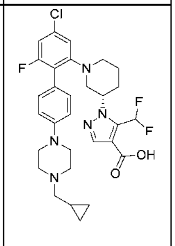 |
| 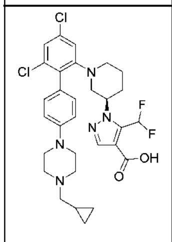 | 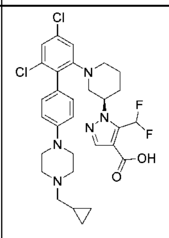 | 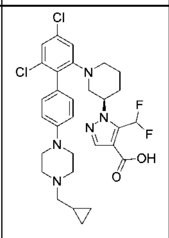 | 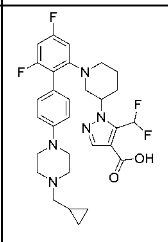 | 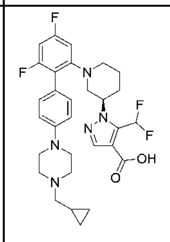 | 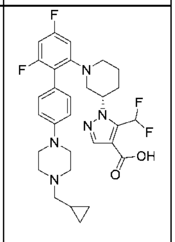 |
| 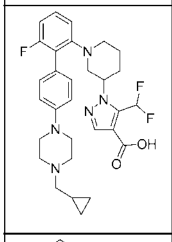 | 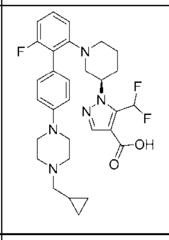 | 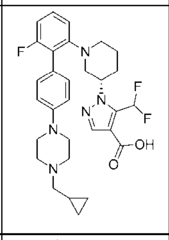 | 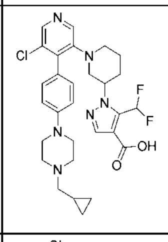 | 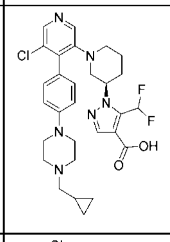 | 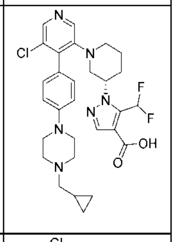 |
| 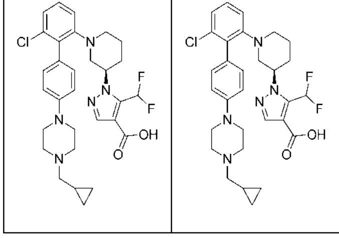 | | 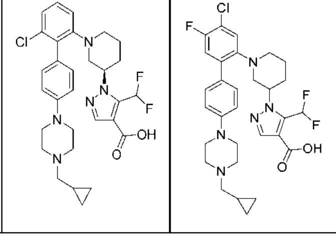 | | 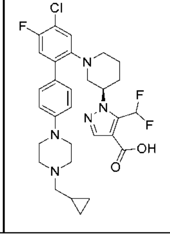 | 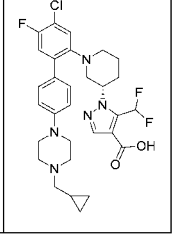 |

| | | | | | |
|---|---|---|---|---|---|
| 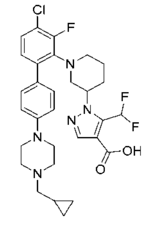 | 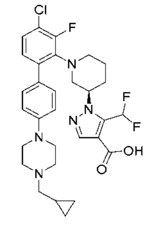 | 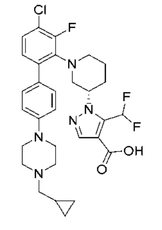 | 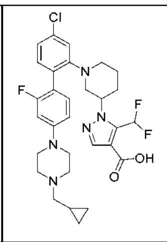 | 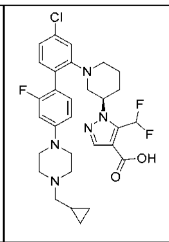 | 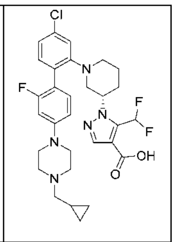 |
| 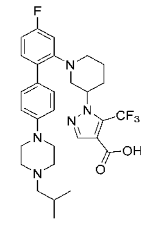 | 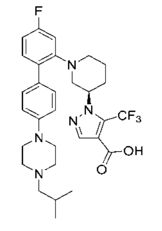 | 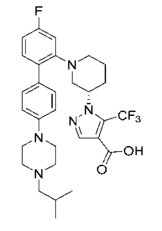 | 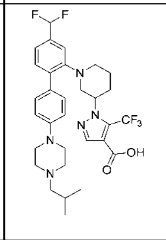 | 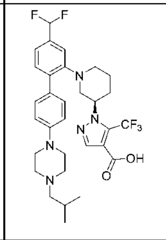 | 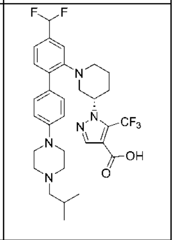 |
| 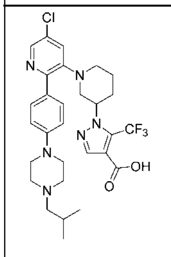 | 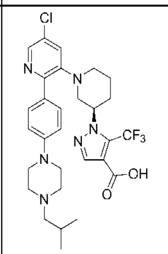 | 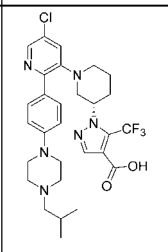 | 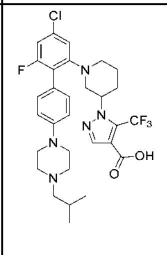 | 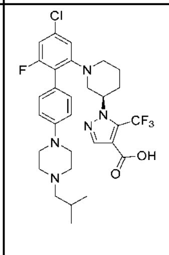 | 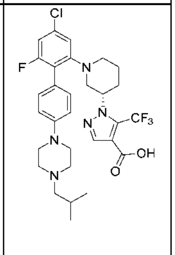 |
| 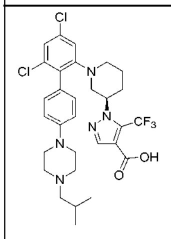 | 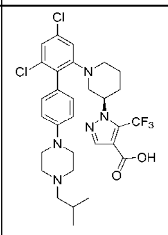 | 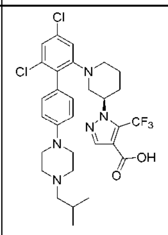 | 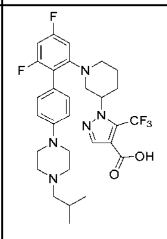 | 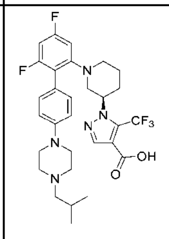 | 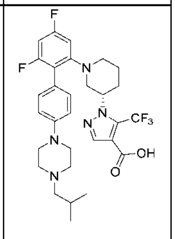 |
| 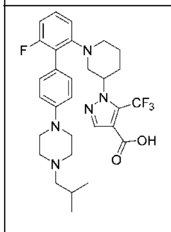 | 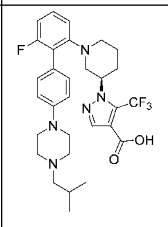 | 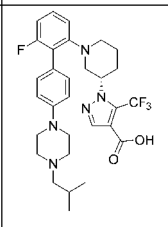 | 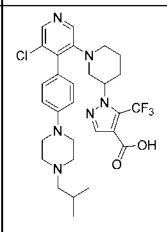 | 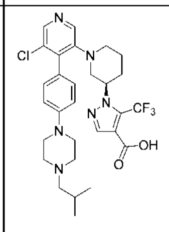 | 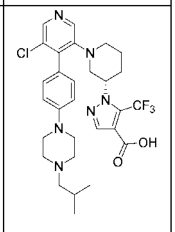 |
| 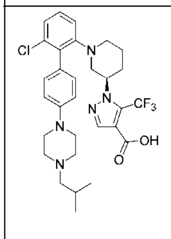 | 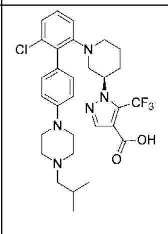 | 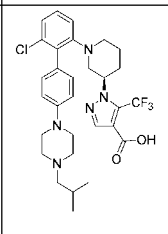 | 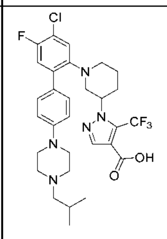 | 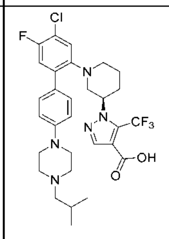 | 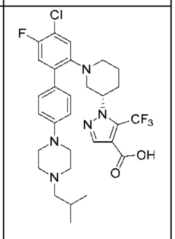 |
| 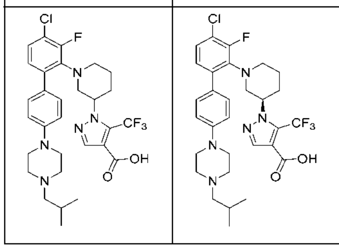 | | 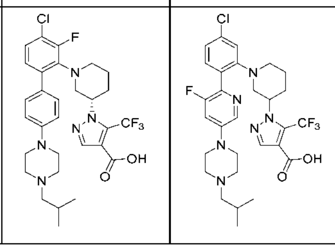 | | 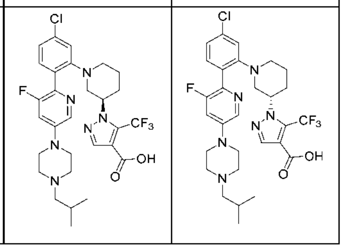 | |

| | | | | | |
|---|---|---|---|---|---|
| 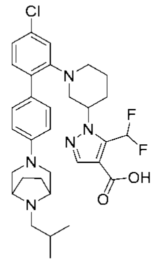 | 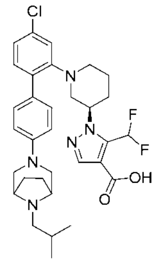 | 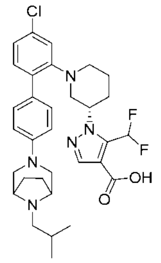 | 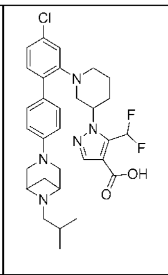 | 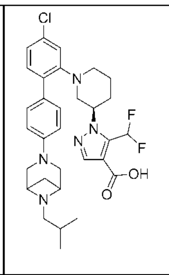 | 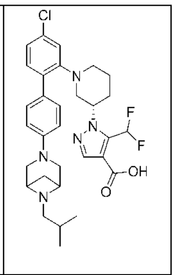 |
| 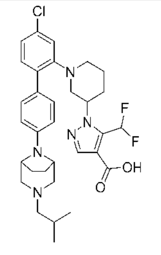 | 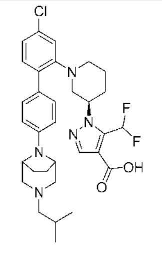 | 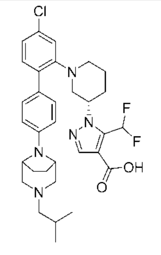 | 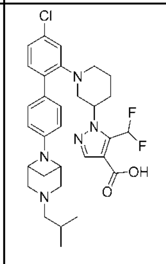 | 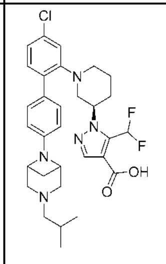 | 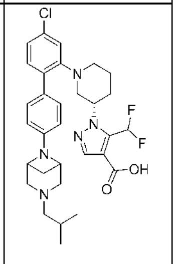 |
| 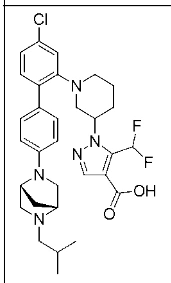 | 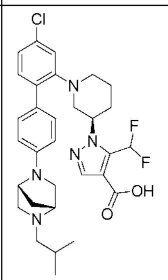 | 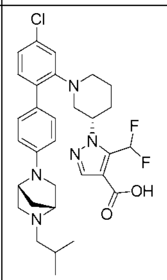 | 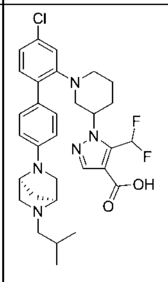 | 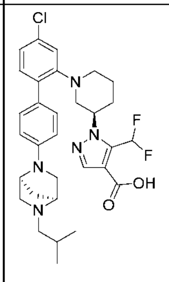 | 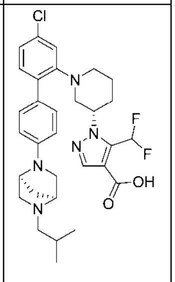 |
| 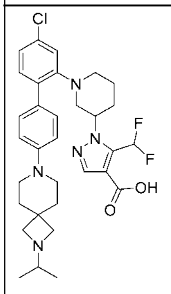 | 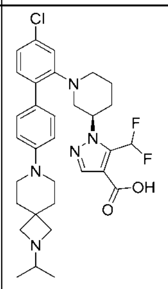 | 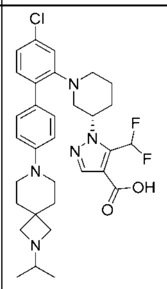 | 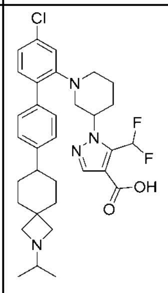 | 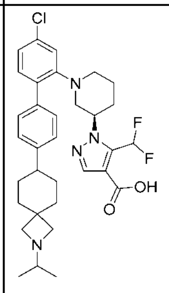 | 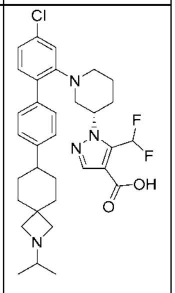 |
| 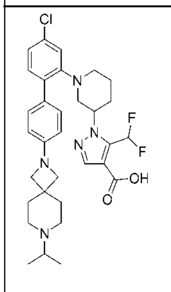 | 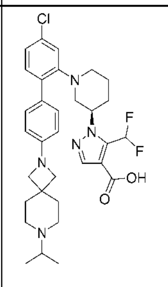 | 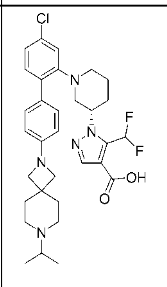 | 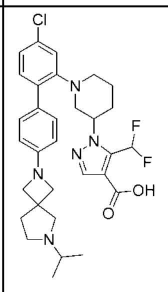 | 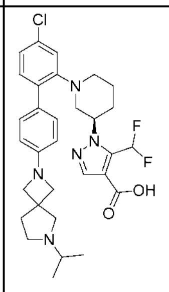 | 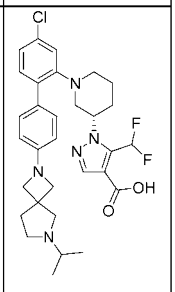 |
| 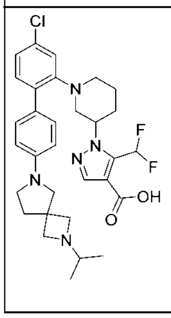 | 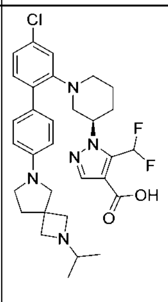 | 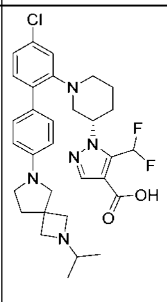 | 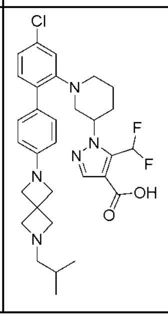 | 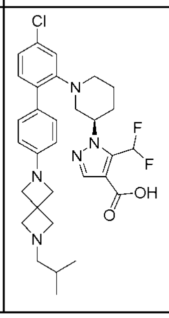 | 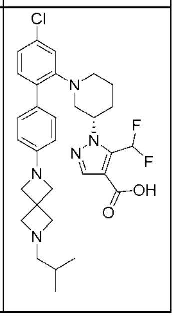 |

| | | | | | |
|---|---|---|---|---|---|
| 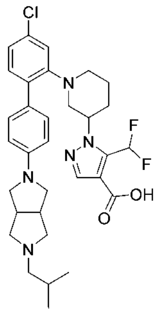 | 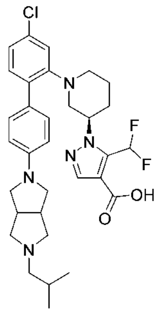 | 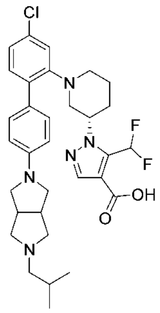 | 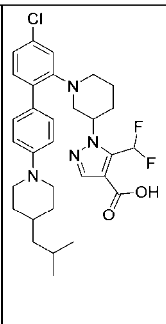 | 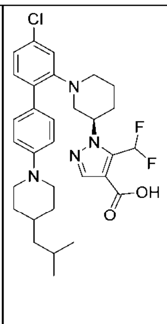 | 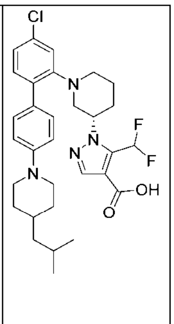 |
| 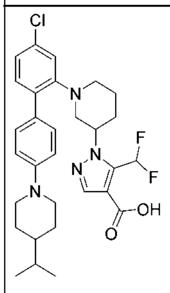 | 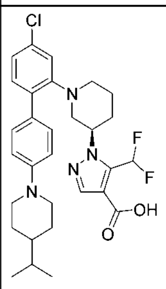 | 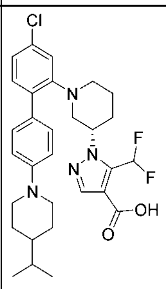 | 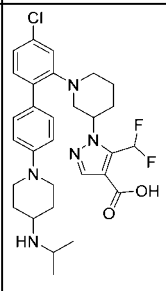 | 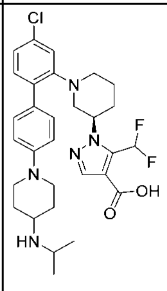 | 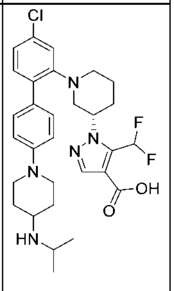 |
| 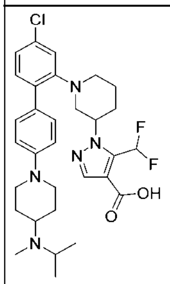 | 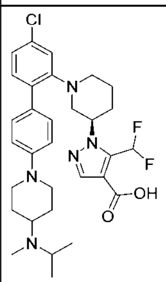 | 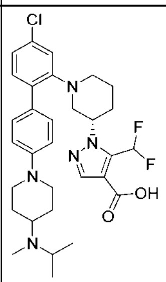 | 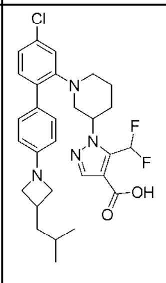 | 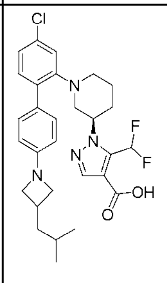 | 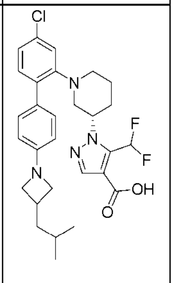 |
| 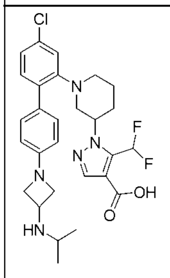 | 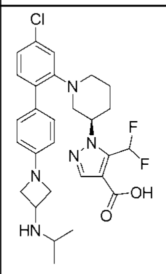 | 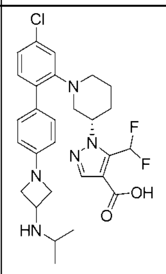 | 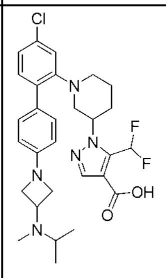 | 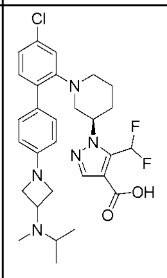 | 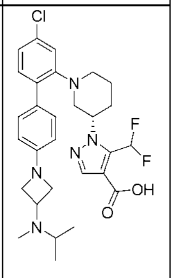 |
| 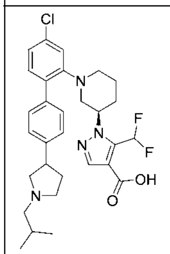 | 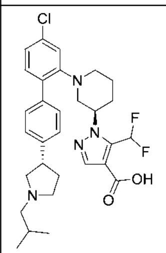 | 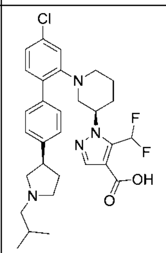 | 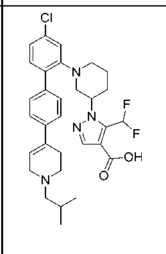 | 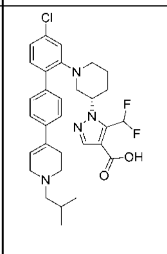 | 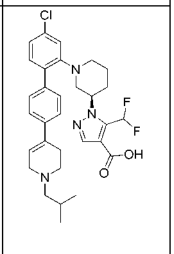 |
| 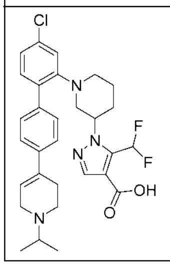 | 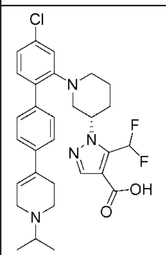 | 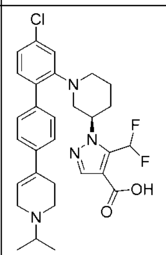 | 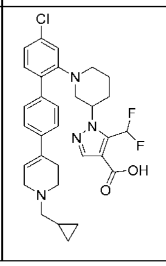 | 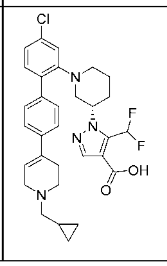 | 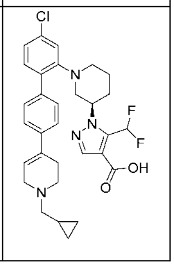 |

| | | | | | |
|---|---|---|---|---|---|
| 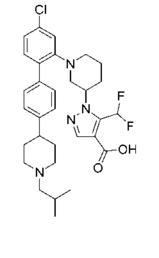 | 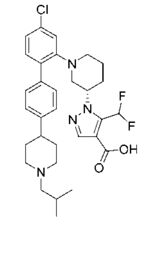 | 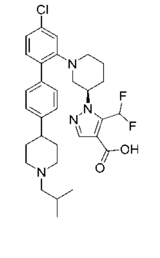 | 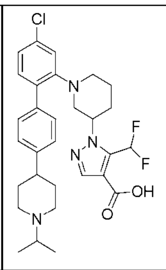 | 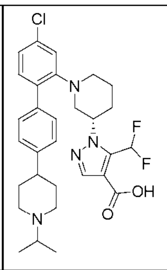 | 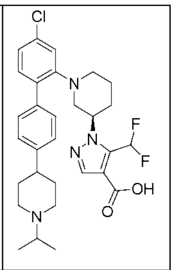 |
| 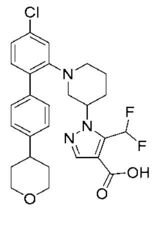 | 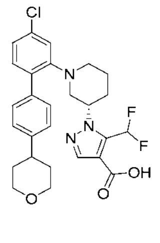 | 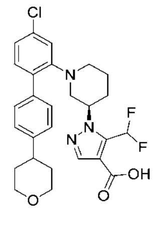 | 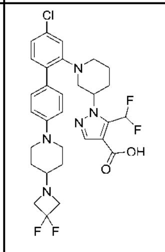 | 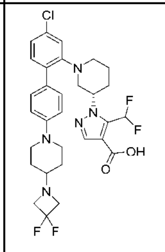 | 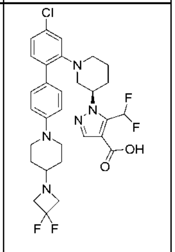 |
| 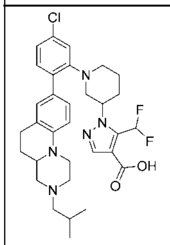 | 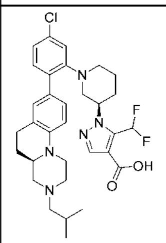 | 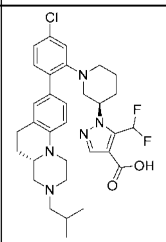 | 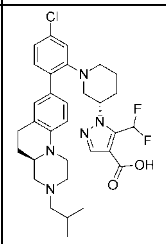 | 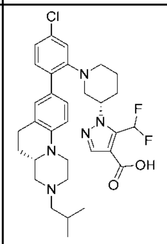 | |
| 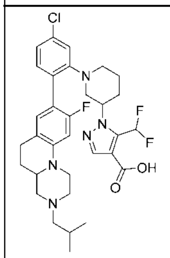 | 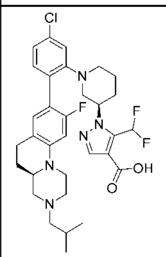 | 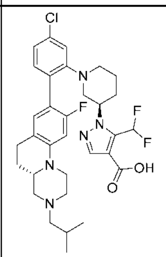 | 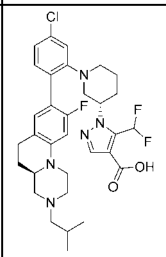 | 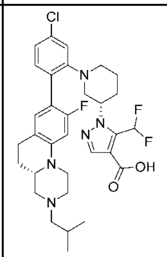 | |
| 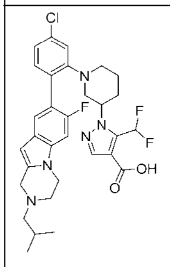 | 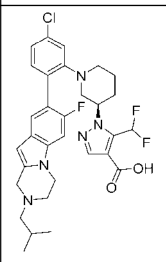 | 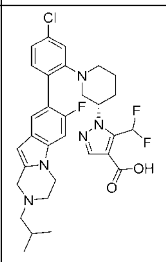 | 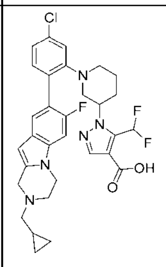 | 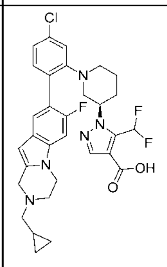 | 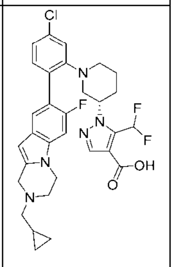 |
| 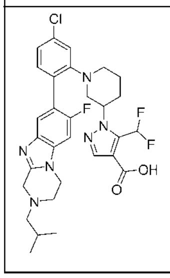 | 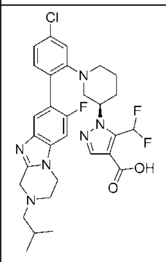 | 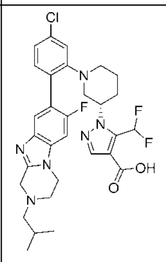 | 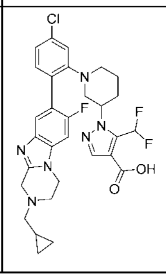 | 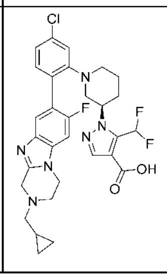 | 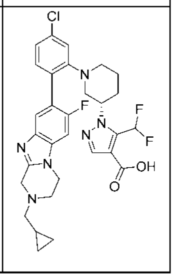 |

| | | | | | |
|---|---|---|---|---|---|
| 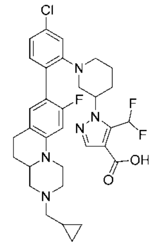 | 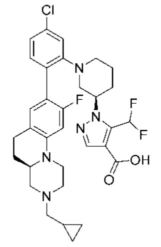 | 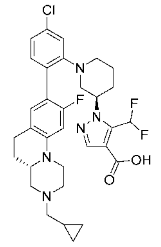 | 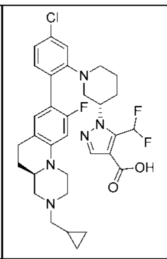 | 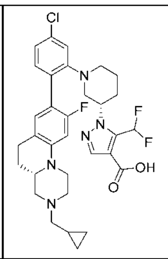 | |
| 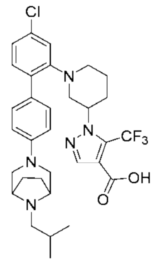 | 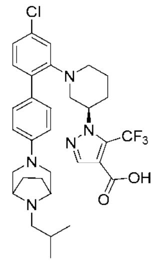 | 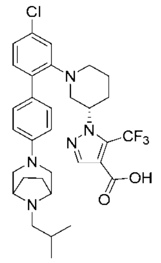 | 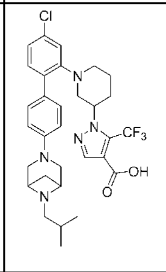 | 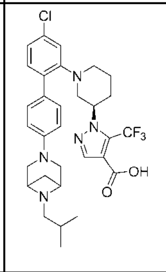 | 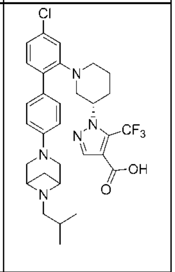 |
| 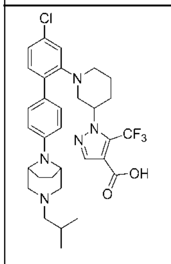 | 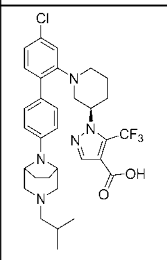 | 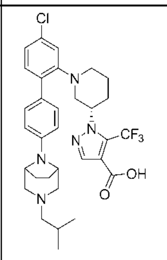 | 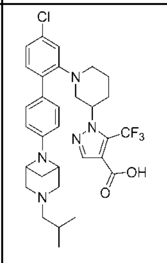 | 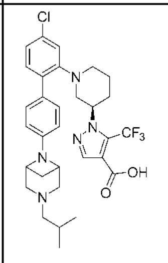 | 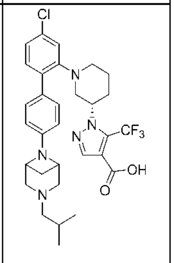 |
| 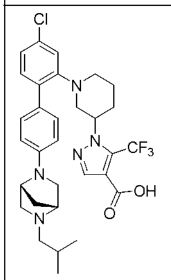 | 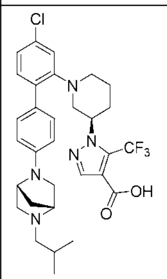 | 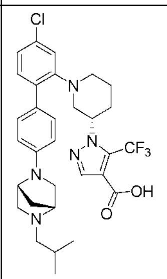 | 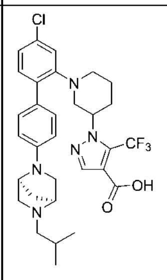 | 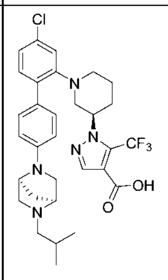 | 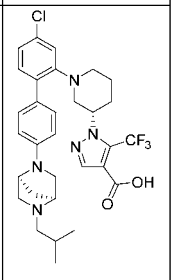 |
| 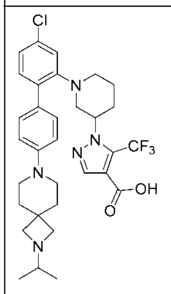 | 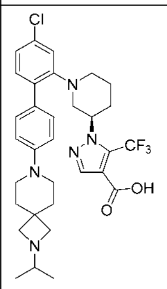 | 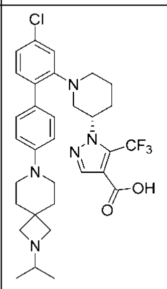 | 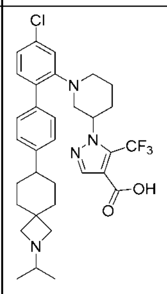 | 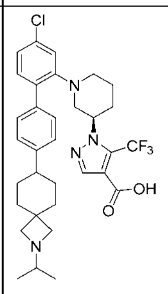 | 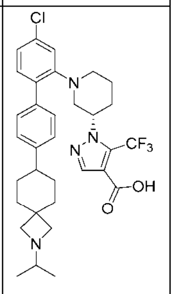 |
| 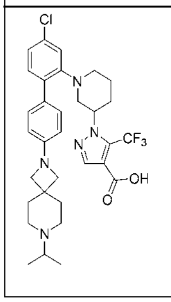 | 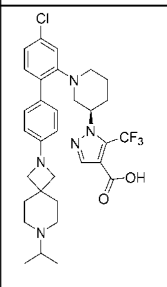 | 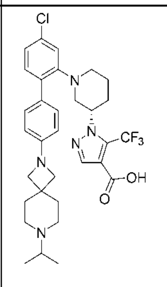 | 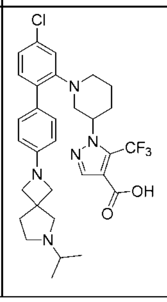 | 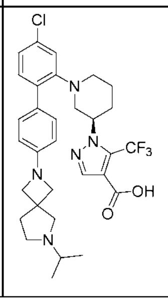 | 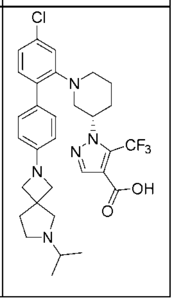 |

| | | | | | |
|---|---|---|---|---|---|
| 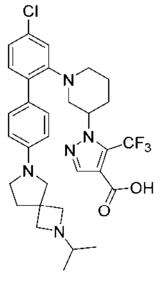 | 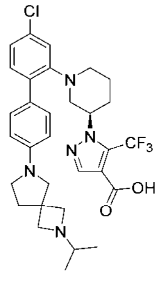 | 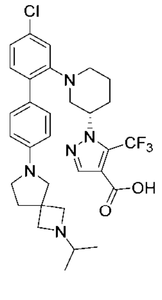 | 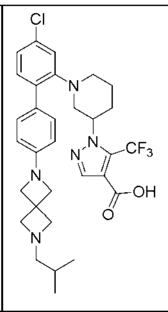 | 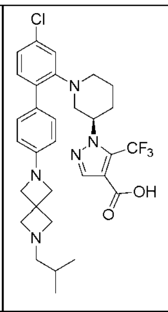 | 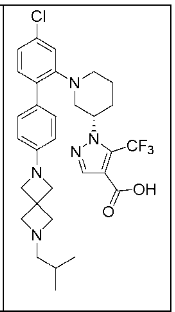 |
| 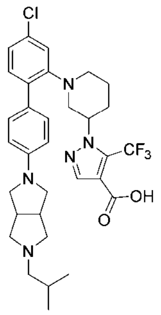 | 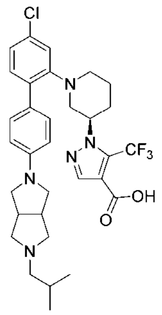 | 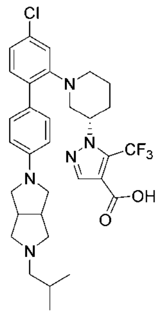 | 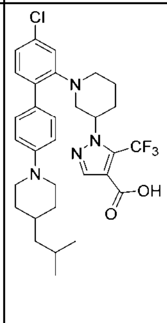 | 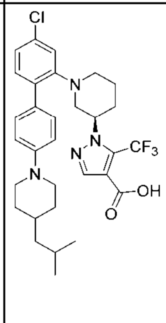 | 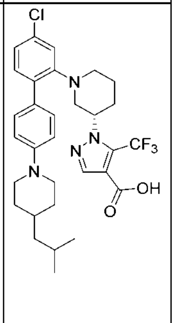 |
| 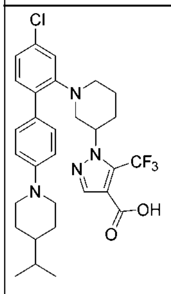 | 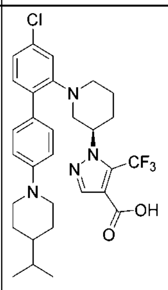 | 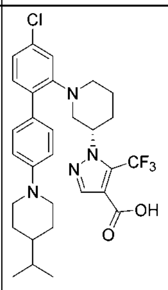 | 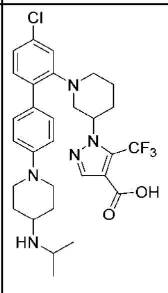 | 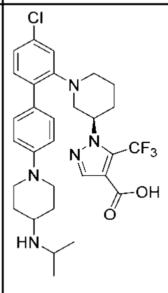 | 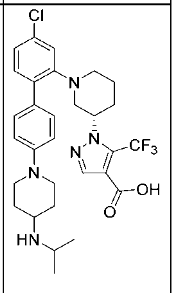 |
| 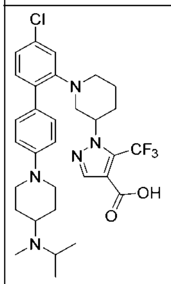 | 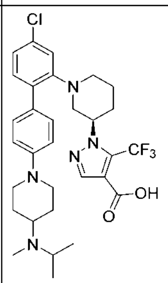 | 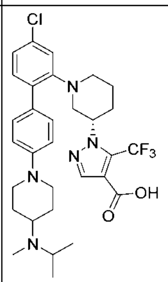 | 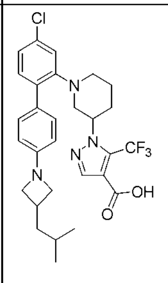 | 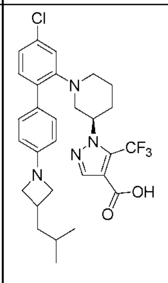 | 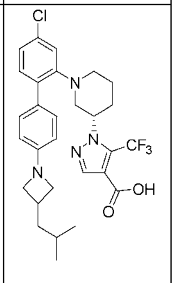 |
| 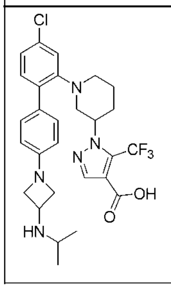 | 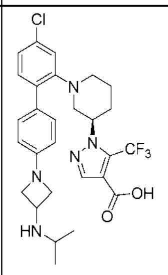 | 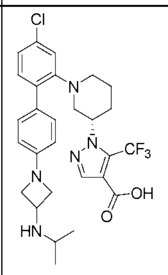 | 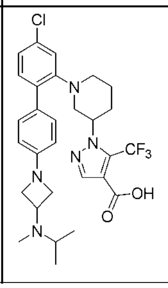 | 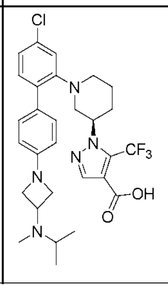 | 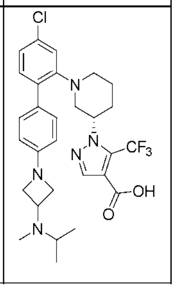 |

| | | | | | |
|---|---|---|---|---|---|
| 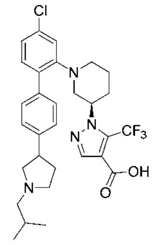 | 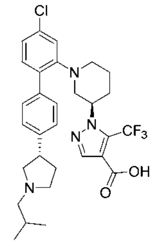 | 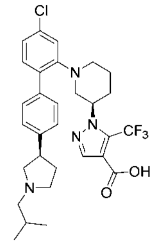 | 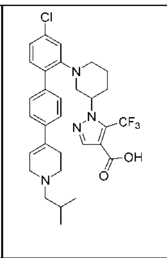 | 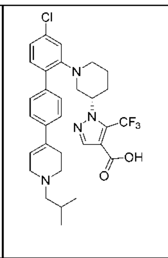 | 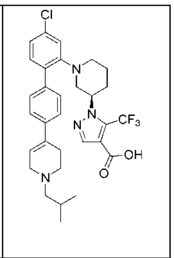 |
| 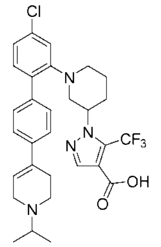 | 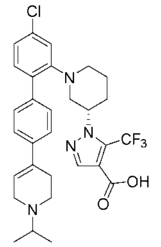 | 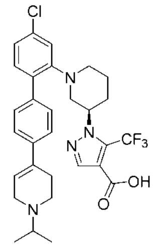 | 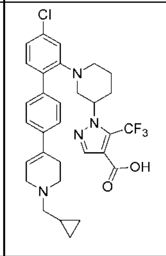 | 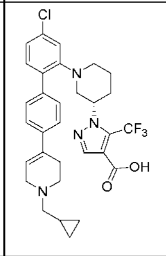 | 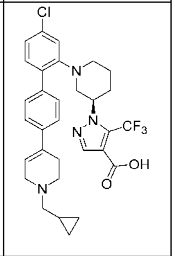 |
| 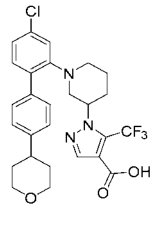 | 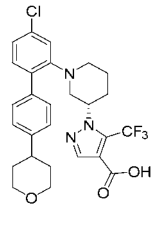 | 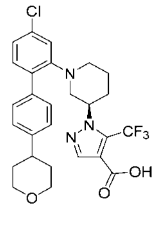 | 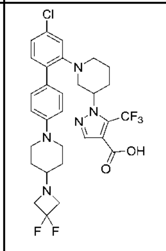 | 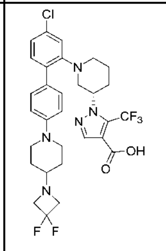 | 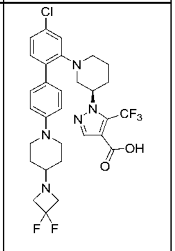 |
| 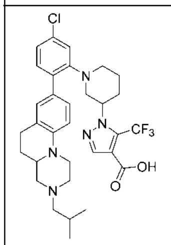 | 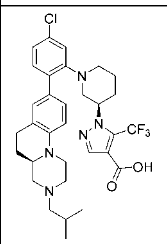 | 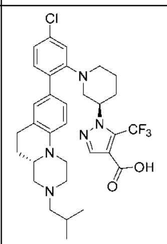 | 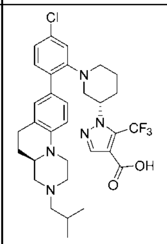 | 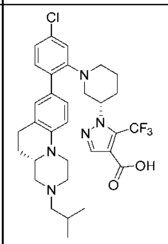 | |
| 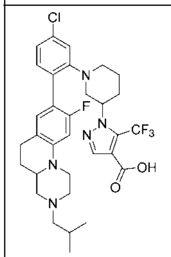 | 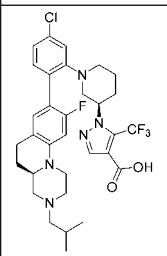 | 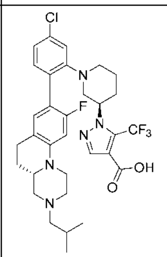 | 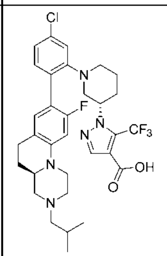 | 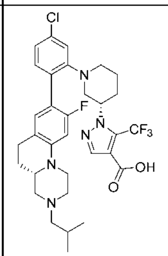 | |
| 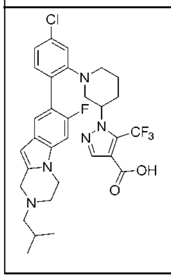 | 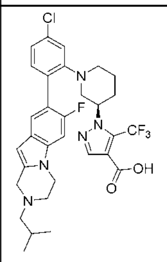 | 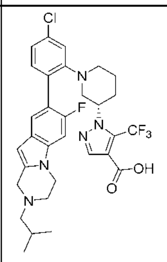 | 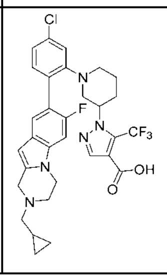 | 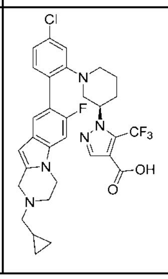 | 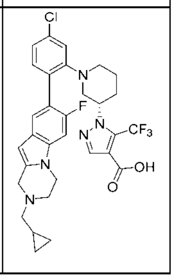 |

| | | | | | |
|---|---|---|---|---|---|
| 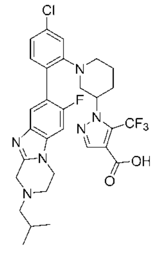 | 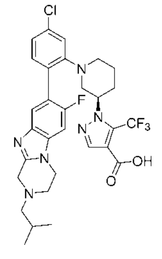 | 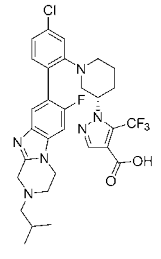 | 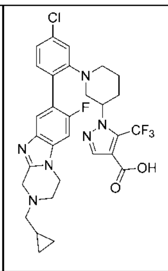 | 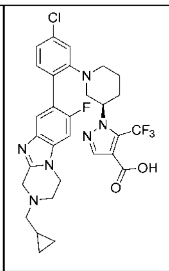 | 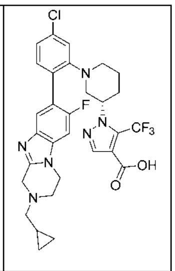 |
| 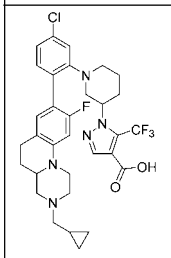 | 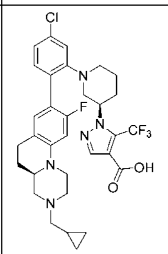 | 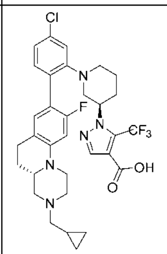 | 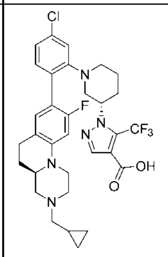 | 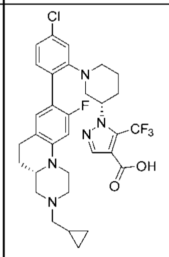 | |
| 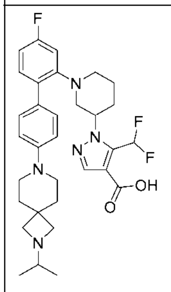 | 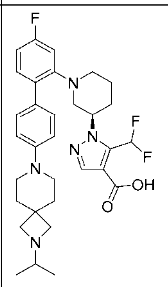 | 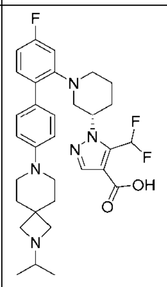 | 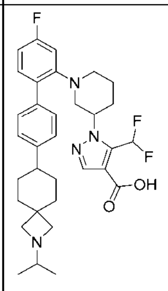 | 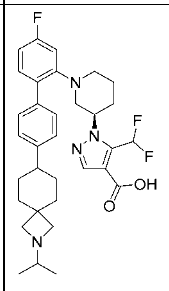 | 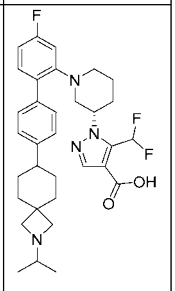 |
| 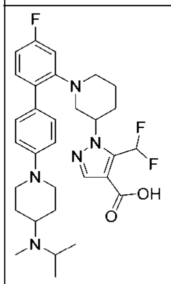 | 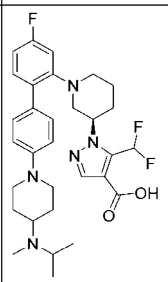 | 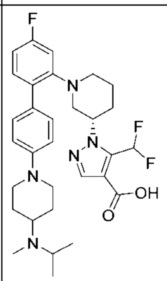 | 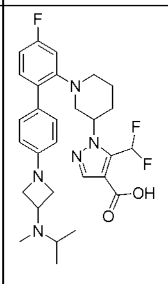 | 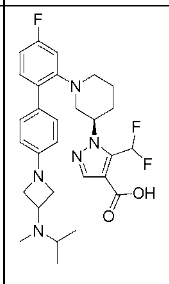 | 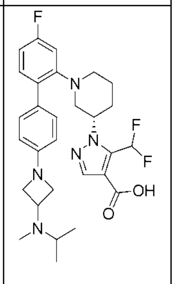 |
| 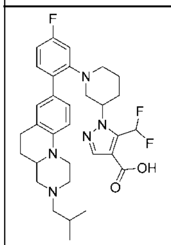 | 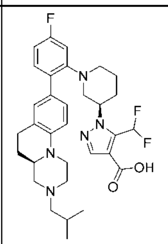 | 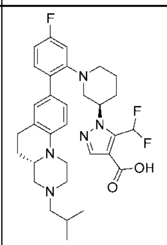 | 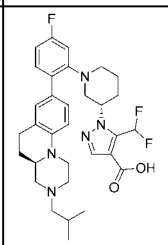 | 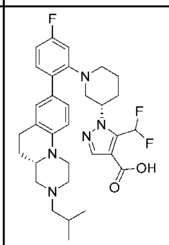 | |
| 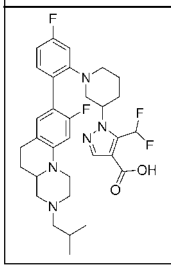 | 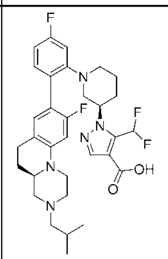 | 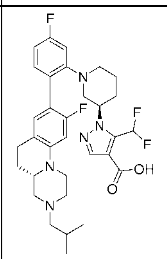 | 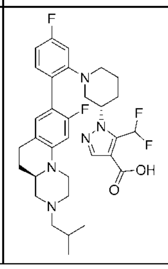 | 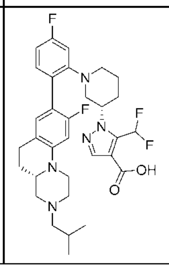 | |

| | | | | | |
|---|---|---|---|---|---|
| 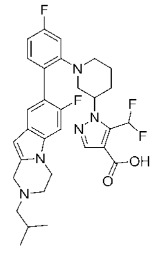 | 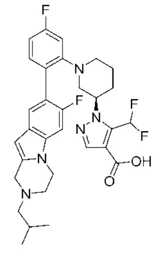 | 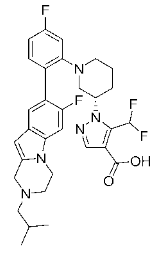 | 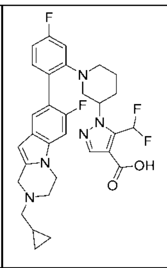 | 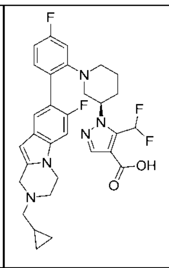 | 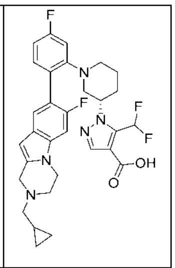 |
| 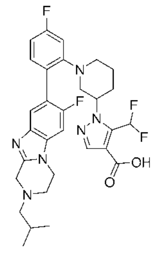 | 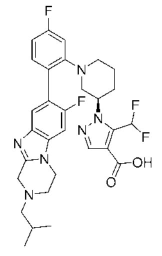 | 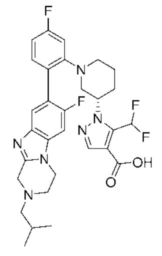 | 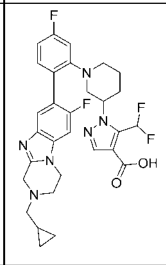 | 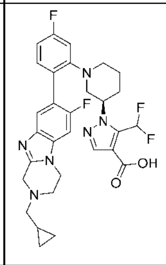 | 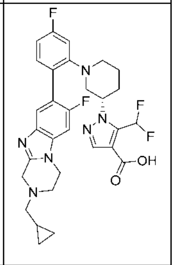 |
| 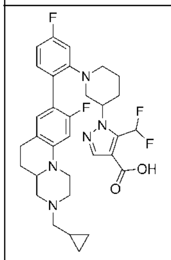 | 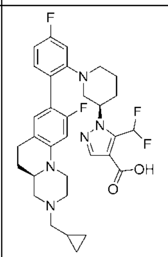 | 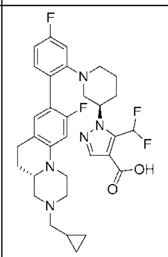 | 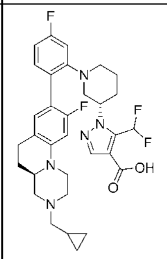 | 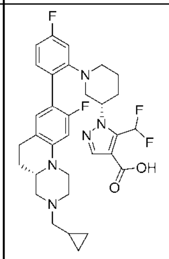 | |
| 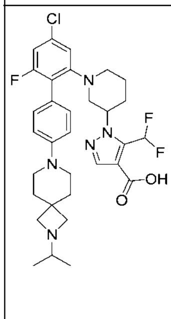 | 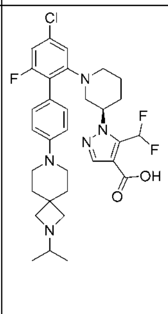 | 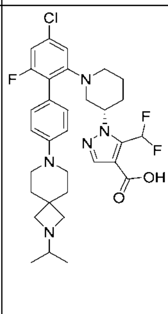 | 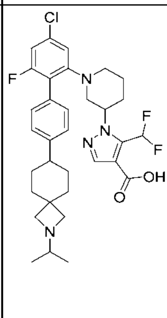 | 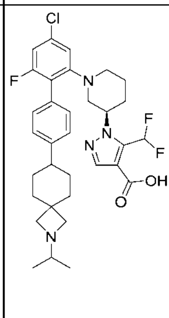 | 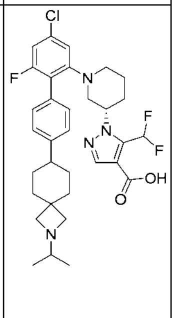 |
| 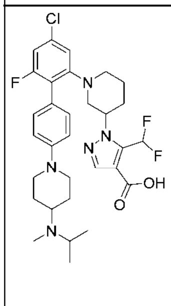 | 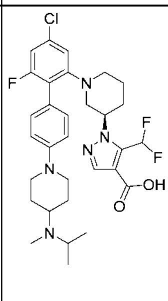 | 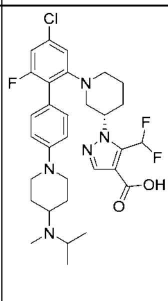 | 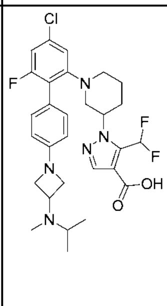 | 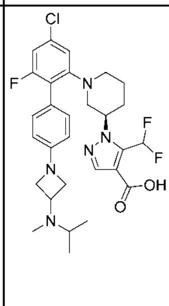 | 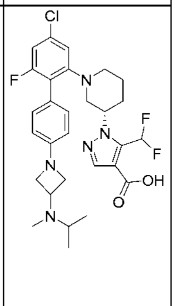 |
| 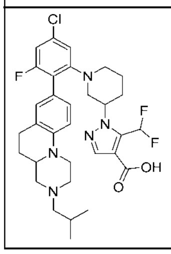 | 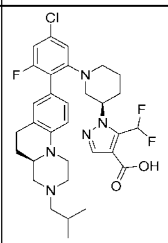 | 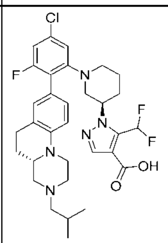 | 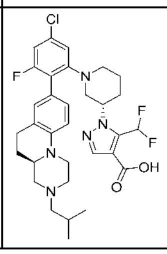 | 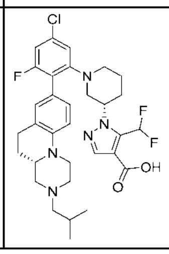 | |

| | | | | | |
|---|---|---|---|---|---|
| 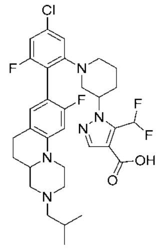 | 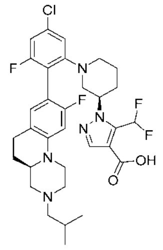 | 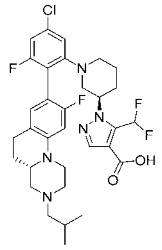 | 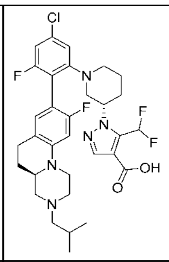 | 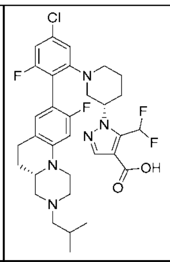 | |
| 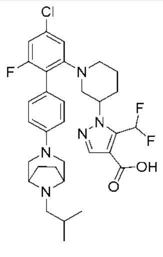 | 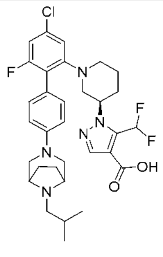 | 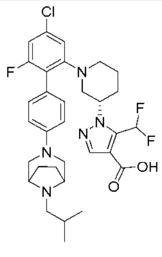 | 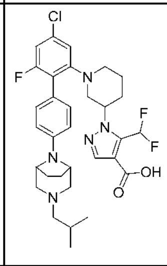 | 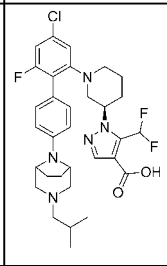 | 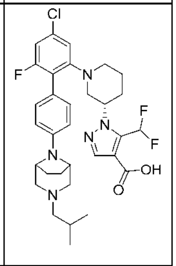 |
| 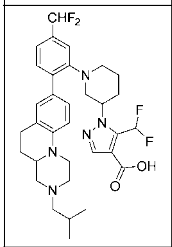 | 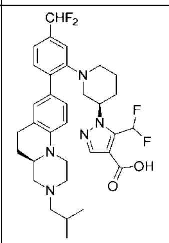 | 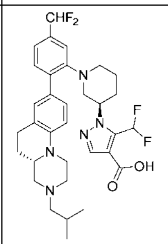 | 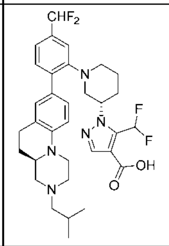 | 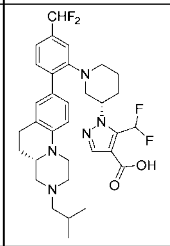 | |
| 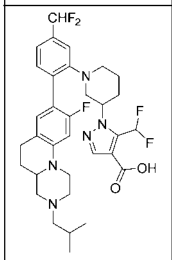 | 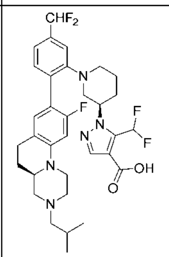 | 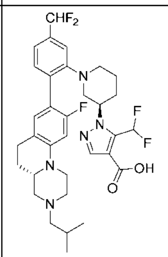 | 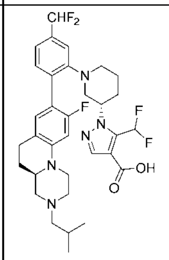 | 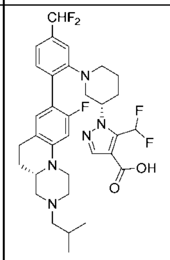 | |
| 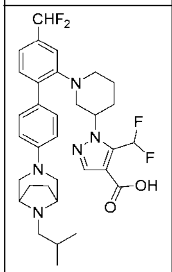 | 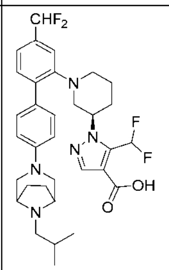 | 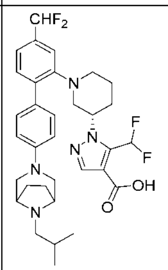 | 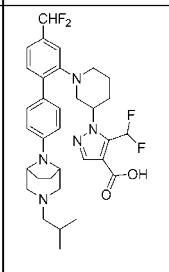 | 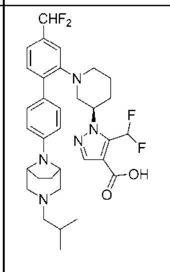 | 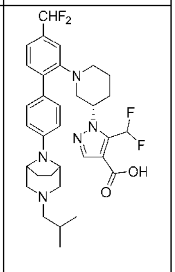 |
| 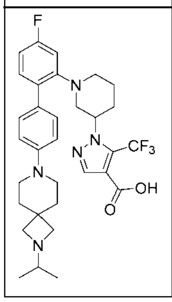 | 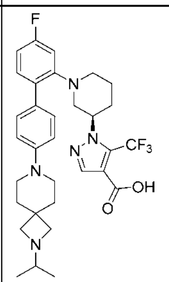 | 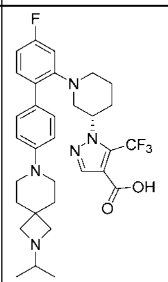 | 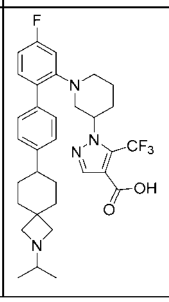 | 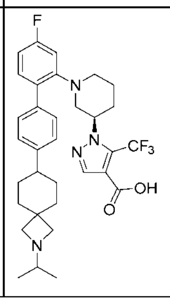 | 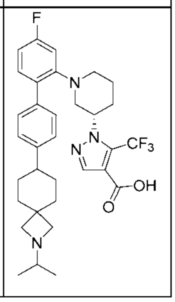 |

| | | | | | |
|---|---|---|---|---|---|
| 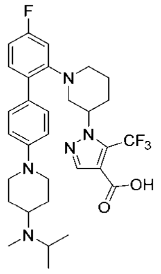 | 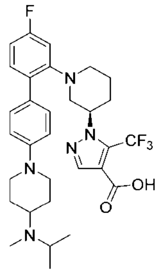 | 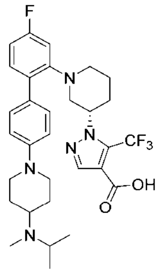 | 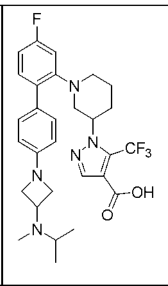 | 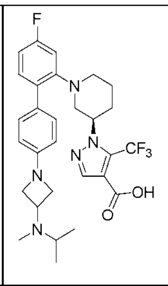 | 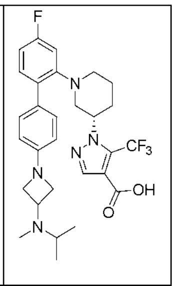 |
| 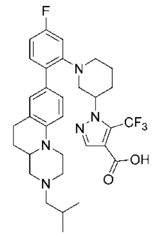 | 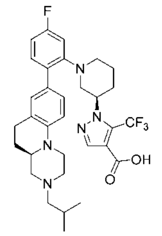 | 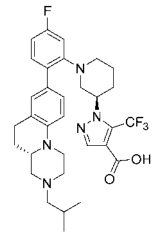 | 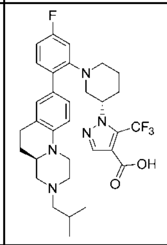 | 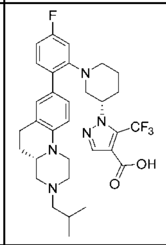 | |
| 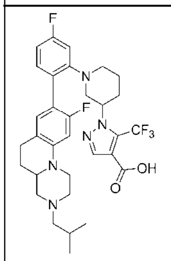 | 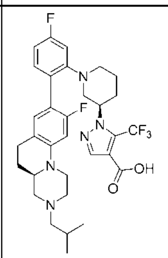 | 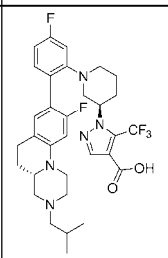 | 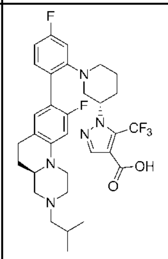 | 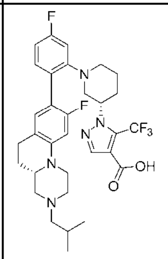 | |
| 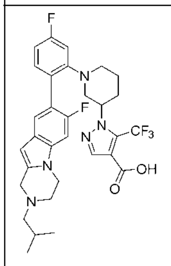 | 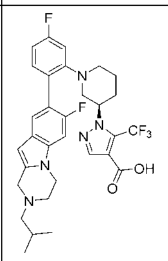 | 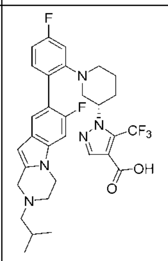 | 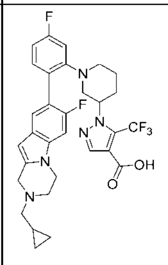 | 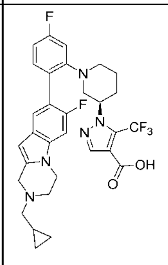 | 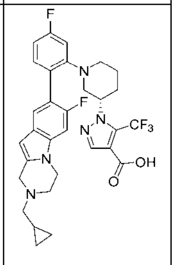 |
| 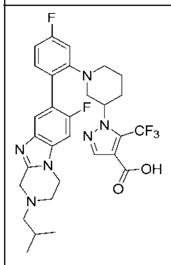 | 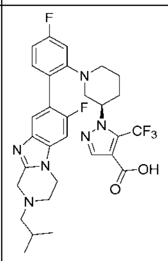 | 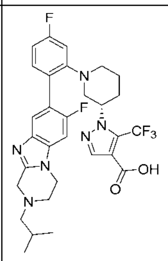 | 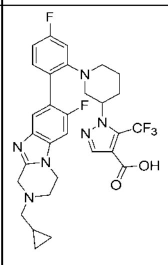 | 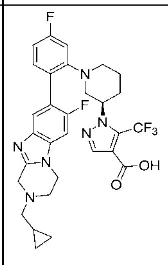 | 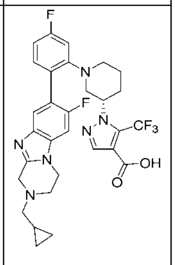 |
| 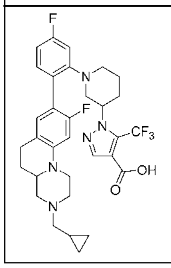 | 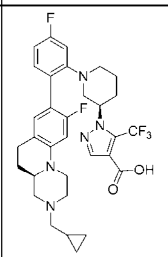 | 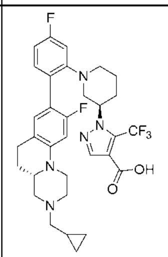 | 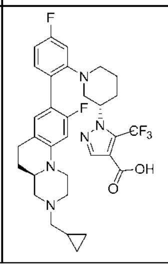 | 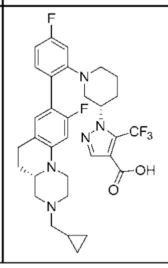 | |

| | | | | | |
|---|---|---|---|---|---|
| 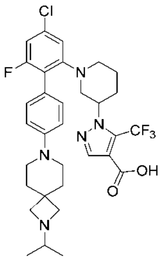 | 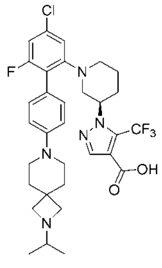 | 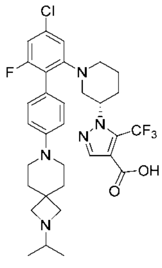 | 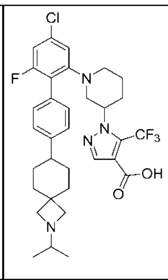 | 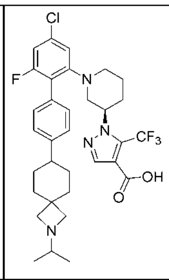 | 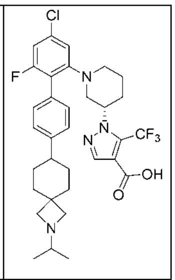 |
| 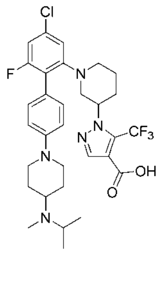 | 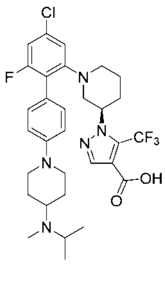 | 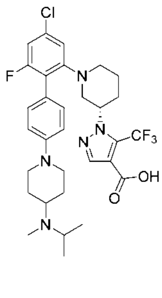 | 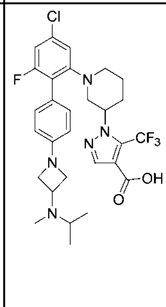 | 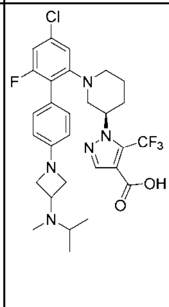 | 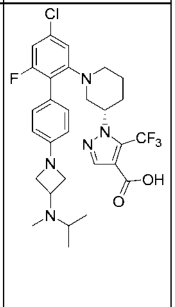 |
| 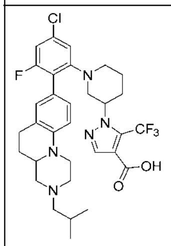 | 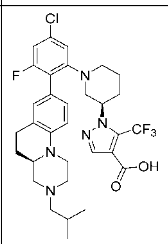 | 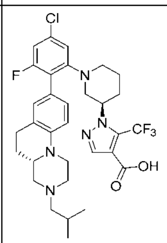 | 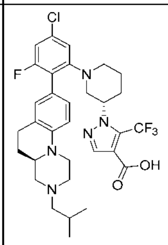 | 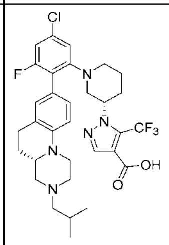 | |
| 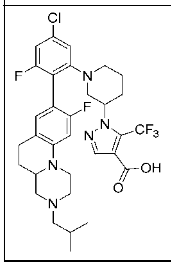 | 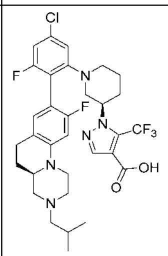 | 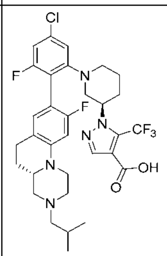 | 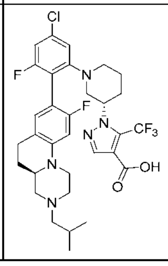 | 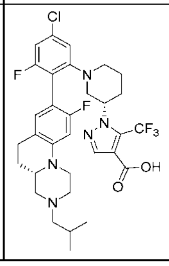 | |

.

The present invention relates to a pharmaceutical composition comprising the compound as described above, or the stereoisomer, tautomer, deuterated compound, solvate, prodrug, metabolite, pharmaceutically acceptable salt, or co-crystal thereof, and a pharmaceutically acceptable carrier.

The present invention relates to the use of the compound as described above, or the stereoisomer, tautomer, deuterated compound, solvate, prodrug, metabolite, pharmaceutically acceptable salt, or co-crystal thereof, or the pharmaceutical composition as described above in the preparation of a medicament for treating a disease associated with sGC.

The present invention relates to the use of the compound as described above, or the stereoisomer, tautomer, deuterated compound, solvate, prodrug, metabolite, pharmaceutically acceptable salt, or co-crystal thereof, or the pharmaceutical composition as described above in the preparation of a medicament for treating a cardiovascular disease, a kidney disease, or a respiratory disease, preferably pulmonary arterial hypertension, pulmonary hypertension, chronic obstructive pulmonary disease, chronic kidney disease, or non-proliferative diabetic retinopathy.

The present invention relates to a pharmaceutical composition or pharmaceutical preparation, comprising a therapeutically effective amount of the compound, or the stereoisomer, deuterated compound, solvate, prodrug, metabolite, pharmaceutically acceptable salt, or co-crystal thereof of the present invention, and a pharmaceutically acceptable excipient. The pharmaceutical composition can be in a unit preparation form (the amount of the active ingredient in the unit preparation is also referred to as the "preparation strength").

The present invention also provides a method for treating a disease in a mammal, comprising administering to the mammal a therapeutically effective amount of the compound, or the stereoisomer, deuterated compound, solvate, prodrug, metabolite, pharmaceutically acceptable salt, or co-crystal thereof, or the pharmaceutical composition of the present invention. In some embodiments, the mammal described in the present invention comprises a human.

The term "effective amount" or "therapeutically effective amount" according to the present application refers to a sufficient amount of the compound disclosed in the present application that is administered to ameliorate, to some extent, one or more symptoms of a disease or condition (e.g., a medicament for a cardiovascular disease, a kidney disease, or a respiratory disease, preferably pulmonary arterial hypertension, pulmonary hypertension, chronic obstructive pulmonary disease, chronic kidney disease, or non-proliferative diabetic retinopathy) being treated. In some embodiments, the outcome is the reduction and/or remission of signs, symptoms, or causes of the disease, or any other desired change in the biological system. For example, an "effective amount" for therapeutic use is an amount of the compound disclosed in the present application that is required to provide a clinically significant reduction of the symptoms of the disease. Examples of the therapeutically effective amount include, but are not limited to 0.01-1500 mg, 0.01-1000 mg, 0.01-800 mg, 0.01-600 mg, 0.1-1500 mg, 0.1-1000 mg, 0.1-800 mg, 0.1-600 mg, 1-1500 mg, 1-1000 mg, 1-800 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 0.01-100 mg, 0.01-50 mg, 0.01-10 mg, 0.01-5 mg, 0.05-10 mg, 0.05-5 mg, 0.1-5 mg, 1-5 mg, 0.1-1 mg, or 0.1-5 mg.

In some embodiments, the pharmaceutical composition comprises the compound, or the stereoisomer, deuterated compound, solvate, prodrug, metabolite, pharmaceutically acceptable salt, or co-crystal thereof of the present invention in an amount including, but not limited to 0.01-1500 mg, 1-1500 mg, 1-1000 mg, 1-800 mg, 1-600 mg, 20-400 mg, 25-200 mg, 0.01 mg, 0.05 mg, 0.1 mg, 0.15 mg, 0.2 mg, 0.3 mg, 0.5 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, or 300 mg.

Provided is a method for treating a disease in a mammal, comprising administering to the subject a therapeutically effective amount of the compound, or the stereoisomer, deuterated compound, solvate, prodrug, metabolite, pharmaceutically acceptable salt, or co-crystal thereof of the present invention, wherein the therapeutically effective amount is preferably 0.01-1500 mg, and the disease is preferably use in a medicament for a cardiovascular disease, a kidney disease, or a respiratory disease, preferably pulmonary arterial hypertension, pulmonary hypertension, chronic obstructive pulmonary disease, chronic kidney disease, or non-proliferative diabetic retinopathy.

Provided is a method for treating a disease in a mammal, comprising administering to the subject a medicament, i.e., the compound, or the stereoisomer, deuterated compound, solvate, prodrug, metabolite, pharmaceutically acceptable salt, or co-crystal thereof of the present invention in a daily dose of 0.01-1500 mg/day, wherein the daily dose can be a single dose or divided doses. In some embodiments, the daily dose includes, but is not limited to 0.01-1500 mg/day, 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day. In some embodiments, the daily dose includes, but is not limited to 0.01 mg/day, 0.05 mg/day, 0.1 mg/day, 0.15 mg/day, 0.2 mg/day, 0.3 mg/day, 0.5 mg/day, 1 mg/day, 2 mg/day, 5 mg/day, 10 mg/day, 20 mg/day, 25 mg/day, 50 mg/day, 100 mg/day, 125 mg/day, 150 mg/day, 200 mg/day, 400 mg/day, 600 mg/day, or 800 mg/day.

The present invention relates to a kit, wherein the kit may comprise a composition in the form of a single dose or multiple doses and comprises the compound, or the stereoisomer, deuterated compound, solvate, prodrug, metabolite, pharmaceutically acceptable salt, or co-crystal thereof of the present invention, and the amount of the compound, or the stereoisomer, deuterated compound, solvate, prodrug, metabolite, pharmaceutically acceptable salt, or co-crystal thereof of the present invention is identical to the amount of same in the pharmaceutical composition as described above.

In the present invention, the amount of the compound, or the stereoisomer, deuterated compound, solvate, prodrug, metabolite, pharmaceutically acceptable salt, or co-crystal thereof of the present invention is calculated in the form of free base in each case.

The term "preparation strength" refers to the weight of the active ingredient contained in each vial, tablet or other unit preparation.

Synthesis method I:

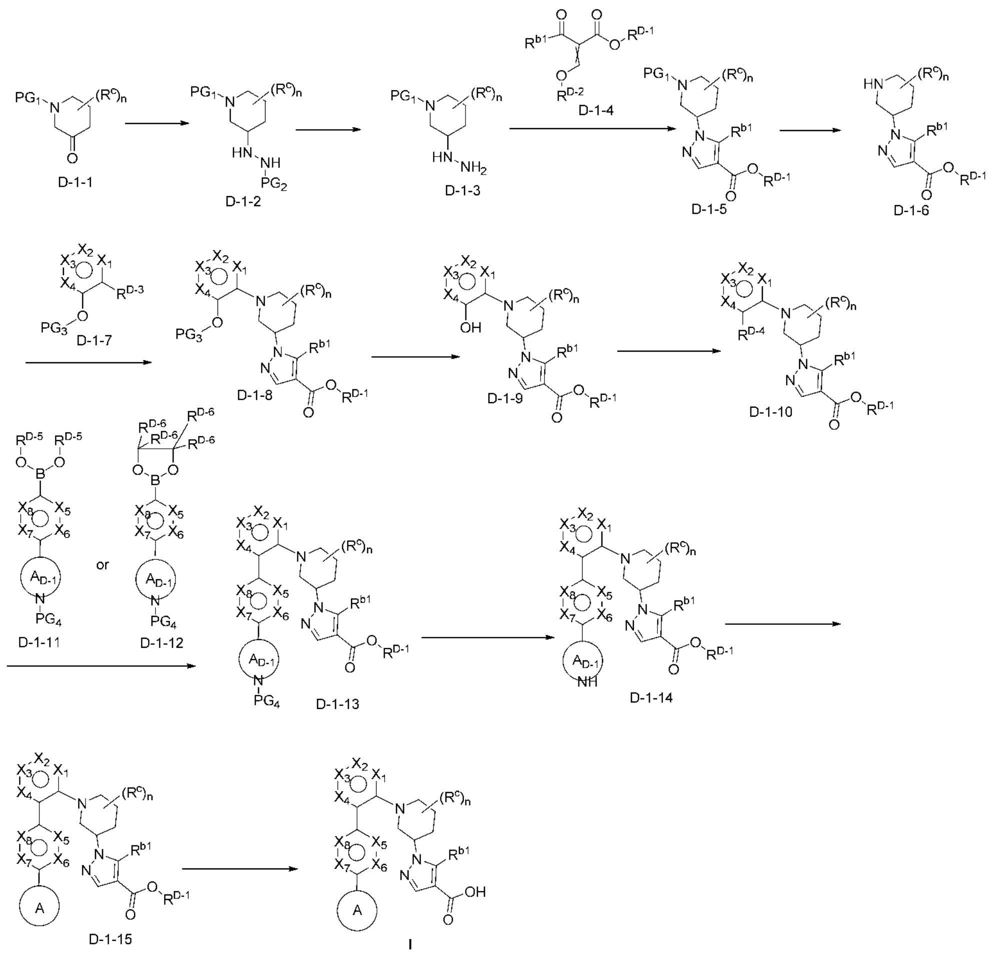

D-1-1, D-1-2, D-1-3, D-1-4, D-1-5, D-1-6, D-1-7, D-1-8, D-1-9, D-1-10, D-1-11 or D-1-12, D-1-13, D-1-14, D-1-15, I

$PG_1$, $PG_2$, and $PG_4$ are each independently selected from amino-protecting groups, and each $PG_3$ is independently selected from hydroxyl-protecting groups;
$R^{D-1}$ and $R^{D-2}$ are each independently $C_{1-6}$ alkyl;
each $R^{D-3}$ is independently selected from halogen or leaving groups, preferably from Br, I, or OTf;
each $R^{D-4}$ is independently selected from oxygen-containing leaving groups;
$R^{D-5}$ and $R^{D-6}$ are each independently selected from H or $C_{1-6}$ alkyl;

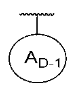

is 4- to 12-membered nitrogen-containing heterocyclyl, wherein the nitrogen-containing heterocyclyl is optionally substituted with 1 to 4 $R^a$; and
the definitions of the remaining groups are consistent with those in the description of the present invention.

The compound of general formula (D-1-1) is subjected to a reductive amination reaction to obtain a compound of general formula (D-1-2);

the compound of general formula (D-1-2) is subjected to removal of the amino-protecting group to obtain a compound of general formula (D-1-3);
the compound of general formula (D-1-3) and a compound of general formula (D-1-4) are subjected to a cyclization reaction to obtain a compound of general formula (D-1-5);
the compound of general formula (D-1-5) is subjected to removal of the amino-protecting group to obtain a compound of general formula (D-1-6);
the compound of general formula (D-1-6) and a compound of general formula (D-1-7) are subjected to a coupling reaction or a substitution reaction to obtain a compound of general formula (D-1-8);
the compound of general formula (D-1-8) is subjected to removal of the hydroxyl-protecting group to obtain a

compound of general formula (D-1-9);

the compound of general formula (D-1-9) is subjected to a substitution reaction to obtain a compound of general formula (D-1-10);

the compound of general formula (D-1-10) and a compound of general formula (D-1-11) or a compound of general formula (D-1-12) are subjected to a coupling reaction to obtain a compound of general formula (D-1-13);

the compound of general formula (D-1-13) is subjected to removal of the amino-protecting group to obtain a compound of general formula (D-1-14);

the compound of general formula (D-1-14) is subjected to a reductive amination reaction, a condensation reaction, a substitution reaction, or a coupling reaction to obtain a compound of general formula (D-1-15); and

the compound of general formula (D-1-13) is subjected to a hydrolysis reaction or a hydrogenation reaction to obtain a compound of general formula (I).

Synthesis method II:

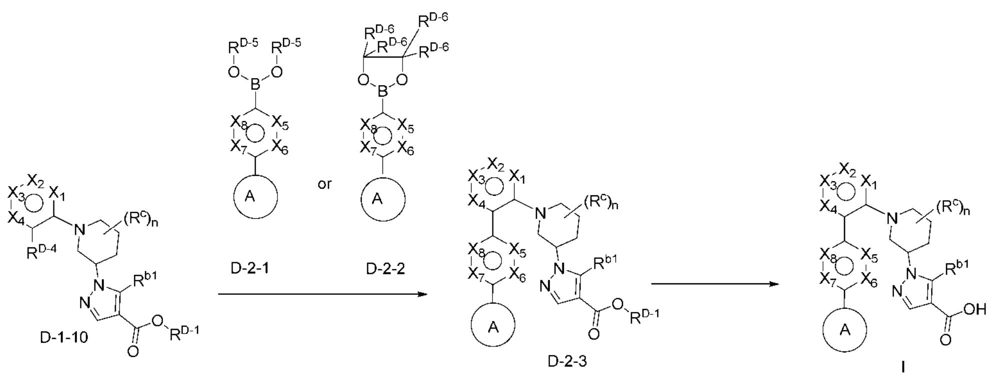

The compound of general formula (D-1-10) and a compound of general formula (D-2-1) or a compound of general formula (D-2-2) are subjected to a coupling reaction to obtain a compound of general formula (D-2-3); and the compound of general formula (D-2-3) is subjected to a hydrolysis reaction or a hydrogenation reaction to obtain a compound of general formula (I).

Unless otherwise stated, the terms used in the description and claims have the following meanings.

The carbon, hydrogen, oxygen, sulfur, nitrogen, F, Cl, Br and I involved in the groups and compounds of the present invention all comprise their isotopes, and the carbon, hydrogen, oxygen, sulfur or nitrogen involved in the groups and compounds of the present invention is optionally further replaced with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen) and tritium (T, also known as superheavy hydrogen), the isotopes of oxygen comprise $^{16}O$, $^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"CN" refers to cyano.

"Halogen" refers to F, Cl, Br or I.

"Halogen-substituted" refers to substitution with F, Cl, Br, or I, including but not limited to substitution with 1 to 10 substituents selected from F, Cl, Br, or I, or substitution with 1 to 6 substituents selected from F, Cl, Br, or I, or substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be monovalent, divalent, trivalent or tetravalent.

"Heteroalkyl" refers to a substituted or unsubstituted alkyl group in which one or more (including but not limited to 2, 3, 4, 5 or 6) carbon atoms are replaced by heteroatoms (including but not limited to N, O or S). Non-limiting examples include $-X-(CH_2)v-X-(CH_2)v-X-(CH_2)v-H$ (v is an integer from 1 to 5; each X is independently selected from a bond or a heteroatom, which includes but is not limited to N, O or S; at least one X is selected from a heteroatom; and N or S in the heteroatom can be oxidized to various oxidation states). Heteroalkyl can be monovalent, divalent, trivalent or tetravalent.

"Alkylene" refers to a substituted or unsubstituted linear or branched divalent saturated hydrocarbon group,

including $-(CH_2)_v-$ (v is an integer from 1 to 10), and examples of alkylene include, but are not limited to, methylene, ethylene, propylene, butylene, etc.

"Heteroalkylene" refers to a substituted or unsubstituted alkylene group in which one or more (including but not limited to 2, 3, 4, 5 or 6) carbon atoms are replaced by heteroatoms (including but not limited to N, O or S). Non-limiting examples include $-X-(CH_2)v-X-(CH_2)v-X-(CH_2)v-$, wherein v is an integer from 1 to 5, each X is independently selected from a bond, N, O or S, and at least one X is selected from N, O or S.

"Cycloalkyl" refers to a substituted or unsubstituted saturated carbocyclic hydrocarbon group, typically having 3 to 12 carbon atoms, and the cycloalkyl can be monocyclic, fused, bridged or spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, 3 to 8 atoms, or 1 to 3 heteroatoms selected from N, O, S, or Se. The C, N and S in the ring of the heterocycloalkyl can be oxidized to various oxidation states. The heterocycloalkyl can be monocyclic, fused, bridged or spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2*H*-pyranyl, dioxolanyl, dioxanyl, pyrrolidyl, piperidyl, imidazolidinyl, oxazolidinyl, oxazinanyl, morpholinyl, hexahydropyrimidyl, piperazinyl, and

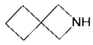.

The heterocycloalkyl can be monovalent, divalent, trivalent or tetravalent.

"Alkenyl" refers to a substituted or unsubstituted linear or branched unsaturated hydrocarbon group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The alkenyl can be monovalent, divalent, trivalent or tetravalent.

"Alkynyl" refers to a substituted or unsubstituted linear or branched unsaturated hydrocarbon group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. The alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

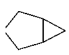, 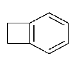, 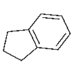 or 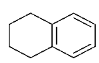.

The "carbocyclyl" or "carbocyclic ring" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system, and contains 1 or more (including but not limited to 2, 3, 4 or 5) heteroatoms selected from N, O, S or Se, and C, N, S, or Se (which may be selectively substituted) in the ring of the heterocyclyl can be oxidized to various oxidation states. The heterocyclyl can be connected to a heteroatom or a carbon atom, can be connected to an aromatic ring or a non-aromatic ring, and is optionally monocyclic, bridged, fused, or spirocyclic. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

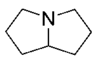 ,

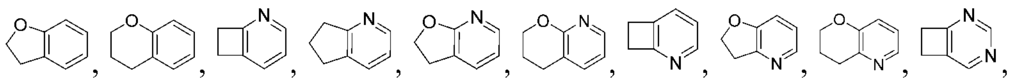

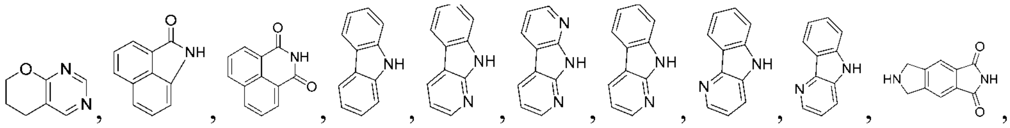

and

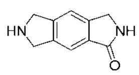 .

The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or $S(=O)_n$ (n is 0, 1, or 2).

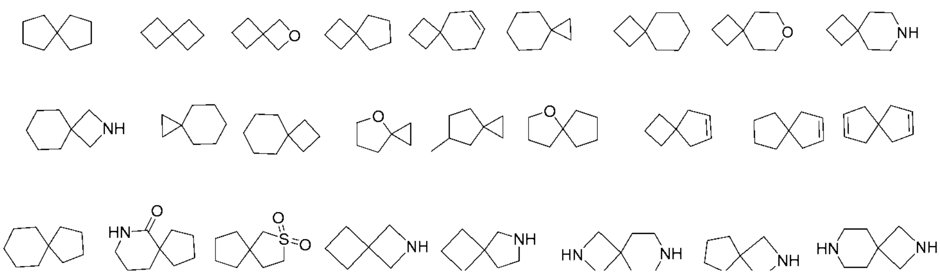

. The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes, but is not limited to 5 to 20, 5 to 14, 5 to 12, or 5 to 10. Non-limiting examples include:

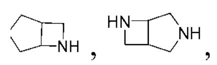

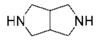 ,

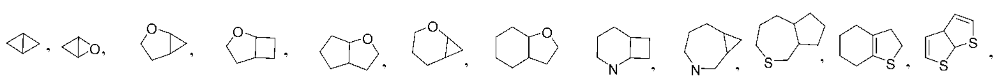

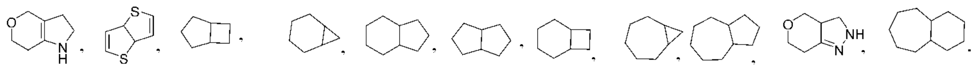

. The "fused ring" or "fused ring group" can be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include

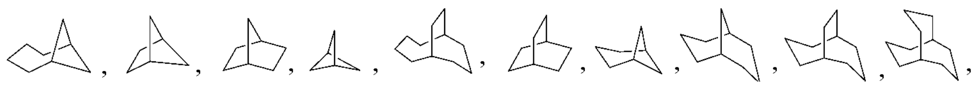

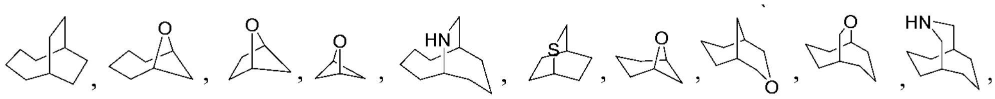

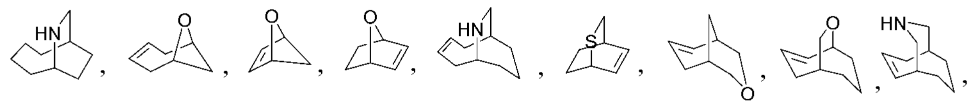

cubane, or adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and

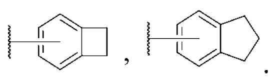

The "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n, or Se(=O)n, wherein n is 0, 1 or 2), wherein the number of ring atoms in the heteroaromatic ring includes, but is not limited to, 5 to 15, 5 to 10, or 5 to 6. The atoms C, N, and S in the ring are optionally oxidized (i.e., C(=O), NO, S(=O)n, and Se(=O)n, wherein n is 1 or 2). Non-limiting examples of heteroaryl include, but are not limited to pyridyl, furyl, thienyl, selenophenyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, pyridonyl, etc. The heteroaryl ring can be fused to a saturated or unsaturated carbocyclic ring or heterocyclic ring, wherein the ring connected to the parent structure is an aryl ring. Non-limiting examples include

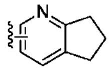, 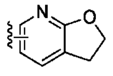, 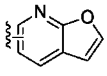, 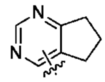, 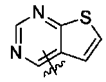, 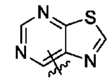, 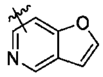, 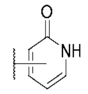,

and

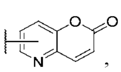, 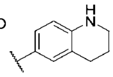, 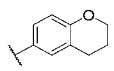.

The definition of the heteroaryl herein is consistent with this definition. The heteroaryl can be monovalent, divalent, trivalent or tetravalent. When divalent, trivalent or tetravalent, the points of connection are on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$, $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulfonyl, or trifluoromethylsulfonyl; alternatively, $R^b$ and $R^c$ may form five- or six-membered cycloalkyl or heterocyclyl; $R^a$ and $R^d$ are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 $R^k$" refers to a substitution with 1, 2, 3 or 4 $R^k$. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from H or F" means that the bridged-heterocyclic ring is optionally substituted with 1, 2, 3 or 4 substituents selected from H or F.

An X- to Y-membered ring (X and Y are integers, $3 \le X < Y$, and $X < Y \le 20$ (i.e., any integer from 4 to 20)) includes X-, X+1-, X+2-, X+3-, X+4-,...or Y-membered rings. Rings include a heterocyclic ring, a carbocyclic ring, an aromatic ring, aryl, heteroaryl, cycloalkyl, a mono-heterocyclic ring, a fused-heterocyclic ring, a spiro-heterocyclic ring, or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A $C_{x-y}$ carbocyclic ring (including aryl, cycloalkyl, a mono-carbocyclic ring, a spiro-carbocyclic ring, a fused-carbocyclic ring, or a bridged-carbocyclic ring) includes $C_x$-, $C_{x+1}$-, $C_{x+2}$-, $C_{x+3}$-, $C_{x+4}$-,...or $C_y$-membered rings (x is an integer, $3 \le x < y$, and y is any integer from 4 to 20). For example, "$C_{3-6}$ cycloalkyl" refers to $C_3$, $C_4$, $C_5$ or $C_6$ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example,

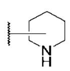

indicates that any connectable site on the piperidyl group can be connected to other groups via one chemical bond, including at least

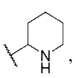, 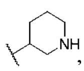, 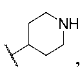, 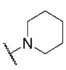

these 4 connection modes. Even if an H atom is shown on the N atom,

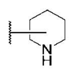

also includes

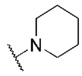.

For example,

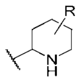

indicates that the R group on the piperidyl group can be located on C or N, including at least

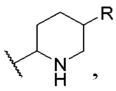, 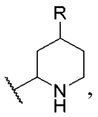, 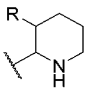, 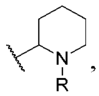, 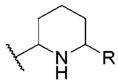.

When the connecting groups listed do not specify their connection direction, their connection directions include both the left-to-right and right-to-left reading orders. For example, for A-L-B, wherein L is -M-W-, it includes both A-M-W-B and A-W-M-B.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

The term "preparation strength" refers to the weight of the active ingredient contained in each vial, tablet or other unit preparation.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo*. The prodrug of the present invention is prepared by modifying an amino or carboxyl group in the compound of the present invention, and the modification can be removed by conventional operations or removed in vivo to obtain a parent compound. When the prodrug of the present invention is administered to a mammalian individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerization and amide-imino alcohol isomerization.

"$IC_{50}$" refers to the concentration of a medicament or inhibitor required to inhibit half of a given biological process (or a component of the process such as an enzyme, a receptor and a cell).

Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of $10^{-6}$ (ppm). NMR is measured with NMR instruments (Bruker Avance III 400 and Bruker Avance 300), and the solvent for determination is deuterated dimethyl sulfoxide (DMSO-$d_6$), deuterated chloroform ($CDCl_3$), and deuterated methanol ($CD_3OD$), and the internal standard is tetramethylsilane (TMS);

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 μm).

Yantai Huanghai $HSGF_{254}$ or Qingdao $GF_{254}$ silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm-0.5 mm;

and for the column chromatography, Yantai Huanghai silica gel of 200-300 mesh is generally used as a carrier.
* next to a chemical bond represents that the chirality of the chiral atom is R or S.
Boc: tert-butoxycarbonyl;
Ts: p-toluenesulfonyl;
Cbz: benzyloxycarbonyl;
TMS: trimethylsilyl.

**Example 1:**

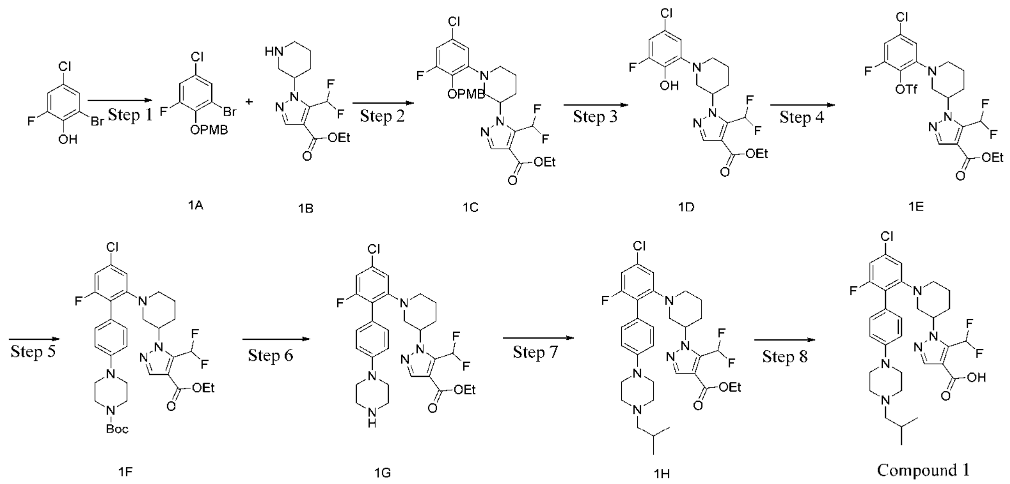


1A 1B 1C 1D 1E 1F 1G 1H Compound 1

Step 1: Synthesis of **1A**

2-Bromo-4-chloro-6-fluorophenol (2.2 g, 9.76 mmol), 4-methoxybenzyl chloride (1.60 g, 10.22 mmol), potassium carbonate (4.05 g, 29.30 mmol), and acetone (30 mL) were added into a reaction flask. The mixture was heated at 70°C in an oil bath overnight and then cooled to room temperature. The mixture was filtered, an appropriate amount of silica gel was added to the filtrate, and the resulting mixture was then concentrated under reduced pressure. The residue was purified by column chromatography with eluent PE/EA = 100/0 to 90/10 to afford **1A** (2.8 g, yield: 83%).

Step 2: Synthesis of **1C**

**1A** (0.60 g, 1.74 mmol), **1B** (CAS: 2781965-82-8, see patent WO 2022122910 for its synthesis, 0.48 g, 1.74 mmol), $Pd_2DBa_3$ (CAS: 60748-47-2, 0.16 g, 0.17 mmol), (R)-SEGPHOS (0.21 g, 0.35 mmol), cesium carbonate (1.70 g, 5.22 mmol), and 1,4-dioxane (15 mL) were added into a reaction flask. The mixture was purged with nitrogen three times, and heated at 100°C in an oil bath overnight and then cooled to room temperature. The mixture was filtered, an appropriate amount of silica gel was added to the filtrate, and the resulting mixture was then concentrated under reduced pressure. The residue was purified by column chromatography (eluent: PE-EA = 100-0 to 90-10) to afford **1C** (0.34 g, yield: 36%).

Step 3: Synthesis of **1D**

**1C** (0.34 g, 0.63 mmol) and dichloromethane (5 mL) were added into a reaction flask. Trifluoroacetic acid (3 mL) was added with stirring, and the mixture was stirred at room temperature overnight. The mixture was concentrated to dryness under reduced pressure. Dichloromethane (20 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography (eluent: PE-EA = 100-0 to 90-10) to afford **1D** (0.20 g, yield: 76%).

Step 4: Synthesis of **1E**

**1D** (0.20 g, 0.48 mmol), triethylamine (0.15 g, 1.44 mmol), and dichloromethane solution (8 mL) were added into a reaction flask. The mixture was cooled to about -30°C, and trifluoromethanesulfonic anhydride (0.27 g, 0.96 mmol) in dichloromethane (2 mL) was added dropwise. After the addition was completed, the mixture was allowed to warm up naturally and reacted for 1 hour. Dichloromethane (10 mL) was added, and the mixture was washed with saturated sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **1E** (0.24 g, yield: 91%).

Step 5: Synthesis of **1F**

**1E** (0.24 g, 0.44 mmol), 4-[4-(N-Boc)piperazin-1-yl]phenylboronic acid pinacol ester (0.26 g, 0.66 mmol), tetrakis(triphenylphosphine)palladium (0.10 g, 0.088 mmol), sodium carbonate (0.14 g, 1.32 mmol), toluene (6 mL), ethanol (6 mL), and water (3 mL) were added into a reaction flask. The mixture was purged with nitrogen three times, and stirred at 95°C in an oil bath under nitrogen atmosphere overnight. The mixture was cooled to room temperature. Ethyl acetate (30 mL) and water (20 mL) were added, and the mixture was stirred and layered. An appropriate amount of silica gel was added to the organic layer, and the resulting mixture was then concentrated under reduced pressure. The residue was purified by column chromatography with eluent PE-EA = 100-0 to 90-10 to afford **1F** (0.18 g, yield: 62%).

Step 6: Synthesis of **1G**

**1F** (0.18 g, 0.27 mmol) and dichloromethane (3 mL) were added into a reaction flask. Trifluoroacetic acid (2 mL) was added with stirring, and the mixture was stirred at room temperature for 2 hours. The mixture was concentrated to dryness under reduced pressure. Dichloromethane (20 mL) was added, and the mixture was washed once with saturated sodium bicarbonate solution. The organic layer was dried over anhydrous sodium sulfate, filtered, and concentrated to dryness under reduced pressure to afford **1G** (0.15 g, yield: 99%).
LCMS m/z = 562.3 $[M+1]^+$

Step 7: Synthesis of **1H**

**1G** (0.15 g, 0.27 mmol), isobutyraldehyde (0.097 g, 1.34 mmol), and dichloromethane (10 mL) were added into a reaction flask. The mixture was stirred at room temperature for 1 hour. Sodium triacetoxyborohydride (0.17 g, 0.80 mmol) and glacial acetic acid (48 mg, 0.80 mmol) were added, and the mixture was stirred at room temperature overnight. Dichloromethane (10 mL) was added, and the mixture was washed with saturated sodium bicarbonate solution. An appropriate amount of silica gel was added to the organic layer, and the resulting mixture was then concentrated under reduced pressure. The residue was purified by column chromatography with eluent PE-EA = 100-0 to 80-20 to afford **1H** (0.12 g, yield: 72%).
LCMS m/z = 618.3 $[M+1]^+$

Step 8: Synthesis of **Compound 1**

**1H** (0.12 g, 0.19 mmol), lithium hydroxide monohydrate (0.080 g, 1.91 mmol), 1,4-dioxane (3 mL), and water (1 mL) were added into a reaction flask. The mixture was stirred at room temperature overnight. Dichloromethane (30 mL) and water (20 mL) were added, and the pH was adjusted to 2-3 with 1 *N* hydrochloric acid. An appropriate amount of silica gel was added to the organic layer, and the resulting mixture was then concentrated under reduced pressure. The residue was purified by column chromatography with eluent DCM-$CH_3OH$ = 100-0 to 90-10 to afford **Compound 1** (0.09 g, yield: 80%).
LCMS m/z = 590.3 $[M+1]^+$

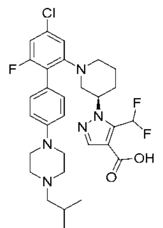
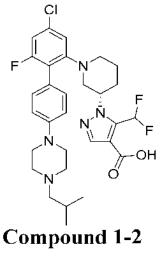

Compound 1-1 and Compound 1-2

**Compound 1** (400 mg) was subjected to chiral separation and purification.

Preparative conditions: instrument: Waters 150 Prep-SFC; chromatographic column: chiral AD column; mobile phase: A for CO2, B for isopropanol (0.1% aqueous ammonia); gradient: B for 30%; flow rate: 120 ml/min; back pressure: 100 bar; column temperature: room temperature; detection wavelength: 220 nm; cycle: 4.5 min.

Analytical conditions: instrument: SHIMADZU LC-30AD SFC; chromatographic column: AD; mobile phase: A for CO2, mobile phase: B for isopropanol (0.05% DEA); gradient: B for 5-40%; flow rate: 3 ml/min; column temperature: 35°C; detection wavelength: 220 nm.

After preparative separation, fractions with the same retention time were combined and concentrated under reduced pressure to afford **Compound 1-1** (116.1 mg) and **Compound 1-2** (151.9 mg).

**Compound 1-1:** retention time under analytical conditions: 1.598 min, LCMS m/z = 590.2 $[M+H]^+$

$^1H$ NMR (400 MHz, $CD_3OD$) δ 7.75 (s, 1H), 7.66 (t, 1H), 7.38 - 7.28 (m, 2H), 7.08 - 6.99 (m, 2H), 6.95 - 6.82 (m, 2H), 4.39 - 4.25 (m, 1H), 3.52 - 3.38 (m, 4H), 3.28 - 3.18 (m, 5H), 3.17 - 3.09 (m, 1H), 3.00 (t, 1H), 2.83 (d, 2H), 2.71 - 2.59 (m, 1H), 2.22 - 2.07 (m, 1H), 2.04 - 1.72 (m, 3H), 1.64 - 1.45 (m, 1H), 1.06 (d, 6H).

**Compound 1-2:** retention time under analytical conditions: 1.959 min, LCMS m/z = 590.2 $[M+H]^+$

$^1H$ NMR (400 MHz, $CD_3OD$) δ 7.75 (s, 1H), 7.66 (t, 1H), 7.37 - 7.28 (m, 2H), 7.07 - 7.00 (m, 2H), 6.95 - 6.83 (m, 2H), 4.41 - 4.24 (m, 1H), 3.52 - 3.38 (m, 4H), 3.28 - 3.17 (m, 5H), 3.17 - 3.08 (m, 1H), 3.06 - 2.95 (m, 1H), 2.82 (d, 2H), 2.72 - 2.59 (m, 1H), 2.21 - 2.06 (m, 1H), 2.04 - 1.70 (m, 3H), 1.64 - 1.47 (m, 1H), 1.06 (d, 6H).

**Example 2:**

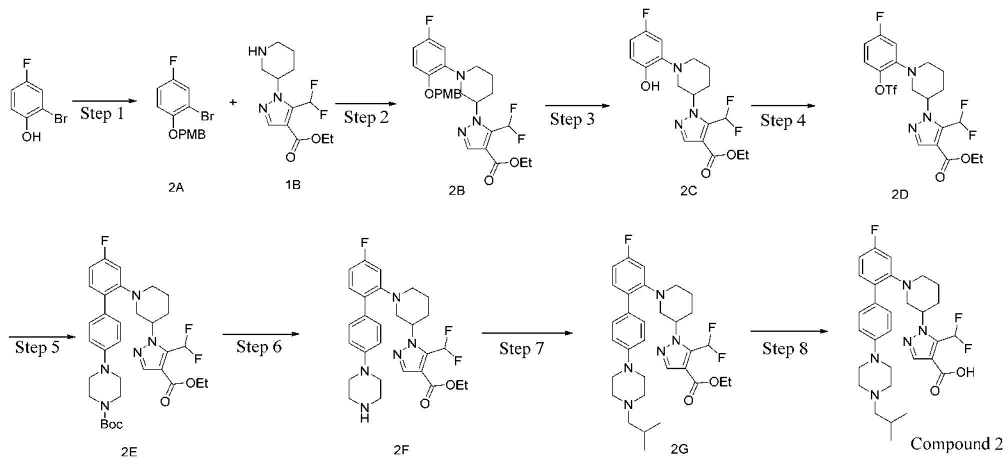


Step 1: Synthesis of **2A**

2-Bromo-4-fluorophenol (3.3 g, 17.32 mmol), 4-methoxybenzyl chloride (2.71 g, 17.32 mmol), potassium carbonate (7.18 g, 51.96 mmol), and acetone (50 mL) were added into a reaction flask. The mixture was heated at 70°C in an oil bath overnight and then cooled to room temperature. The mixture was filtered, an appropriate amount of silica gel was added to the filtrate, and the resulting mixture was then concentrated under reduced pressure. The residue was

purified by column chromatography with eluent PE-EA = 100-0 to 90-10 to afford **2A** (3.8 g, yield: 71%).

Step 2: Synthesis of **2B**

**2A** (0.75 g, 2.41 mmol), **1B** (0.66 g, 2.41 mmol), $Pd_2DBa_3$ (0.22 g, 0.24 mmol), (R)-SEGPHOS (0.29 g, 0.48 mmol), cesium carbonate (2.36 g, 7.25 mmol), and 1,4-dioxane (15 mL) were added into a reaction flask. The mixture was purged with nitrogen three times, and heated at 100°C in an oil bath overnight and then cooled to room temperature. The mixture was filtered, an appropriate amount of silica gel was added to the filtrate, and the resulting mixture was then concentrated under reduced pressure. The residue was purified by column chromatography with eluent PE-EA = 100-0 to 90-10 to afford **2B** (0.45 g, yield: 37.08%).
LCMS m/z = 504.2 $[M+1]^+$

Step 3: Synthesis of **2C**

**2B** (0.45 g, 0.89 mmol) and dichloromethane (5 mL) were added into a reaction flask. Trifluoroacetic acid (3 mL) was added with stirring, and the mixture was stirred at room temperature overnight. The mixture was concentrated to dryness under reduced pressure. Dichloromethane (20 mL) was added, and the organic phase was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **2C** (0.32 g, yield: 94%).

Step 4: Synthesis of **2D**

**2C** (0.32 g, 0.83 mmol), triethylamine (0.26 g, 2.52 mmol), and dichloromethane solution (8 mL) were added into a reaction flask. The mixture was cooled to about -30°C, and trifluoromethanesulfonic anhydride (0.47 g, 1.67 mmol) in dichloromethane (2 mL) was added dropwise. After the addition was completed, the mixture was allowed to warm up naturally and reacted for 1 hour. Dichloromethane (10 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **2D** (0.31 g, yield: 72%).
LCMS m/z = 516.2 $[M+1]^+$

Step 5: Synthesis of **2E**

**2D** (0.31 g, 0.60 mmol), 4-[4-(N-Boc)piperazin-1-yl]phenylboronic acid pinacol ester (0.35 g, 0.90 mmol), tetrakis(triphenylphosphine)palladium (0.14 g, 0.12 mmol), sodium carbonate (0.19 g, 1.79 mmol), toluene (6 mL), ethanol (6 mL), and water (3 mL) were added into a reaction flask. The mixture was purged with nitrogen three times, and stirred at 95°C in an oil bath under nitrogen atmosphere overnight. The mixture was cooled to room temperature. Ethyl acetate (30 mL) and water (20 mL) were added, and the mixture was stirred and layered. An appropriate amount of silica gel was added to the organic layer, and the resulting mixture was then concentrated under reduced pressure. The residue was purified by column chromatography with eluent PE-EA = 100-0 to 80-20 to afford **2E** (0.06 g, yield: 16%).
LCMS m/z = 628.3 $[M+1]^+$

Step 6: Synthesis of **2F**

**2E** (62 mg, 0.096 mmol) and dichloromethane (3 mL) were added into a reaction flask. Trifluoroacetic acid (1 mL) was added with stirring, and the mixture was stirred at room temperature for 2 hours. The mixture was concentrated to dryness under reduced pressure. Dichloromethane (20 mL) was added, and the mixture was washed once with saturated aqueous sodium bicarbonate solution. The organic layer was dried over anhydrous sodium sulfate, filtered, and concentrated to dryness under reduced pressure to afford **2F** (40 mg, yield: 77%).

Step 7: Synthesis of **2G**

**2F** (40 mg, 0.076 mmol), isobutyraldehyde (28 mg, 0.38 mmol), and dichloromethane (5 mL) were added into a reaction flask. The mixture was stirred at room temperature for 1 hour. Sodium triacetoxyborohydride (48 mg, 0.23 mmol) and glacial acetic acid (23 mg, 0.23 mmol) were added, and the mixture was stirred at room temperature overnight. Dichloromethane (10 mL) was added, and the mixture was washed with saturated aqueous sodium bicarbonate solution. An appropriate amount of silica gel was added to the organic layer, and the resulting mixture was then concentrated under

reduced pressure. The residue was purified by column chromatography with eluent PE-EA = 100-0 to 80-20 to afford **2G** (30 mg, yield: 68%).
LCMS m/z = 584.3 $[M+1]^+$

Step 8: Synthesis of **Compound 2**

**2G** (30 mg, 0.051 mmol), lithium hydroxide monohydrate (21 mg, 0.51 mmol), 1,4-dioxane (3 mL), and water (1 mL) were added into a reaction flask. The mixture was stirred at room temperature overnight. Dichloromethane (30 mL) and water (20 mL) were added, and the pH was adjusted to 2-3 with 1 *N* hydrochloric acid. An appropriate amount of silica gel was added to the organic layer, and the resulting mixture was then concentrated under reduced pressure. The residue was purified by column chromatography with eluent DCM-CH3OH = 100-0 to 90-10 to afford **Compound 2** (10 mg, yield: 35%).
LCMS m/z = 556.3 $[M+1]^+$

$^1$H NMR (400 MHz, DMSO-$d_6$) δ 7.95 (s, 1H), 7.50 (t, 1H), 7.34 (d, 2H), 7.31 - 7.21 (m, 1H), 7.07 (d, 2H), 6.99 - 6.85 (m, 2H), 4.40-4.30 (m, 1H), 4.01 - 3.74 (m, 2H), 3.69 - 3.53 (m, 2H), 3.25 - 3.09 (m, 5H), 3.08 - 2.97 (m, 4H), 2.62 - 2.52 (m, 1H), 2.21 - 2.08 (m, 1H), 2.05 - 1.80 (m, 2H), 1.76 - 1.59 (m, 1H), 1.47 - 1.31 (m, 1H), 1.00 (d, 6H).

**Example 3:**

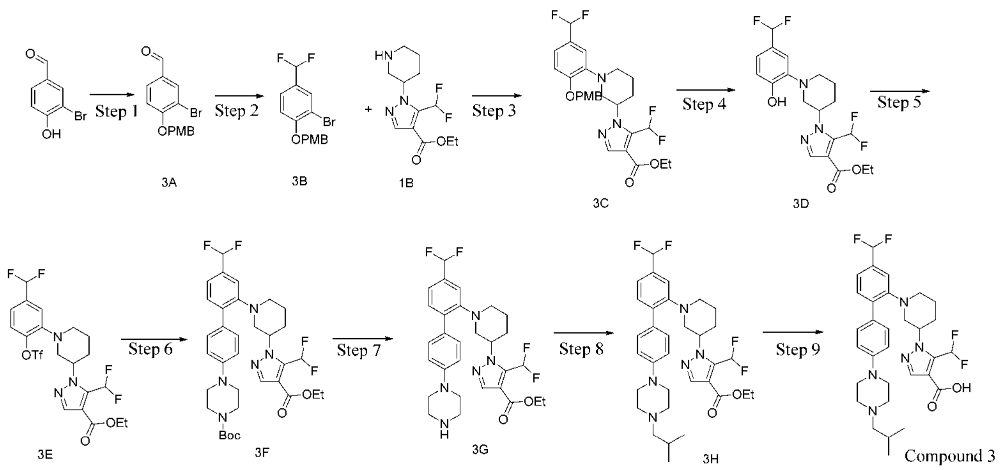


Step 1: Synthesis of **3A**

3-Bromo-4-hydroxybenzaldehyde (3 g, 14.89 mmol), 4-methoxybenzyl chloride (2.45 g, 15.63 mmol), potassium carbonate (4.12 g, 29.71 mmol), and DMF (30 mL) were added into a reaction flask. The mixture was heated at 50°C in an oil bath for 4 hours and then cooled to room temperature. Ethyl acetate (100 mL) was added, and the mixture was washed with saturated aqueous sodium chloride solution. An appropriate amount of silica gel was added to the organic layer, and the resulting mixture was then concentrated under reduced pressure. The residue was purified by column chromatography with eluent PE-EA = 100-0 to 90-10 to afford **3A** (4.5 g, yield: 94%).

Step 2: Synthesis of **3B**

**3A** (4.30 g, 13.39 mmol) and dichloromethane (40 mL) were added into a reaction flask. DAST (4.32 g, 26.78 mmol) in dichloromethane (10 mL) was added dropwise while the mixture was cooled in an ice-water bath. After the addition was completed, the mixture was stirred at room temperature for 5 hours. Methanol (10 mL) was added dropwise while the mixture was cooled in an ice-water bath to quench the reaction. The reaction solution was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **3B** (4 g, yield: 87%).

Step 3: Synthesis of **3C**

**3B** (1 g, 2.91 mmol), **1B** (0.80 g, 2.91 mmol), $Pd_2DBa_3$ (0.27 g, 0.29 mmol), (R)-SEGPHOS (0.36 g, 0.58 mmol), cesium carbonate (2.84 g, 8.73 mmol), and 1,4-dioxane (20 mL) were added into a reaction flask. The mixture was purged with nitrogen three times, and heated at 100°C in an oil bath overnight and then cooled to room temperature. The mixture was filtered, an appropriate amount of silica gel was added to the filtrate, and the resulting mixture was then concentrated under reduced pressure. The residue was purified by column chromatography with eluent PE-EA = 100-0 to 90-10 to afford **3C** (0.7 g, yield: 45%).

Step 4: Synthesis of **3D**

**3C** (0.7 g, 1.31 mmol) and dichloromethane (5 mL) were added into a reaction flask. Trifluoroacetic acid (3 mL) was added with stirring, and the mixture was stirred at room temperature overnight. The mixture was concentrated to dryness under reduced pressure. Dichloromethane (20 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **3D** (0.3 g, yield: 55%).

Step 5: Synthesis of **3E**

**3D** (0.3 g, 0.72 mmol), triethylamine (0.22 g, 2.17 mmol), and dichloromethane solution (8 mL) were added into a reaction flask. The mixture was cooled to about -30°C, and trifluoromethanesulfonic anhydride (0.41 g, 1.45 mmol) in dichloromethane (2 mL) was added dropwise. After the addition was completed, the mixture was allowed to warm up naturally and reacted for 1 hour. Dichloromethane (10 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **3E** (0.28 g, yield: 71%).

Step 6: Synthesis of **3F**

**3E** (0.28 g, 0.51 mmol), 4-[4-(N-Boc)piperazin-1-yl]phenylboronic acid pinacol ester (0.30 g, 0.77 mmol), tetrakis(triphenylphosphine)palladium (0.12 g, 0.10 mmol), sodium carbonate (0.16 g, 1.53 mmol), toluene (6 mL), ethanol (6 mL), and water (3 mL) were added into a reaction flask. The mixture was purged with nitrogen three times, and stirred at 95°C in an oil bath under nitrogen atmosphere overnight. The mixture was cooled to room temperature. Ethyl acetate (30 mL) and water (20 mL) were added, and the mixture was stirred and layered. An appropriate amount of silica gel was added to the organic layer, and the resulting mixture was then concentrated under reduced pressure. The residue was purified by column chromatography with eluent PE-EA = 100-0 to 80-20 to afford **3F** (0.25 g, yield: 74%).

Step 7: Synthesis of **3G**

**3F** (0.25 g, 0.38 mmol) and dichloromethane (5 mL) were added into a reaction flask. Trifluoroacetic acid (3 mL) was added with stirring, and the mixture was stirred at room temperature for 2 hours. The mixture was concentrated to dryness under reduced pressure. Dichloromethane (20 mL) was added, and the mixture was washed once with saturated aqueous sodium bicarbonate solution. The organic layer was dried over anhydrous sodium sulfate, filtered, and concentrated to dryness under reduced pressure to afford **3G** (0.18 g, yield: 85%).
LCMS m/z = 560.3 $[M+1]^+$

Step 8: Synthesis of **3H**

**3G** (0.18 g, 0.32 mmol), isobutyraldehyde (0.12 g, 1.66 mmol), and dichloromethane (10 mL) were added into a reaction flask. The mixture was stirred at room temperature for 1 hour. Sodium triacetoxyborohydride (0.20 g, 0.94 mmol) and glacial acetic acid (0.058 g, 0.97 mmol) were added, and the mixture was stirred at room temperature overnight. Dichloromethane (10 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 80-20 to afford **3H** (0.18 g, yield: 81%).
LCMS m/z = 616.4 $[M+1]^+$

Step 9: Synthesis of **Compound 3**

**3H** (0.16 g, 0.26 mmol), lithium hydroxide monohydrate (0.11 g, 2.6 mmol), 1,4-dioxane (3 mL), and water (1 mL) were added into a reaction flask. The mixture was stirred at room temperature overnight. Dichloromethane (30 mL) and water (20 mL) were added, and the pH was adjusted to 2-3 with 1 *N* hydrochloric acid. An appropriate amount of silica gel was added to the organic layer, and the resulting mixture was then concentrated under reduced pressure. The residue was purified by column chromatography with eluent DCM-$CH_3OH$ = 100-0 to 90-10 to afford **Compound 3** (0.12 g, yield: 79%). LCMS m/z = 588.3 $[M+1]^+$

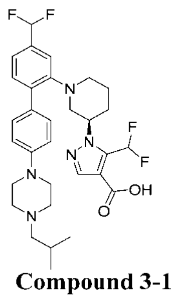

**Compound 3-1**

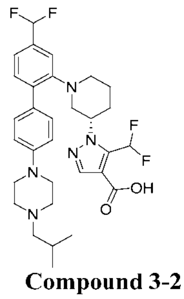

**Compound 3-2**

**Compound 3** (350 mg) was subjected to chiral separation and purification.

Preparative conditions: instrument: Waters 150 Prep-SFC; chromatographic column: chiral AD column; mobile phase: A for $CO_2$, B for isopropanol (0.1% aqueous ammonia); gradient: B for 35%; flow rate: 110 ml/min; back pressure: 100 bar; column temperature: room temperature; detection wavelength: 220 nm; cycle: 3 min.

Analytical conditions: instrument: SHIMADZU LC-30AD SFC; chromatographic column: AD; mobile phase: A for $CO_2$, mobile phase: B for isopropanol (0.05% DEA); gradient: B for 5-40%; flow rate: 3 ml/min; column temperature: 35°C; detection wavelength: 220 nm. After preparative separation, fractions with the same retention time were combined and concentrated under reduced pressure to afford **Compound 3-1** (126.1 mg) and **Compound 3-2** (178.4 mg).

**Compound 3-1:** retention time under analytical conditions: 1.592 min, LCMS m/z = 588.3 $[M+H]^+$

$^1H$ NMR (400 MHz, $CD_3OD$) δ 7.76 (s, 1H), 7.67 (t, 1H), 7.53 - 7.47 (m, 2H), 7.29 - 7.18 (m, 3H), 7.08 - 7.00 (m, 2H), 6.72 (t, 1H), 4.53 - 4.38 (m, 1H), 3.50 - 3.38 (m, 4H), 3.27 - 3.11 (m, 6H), 3.06 - 2.97 (m, 1H), 2.78 (d, 2H), 2.76 - 2.64 (m, 1H), 2.20 - 2.06 (m, 1H), 2.06 - 1.77 (m, 3H), 1.74 - 1.58 (m, 1H), 1.05 (d, 6H).

**Compound 3-2:** retention time under analytical conditions: 1.918 min, LCMS m/z = 588.3 $[M+H]^+$

$^1H$ NMR (400 MHz, $CD_3OD$) δ 7.75 (s, 1H), 7.68 (t, 1H), 7.52 (d, 2H), 7.33 - 7.18 (m, 3H), 7.05 (d, 2H), 6.73 (t, 1H), 4.55 - 4.39 (m, 1H), 3.49 - 3.38 (m, 4H), 3.28 - 3.10 (m, 6H), 3.01 (t, 1H), 2.77 (d, 2H), 2.74 - 2.65 (m, 1H), 2.19 - 2.07 (m, 1H), 2.07 - 1.77 (m, 3H), 1.74 - 1.56 (m, 1H), 1.05 (d, 6H).

**Example 4:**

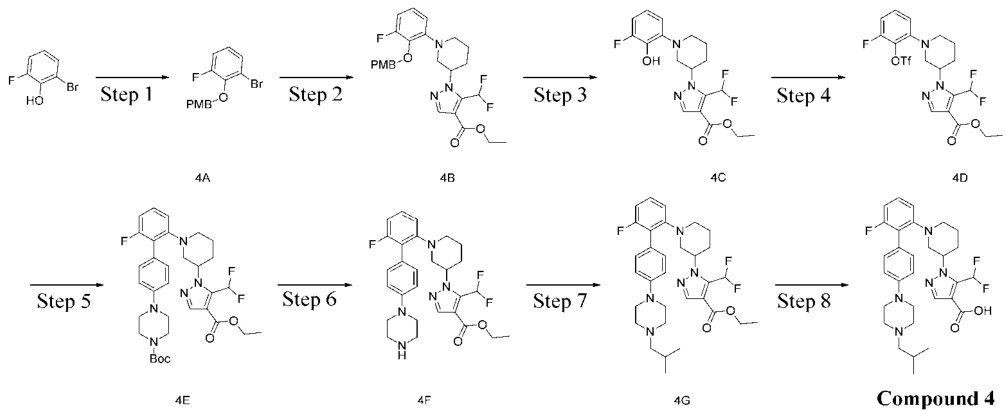


4A 4B 4C 4D

4E 4F 4G **Compound 4**

Step 1: Preparation of **4A**

2-Bromo-6-fluorophenol (3.3 g, 17.32 mmol), 4-methoxybenzyl chloride (2.71 g, 17.32 mmol), and potassium carbonate (7.18 g, 52.04 mmol) were added into acetone (50 mL). After the addition was completed, the mixture was stirred at 70°C in an oil bath overnight. The mixture was cooled to room temperature and filtered. The filtrate was concentrated under reduced pressure. The residue was purified by column chromatography (petroleum ether/ethyl acetate (V/V) = 1/0 to 10/1) to afford Compound **4A** (3.8 g, yield: 70%).

Step 2: Preparation of **4B**

Compound **4A** (1 g, 3.21 mmol) and **1B** (0.92 g, 3.37 mmol) were added into 1,4-dioxane (15 mL). $Pd_2(dba)_3$ (0.29 g, 0.32 mmol), 4,4'-bis-1,3-benzodioxol-5,5'-diylbis(diphenylphosphine) ((R)-SEGPHOS) (0.39 g, 0.64 mmol), and cesium carbonate (2.61 g, 8.01 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 2/1) to afford Compound **4B** (0.54 g, yield: 33%).
LCMS m/z = 504.3 $[M+H]^+$

Step 3: Preparation of **4C**

**4B** (0.54 g, 1.07 mmol) was dissolved in DCM (10 mL), and TFA (0.61 g, 5.35 mmol) was added. The mixture was stirred at room temperature overnight. Most of the solvent was concentrated under reduced pressure. The residue was redissolved in ethyl acetate, washed once with water, washed once with saturated aqueous sodium bicarbonate solution, and finally washed once with saturated brine. The resulting mixture was dried over anhydrous sodium sulfate and concentrated under reduced pressure. The residue was purified by column chromatography (mobile phase: petroleum ether/ethyl acetate (V/V) = 1/0 to 2/1) to afford Compound **4C** (0.38 g, yield: 92%).
LCMS m/z = 384.4 $[M+H]^+$

Step 4: Preparation of **4D**

**4C** (0.38 g, 0.99 mmol) was dissolved in DCM (5 mL). Triethylamine (0.30 g, 2.97 mmol) was added, and trifluoromethanesulfonic anhydride (0.56 g, 1.98 mmol) was added dropwise in an ice-water bath. The mixture was stirred at room temperature overnight. Dichloromethane and water were added, and the mixture was stirred and layered. The organic phase was washed with saturated brine, dried over anhydrous sodium sulfate, and concentrated under reduced pressure. The residue was purified by column chromatography on silica gel (mobile phase: petroleum ether/ethyl acetate (V/V) = 1/0 to 10/1) to afford Compound **4D** (0.45 g, yield: 88%).
LCMS m/z = 516.1 $[M+H]^+$

Step 5: Preparation of **4E**

**4D** (0.22 g, 0.43 mmol) and 4-[4-(N-Boc)piperazin-1-yl]phenylboronic acid pinacol ester (0.25 g, 0.65 mmol)

were dissolved in toluene (3 mL), ethanol (3 mL), and water (1 mL). Tetrakis(triphenylphosphine)palladium (0.099 g, 0.086 mmol) and sodium carbonate (0.14 g, 1.29 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **4E** (0.15 g, yield: 55%).
LCMS m/z = 628.3 $[M+H]^+$

Step 6: Preparation of **4F**

**4E** (0.15 g, 0.24 mmol) was dissolved in 4 M HCl/1,4-dioxane solution (5 mL), and the mixture was stirred at room temperature for 1 h. The mixture was concentrated under reduced pressure. Ethyl acetate and saturated aqueous sodium bicarbonate solution were added, and the mixture was stirred and layered. The organic phase was washed with saturated brine, dried over anhydrous sodium sulfate, and concentrated under reduced pressure to afford Compound 4F (0.12 g, yield: 94%).
LCMS m/z = 528.6 $[M+H]^+$

Step 7: Preparation of **4G**

**4F** (0.12 g, 0.23 mmol) and isobutyraldehyde (0.033 g, 0.46 mmol) were dissolved in DCM (5 mL). Glacial acetic acid (0.055 g, 0.92 mmol) was added, and the mixture was stirred at room temperature for 10 min. Sodium triacetoxyborohydride (0.15 g, 0.71 mmol) was added, and the mixture was reacted at room temperature overnight. Ethyl acetate and saturated aqueous sodium bicarbonate solution were added, and the mixture was stirred and layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **4G** (0.12 g, yield: 89%).
LCMS m/z = 584.3 $[M+H]^+$

Step 8: Preparation of **Compound 4**

Compound **4G** (0.12 g, 0.21 mmol) was dissolved in a mixed solvent of 1,4-dioxane (3 mL) and water (1.5 mL). Lithium hydroxide (0.088 g, 3.67 mmol) was added, and the mixture was stirred at room temperature for 3 h. The pH of the reaction solution was adjusted to 3-4 with 1 *N* aqueous hydrochloric acid. Ethyl acetate (20 mL) was added, and the organic phase was washed with saturated brine (10 mL), dried over anhydrous sodium sulfate, and concentrated under reduced pressure. The residue was purified by column chromatography (dichloromethane/methanol (V/V) = 1/0 to 10/1) to afford **Compound 4** (90 mg, yield: 77%).
LCMS m/z = 556.2 $[M+H]^+$

$^1$H NMR (400 MHz, DMSO-$d_6$) $\delta$ 7.95 (s, 1H), 7.52 (t, 1H), 7.37 - 7.23 (m, 3H), 7.07 (d, 2H), 6.97 - 6.87 (m, 2H), 4.44 - 4.27 (m, 1H), 3.91 - 3.80 (m, 2H), 3.66 - 3.56 (m, 2H), 3.24 - 3.10 (m, 5H), 3.08 - 2.97 (m, 4H), 2.60 - 2.52 (m, 1H), 2.22 - 2.09 (m, 1H), 2.01 - 1.80 (m, 2H), 1.74 - 1.62 (m, 1H), 1.44 - 1.36 (m, 1H), 1.02 (d, 6H).

**Example 5:**

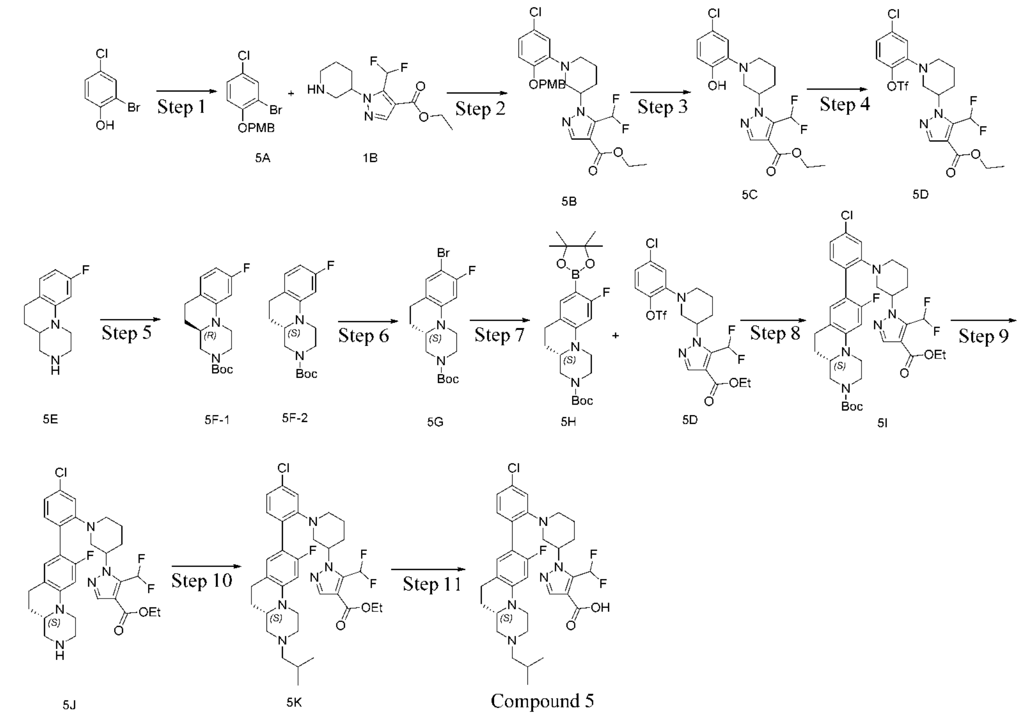


Step 1: Synthesis of **5A**

2-Bromo-4-chlorophenol (25 g, 120.54 mmol), 4-methoxybenzyl chloride (18.88 g, 120.54 mmol), potassium carbonate (33.32 g, 241.08 mmol), and acetone (300 mL) were added into a reaction flask. The mixture was heated at 70°C in an oil bath overnight and then cooled to room temperature. The mixture was filtered, an appropriate amount of silica gel was added to the filtrate, and the resulting mixture was then concentrated under reduced pressure. The residue was purified by column chromatography with eluent PE-EA = 100-0 to 90-10 to afford **5A** (30 g, yield: 76%).

Step 2: Synthesis of **5B**

**5A** (12 g, 36.63 mmol), **1B** (10.1 g, 36.63 mmol), $Pd_2DBa_3$ (3.35 g, 3.66 mmol), (R)-SEGPHOS (4.47 g, 7.33 mmol), cesium carbonate (35.80 g, 109.93 mmol), and 1,4-dioxane (150 mL) were added into a reaction flask. The mixture was purged with nitrogen three times, and heated at 100°C in an oil bath overnight and then cooled to room temperature. An appropriate amount of silica gel was added, and the resulting mixture was then concentrated under reduced pressure. The residue was purified by column chromatography with eluent PE-EA = 100-0 to 90-10 to afford **5B** (10 g, yield: 53%).

Step 3: Synthesis of **5C**

**5B** (10 g, 19.23 mmol) and dichloromethane (50 mL) were added into a reaction flask. Trifluoroacetic acid (40 mL) was added with stirring, and the mixture was stirred at room temperature overnight. The mixture was concentrated to dryness under reduced pressure. Dichloromethane (100 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **5C** (6.5 g, yield: 85%).

Step 4: Synthesis of **5D**

**5C** (5 g, 12.51 mmol), triethylamine (3.80 g, 37.53 mmol), and dichloromethane solution (50 mL) were added into a reaction flask. The mixture was cooled to about -30°C, and trifluoromethanesulfonic anhydride (4.1 g, 14.5 mmol) in dichloromethane (10 mL) was added dropwise. After the addition was completed, the mixture was allowed to warm up naturally and reacted for 1 hour. The mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 90-10 to afford **5D** (4.60 g, yield: 69%).

Step 5: Synthesis of **5F**

Compound **5E** (2.8 g, 13.58 mmol) (see *Bioorganic & Medicinal Chemistry Letters*, **2016**, *26*, 5877-5882 for its synthesis method) was dissolved in dichloromethane (50 mL). $Boc_2O$ (5.93 g, 27.17 mmol) and DMAP (3.32 g, 27.18 mmol) were added. The mixture was reacted at room temperature for 16 h. The reaction system was washed with 0.5 mol/L hydrochloric acid (50 mL), dried over anhydrous sodium sulfate, and concentrated under reduced pressure. The crude was separated and purified by column chromatography on silica gel (ethyl acetate : petroleum ether (v/v) = 0 : 1 to 1 : 9) to afford the racemate of Compound **5F** (3.4 g, yield: 82%).

The racemate of **5F** was subjected to chiral resolution, and the chiral resolution method was as follows.

1. Instrument: SFC Prep 150 AP; chromatographic column: Daicel IC-H (19 mm × 250 mm).
2. The sample was dissolved in methanol and filtered through a 0.45 μm filter head to prepare a sample solution.
3. Preparative chromatography conditions: a. the mobile phase consists of systems A and B: mobile phase A: $CO_2$; mobile phase B: methanol/isopropanol (v/v) = 1 : 1; b. isocratic elution, mobile phase B: 20%; c. flow rate: 40 mL/min.

Retention time: chiral isomer 1 (Compound **5F-1**): 5.7 min, chiral isomer 2 (Compound **5F-2**): 6.47 min.

According to the MicroED structure determination, Compound **5F-1** is in the R configuration, and Compound **5F-2** is in the S configuration.

LCMS m/z = 307.3 $[M+1]^+$

Step 6: Synthesis of **5G**

**5F-2** (3 g, 9.79 mmol) and acetonitrile (40 mL) were added into a reaction flask. NBS (1.83 g, 10.28 mmol) was added portionwise with stirring. After the addition was completed, the mixture was stirred at room temperature for 1 hour. An appropriate amount of silica gel was added, and the resulting mixture was then concentrated under reduced pressure. The residue was purified by column chromatography with eluent PE-EA = 100-0 to 90-10 to afford **5G** (1.9 g, yield: 50%).

Step 7: Synthesis of **5H**

**5G** (0.8 g, 2.08 mmol), bis(pinacolato)diboron (1.06 g, 4.18 mmol), $Pd(dppf)Cl_2$ (0.30 g, 0.42 mmol), potassium acetate (0.61 g, 6.22 mmol), and 1,4-dioxane (15 mL) were added into a reaction flask. The mixture was purged with nitrogen three times, and heated at 95°C in an oil bath under nitrogen atmosphere overnight and then cooled to room temperature. An appropriate amount of silica gel was added, and the resulting mixture was then concentrated under reduced pressure. The residue was purified by column chromatography with eluent PE-EA = 100-0 to 90-10 to afford **5H** (0.47 g, yield: 52%).

Step 8: Synthesis of **5I**

**5D** (0.38 g, 0.71 mmol), **5H** (0.46 g, 1.06 mmol), tetrakis(triphenylphosphine)palladium (0.16 g, 0.14 mmol), sodium carbonate (0.23 g, 2.13 mmol), toluene (6 mL), ethanol (6 mL), and water (3 mL) were added into a reaction flask. The mixture was purged with nitrogen three times, and stirred at 95°C in an oil bath under nitrogen atmosphere overnight. The mixture was cooled to room temperature. Ethyl acetate (30 mL) and water (20 mL) were added, and the mixture was stirred and layered. An appropriate amount of silica gel was added to the organic layer, and the resulting mixture was then concentrated under reduced pressure. The residue was purified by column chromatography with eluent PE-EA = 100-0 to 90-10 to afford **5I** (0.37 g, yield: 61%).

LCMS m/z = 688.3 $[M+1]^+$

Step 9: Synthesis of **5J**

**5I** (0.37 g, 0.54 mmol) and dichloromethane (5 mL) were added into a reaction flask. Trifluoroacetic acid (3 mL) was added with stirring, and the mixture was stirred at room temperature for 2 hours. The mixture was concentrated to dryness under reduced pressure. Dichloromethane (20 mL) was added, and the mixture was washed once with saturated aqueous sodium bicarbonate solution. The organic layer was dried over anhydrous sodium sulfate, filtered, and concentrated to dryness under reduced pressure to afford **5J** (0.29 g).

Step 10: Synthesis of **5K**

**5J** (0.29 g, 0.49 mmol), isobutyraldehyde (0.17 g, 2.41 mmol), and dichloromethane (10 mL) were added into a

reaction flask. The mixture was stirred at room temperature for 1 hour. Sodium triacetoxyborohydride (0.31 g, 1.47 mmol) and glacial acetic acid (0.088 g, 1.47 mmol) were added, and the mixture was stirred at room temperature for about 48 hours. Dichloromethane (10 mL) was added, and the mixture was washed with saturated aqueous sodium bicarbonate solution. An appropriate amount of silica gel was added to the organic layer, and the resulting mixture was then concentrated under reduced pressure. The residue was purified by column chromatography with eluent PE-EA = 100-0 to 80-20 to afford **5K** (0.17 g).
LCMS m/z = 644.4 $[M+1]^+$

Step 11: Synthesis of **Compound 5**

**5K** (0.17 g, 0.26 mmol), lithium hydroxide monohydrate (0.11 g, 2.6 mmol), 1,4-dioxane (3 mL), and water (1 mL) were added into a reaction flask. The mixture was stirred at room temperature overnight. Dichloromethane (30 mL) and water (20 mL) were added, and the pH was adjusted to 2-3 with 1 *N* hydrochloric acid. An appropriate amount of silica gel was added to the organic layer, and the resulting mixture was then concentrated under reduced pressure. The residue was purified by column chromatography with eluent DCM-$CH_3OH$ = 100-0 to 90-10 to afford **Compound 5** (0.08 g).
LCMS m/z = 616.3 $[M+1]^+$

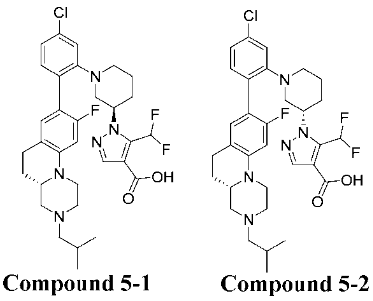

**Compound 5-1** **Compound 5-2**

**Compound 5** (400 mg) was subjected to chiral separation and purification.

Preparative conditions: instrument: Waters 150 Prep-SFC; chromatographic column: chiral AD column; mobile phase: A for $CO_2$, B for isopropanol (0.1% aqueous ammonia); gradient: B for 50%; flow rate: 100 ml/min; back pressure: 100 bar; column temperature: room temperature; detection wavelength: 220 nm; cycle: 2.9 min.

Analytical conditions: instrument: SHIMADZU LC-30AD SFC; chromatographic column: chiral AD column; mobile phase: A for $CO_2$, mobile phase: B for isopropanol (0.05% DEA); gradient: B for 5-40%; flow rate: 3 mL/min; column temperature: 35°C; detection wavelength: 220 nm.

After preparative separation, fractions with the same retention time were combined and concentrated under reduced pressure to afford Compound 5-1 (174 mg) and Compound 5-2 (191.4 mg).

**Compound 5-1**: retention time under analytical conditions: 1.755 min, LCMS m/z = 616.6 $[M+H]^+$

$^1H$ NMR (400 MHz, $CD_3OD/CDCl_3$(v/v)=1/1) δ 7.86 (s, 1H), 7.59 (t, 1H), 7.18 - 7.10 (m, 1H), 7.09 - 6.97 (m, 3H), 6.59 (d, 1H), 4.50 - 4.37 (m, 1H), 3.80 (d, 1H), 3.32 - 3.20 (m, 3H), 3.20 - 3.02 (m, 4H), 2.91 - 2.79 (m, 1H), 2.78 - 2.62 (m, 2H), 2.61 - 2.43 (m, 3H), 2.25 (t, 1H), 2.10 - 1.89 (m, 4H), 1.87 - 1.72 (m, 2H), 1.64 - 1.46 (m, 1H), 1.02 (d, 6H).

**Compound 5-2:** retention time under analytical conditions: 2.284 min, LCMS m/z = 616.7 $[M+H]^+$

$^1H$ NMR (400 MHz, $CD_3OD/CDCl_3$(v/v)=1/1) δ 7.86 (s, 1H), 7.62 (t, 1H), 7.18 - 6.99 (m, 4H), 6.59 (d, 1H), 4.50 - 4.35 (m, 1H), 3.88 - 3.70 (m, 1H), 3.34 - 3.26 (m, 2H), 3.26 - 3.00 (m, 5H), 2.97 - 2.83 (m, 1H), 2.79 - 2.63 (m, 2H), 2.61 - 2.43 (m, 3H), 2.26 (t, 1H), 2.12 - 1.89 (m, 4H), 1.87 - 1.69 (m, 2H), 1.64 - 1.46 (m, 1H), 1.09 - 0.98 (m, 6H).

**Example 6:**

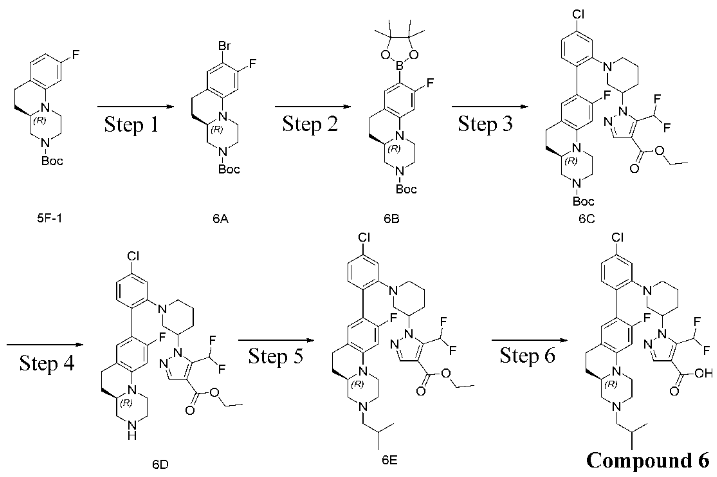


5F-1 6A 6B 6C

6D 6E Compound 6

Step 1: Preparation of **6A**

Compound **5F-1** (1.4 g, 4.57 mmol) was dissolved in acetonitrile (50 ml). NBS (0.81 g, 4.57 mmol) was added, and the mixture was stirred for 1 hour. Then, the reaction solution was concentrated under reduced pressure. The residue was separated and purified by column chromatography on silica gel (ethyl acetate : petroleum ether (V : V) = 0 : 1 to 1 : 9) to afford **6A** (1.6 g, yield: 91%).

Step 2: Preparation of **6B**

Compound **6A** (0.6 g, 1.56 mmol) and bis(pinacolato)diboron (0.59 g, 2.35 mmol) were dissolved in 1,4-dioxane (10 mL). [1,1'-Bis(diphenylphosphino)ferrocene]dichloropalladium (0.23 g, 0.31 mmol) and potassium acetate (0.46 g, 4.69 mmol) were added. The mixture was purged with nitrogen three times, and heated to 100°C and stirred overnight under nitrogen atmosphere. The mixture was cooled to room temperature. Ethyl acetate and water were added, and the mixture was stirred and layered. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 5/1) to afford Compound **6B** (0.45 g, yield: 66%).
LCMS m/z = 433.3 $[M+H]^+$

Step 3: Preparation of **6C**

**5D** (0.22 g, 0.41 mmol) and **6B** (0.27 g, 0.61 mmol) were dissolved in toluene (3 mL), ethanol (3 mL), and water (1 mL). Tetrakis(triphenylphosphine)palladium (0.095 g, 0.082 mmol) and sodium carbonate (0.13 g, 1.23 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **6C** (0.23 g, yield: 81%).
LCMS m/z = 688.3 $[M+H]^+$

Step 4: Preparation of **6D**

**6C** (0.23 g, 0.33 mmol) was dissolved in 4 M HCl/1,4-dioxane solution (5 mL), and the mixture was stirred at room temperature for 1 h. The mixture was concentrated under reduced pressure. Ethyl acetate and saturated aqueous sodium bicarbonate solution were added, and the mixture was stirred and layered. The organic phase was washed with saturated brine, dried over anhydrous sodium sulfate, and concentrated under reduced pressure to afford Compound **6D** (0.18 g, yield: 92%).
LCMS m/z = 588.2 $[M+H]^+$

Step 5: Preparation of **6E**

**6D** (0.18 g, 0.31 mmol) and isobutyraldehyde (0.045 g, 0.62 mmol) were dissolved in DCM (5 mL). Glacial acetic

acid (0.074 g, 1.24 mmol) was added, and the mixture was stirred at room temperature for 10 min. Sodium triacetoxyborohydride (0.2 g, 0.93 mmol) was added, and the mixture was reacted at room temperature overnight. Ethyl acetate and saturated aqueous sodium bicarbonate solution were added, and the mixture was stirred and layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **6E** (0.17 g, yield: 85%).
LCMS m/z = 644.3 $[M+H]^+$

Step 6: Preparation of **Compound 6**

Compound **6E** (0.17 g, 0.26 mmol) was dissolved in a mixed solvent of 1,4-dioxane (3 mL) and water (1.5 mL). Lithium hydroxide (0.11 g, 2.59 mmol) was added, and the mixture was stirred at room temperature for 3 h. The pH of the reaction solution was adjusted to 3-4 with 1 *N* aqueous hydrochloric acid. Ethyl acetate (20 mL) was added, and the organic phase was washed with saturated brine (10 mL), dried over anhydrous sodium sulfate, and concentrated under reduced pressure. The residue was purified by column chromatography (dichloromethane/methanol (V/V) = 1/0 to 10/1) to afford **Compound 6** (0.14 g, yield: 87%).
LCMS m/z = 616.2 $[M+1]^+$

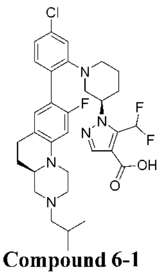
Compound 6-1

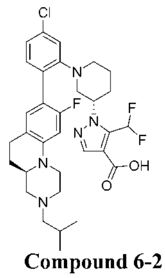
Compound 6-2

**Compound 6** (140 mg) was subjected to chiral resolution, and the preparation method was as follows.
Instrument: SFC Prep 150 AP; chromatographic column: AD (19 mm × 250 mm).
The sample was dissolved in methanol and filtered through a 0.45 μm filter head to prepare a sample solution.
Preparative chromatography conditions: a. the mobile phase consists of systems A and B: mobile phase A: $CO_2$; mobile phase B: methanol (0.1% aqueous ammonia); b. isocratic elution, mobile phase B: 35%; c. flow rate: 30 mL/min.
After preparative separation, fractions with the same retention time were combined and concentrated under reduced pressure to afford **Compound 6-1** (30 mg) and **Compound 6-2** (70 mg).

**Compound 6-1:** retention time under preparation method: 8.4 min, LCMS m/z = 616.2 $[M+1]^+$

$^1$H NMR (400 MHz, DMSO-$d_6$) δ 7.77 (s, 1H), 7.73 (t, 1H), 7.17 - 7.00 (m, 4H), 6.67 (d, 1H), 4.40 - 4.26 (m, 1H), 3.81 - 3.71 (m, 1H), 3.20 - 3.14 (m, 1H), 3.09- 2.54 (m, 9H), 2.13 - 1.98 (m, 3H), 1.97 - 1.68 (m, 6H), 1.67 - 1.53 (m, 1H), 1.45 - 1.31 (m, 1H), 0.95 - 0.83 (m, 6H).

**Compound 6-2:** retention time under preparation method: 9.6 min, LCMS m/z = 616.2 $[M+1]^+$

$^1$H NMR (400 MHz, $CD_3OD$) δ 7.79 (s, 1H), 7.66 (t, 1H), 7.15 - 6.95 (m, 4H), 6.70 (d, 1H), 4.41 - 4.31 (m, 1H), 4.07 - 3.93 (m, 1H), 3.60 - 3.50 (m, 1H), 3.49 - 3.42 (m, 1H), 3.38 - 3.32 (m, 1H), 3.21 - 3.11 (m, 2H), 3.10 - 2.90 (m, 3H), 2.89 - 2.77 (m, 3H), 2.76 - 2.63 (m, 3H), 2.25 - 2.10 (m, 1H), 2.06 - 1.90 (m, 3H), 1.85 - 1.72 (m, 2H), 1.64 - 1.47 (m, 1H), 1.07 (d, 6H).

**Example 7:**

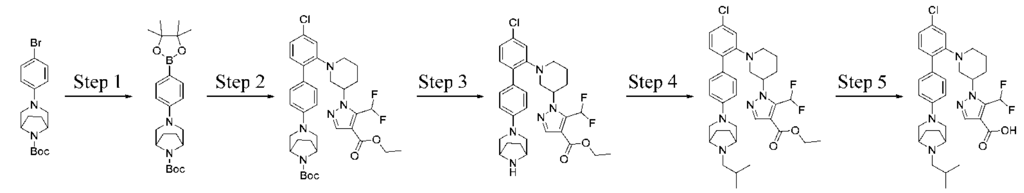


7A 7B 7C 7D 7E **Compound 7**

Step 1: Preparation of **7B**

Compound **7A** (2 g, 5.45 mmol) (see WO 2009050236 for its synthesis method) and bis(pinacolato)diboron (2.08 g, 8.18 mmol) were dissolved in 1,4-dioxane (30 mL). [1,1'-Bis(diphenylphosphino)ferrocene]dichloropalladium (0.40 g, 0.55 mmol) and potassium acetate (1.60 g, 16.35 mmol) were added. The mixture was purged with nitrogen three times, and heated to 100°C and stirred overnight under nitrogen atmosphere. The mixture was cooled to room temperature, and extracted with ethyl acetate and water. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 5/1) to afford Compound **7B** (1.1 g, yield: 48%).
LCMS m/z = 415.3 $[M+H]^+$

Step 2: Preparation of **7C**

**5D** (0.40 g, 0.75 mmol) and **7B** (0.47 g, 1.13 mmol) were dissolved in toluene (6 mL), ethanol (6 mL), and water (2 mL). Tetrakis(triphenylphosphine)palladium (0.17 g, 0.15 mmol) and sodium carbonate (0.24 g, 2.25 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **7C** (0.4 g, yield: 79%).
LCMS m/z = 670.3 $[M+H]^+$

Step 3: Preparation of **7D**

**7C** (0.4 g, 0.60 mmol) was dissolved in 4 M HCl/1,4-dioxane (5 mL), and the mixture was stirred at room temperature for 1 h. The mixture was concentrated under reduced pressure. Ethyl acetate and saturated aqueous sodium bicarbonate solution were added, and the mixture was stirred and layered. The organic phase was washed with saturated brine, dried over anhydrous sodium sulfate, and concentrated under reduced pressure to afford Compound **7D** (0.34 g, yield: 99%).
LCMS m/z = 570.3 $[M+H]^+$

Step 5: Preparation of **7E**

**7D** (0.34 g, 0.60 mmol) and isobutyraldehyde (0.087 g, 1.2 mmol) were dissolved in DCM (10 mL). Glacial acetic acid (0.14 g, 2.4 mmol) was added, and the mixture was stirred at room temperature for 10 min. Sodium triacetoxyborohydride (0.38 g, 1.80 mmol) was added, and the mixture was reacted at room temperature overnight. Ethyl acetate and saturated aqueous sodium bicarbonate solution were added, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **7E** (0.3 g, yield: 79%).
LCMS m/z = 626.3 $[M+H]^+$

Step 6: Preparation of **Compound 7**

Compound **7E** (0.3 g, 0.48 mmol) was dissolved in a mixed solvent of 1,4-dioxane (3 mL) and water (1.5 mL). Lithium hydroxide monohydrate (0.20 g, 4.77 mmol) was added, and the mixture was stirred at room temperature for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified by column chromatography (dichloromethane/methanol (V/V) = 1/0 to 10/1) to afford **Compound 7** (0.14 g, yield: 48%).

LCMS m/z = 598.2 $[M+1]^+$

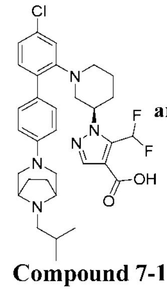

and

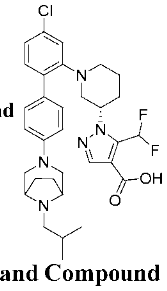

**Compound 7-1 and Compound 7-2**

**Compound 7** (100 mg) was subjected to chiral resolution, and the preparation method was as follows.

Instrument: SFC Prep 150 AP; chromatographic column: AD (19 mm × 250 mm).

The sample was dissolved in methanol and filtered through a 0.45 μm filter head to prepare a sample solution.

Preparative chromatography conditions: a. the mobile phase consists of systems A and B: mobile phase A: $CO_2$; mobile phase B: methanol/ethanol (0.1% aqueous ammonia) = 1/1; b. isocratic elution, mobile phase B: 30%; c. flow rate: 40 mL/min.

After preparative separation, fractions with the same retention time were combined and concentrated under reduced pressure to afford **Compound 7-1** (30 mg) and **Compound 7-2** (25 mg).

**Compound 7-1:** retention time under preparation method: 12.3 min, LCMS m/z = 598.2 $[M+1]^+$

$^1$H NMR (400 MHz, $CD_3OD$) δ 7.71 (s, 1H), 7.70 (t, 1H), 7.48 - 7.42 (m, 2H), 7.15 - 7.01 (m, 3H), 6.98 - 6.90 (m, 2H), 4.46 - 4.35 (m, 1H), 4.12 - 3.98 (m, 2H), 3.77 - 3.63 (m, 2H), 3.30 - 3.23 (m, 2H), 3.21 - 3.10 (m, 2H), 2.99 - 2.84 (m, 3H), 2.76 - 2.65 (m, 1H), 2.31 - 2.21 (m, 2H), 2.21 - 2.08 (m, 3H), 2.05 - 1.97 (m, 1H), 1.96 - 1.81 (m, 2H), 1.74 - 1.59 (m, 1H), 1.11 (t, 6H).

**Compound 7-2:** retention time under preparation method: 17.1 min, LCMS m/z = 598.2 $[M+1]^+$

$^1$H NMR (400 MHz, $CD_3OD$) δ 7.71 (s, 1H), 7.70 (t, 1H), 7.49 - 7.40 (m, 2H), 7.15 - 7.01 (m, 3H), 6.99 - 6.89 (m, 2H), 4.46 - 4.31 (m, 1H), 4.16 - 3.96 (m, 2H), 3.80 - 3.62 (m, 2H), 3.29 - 3.23 (m, 2H), 3.22 - 3.10 (m, 2H), 2.99 - 2.85 (m, 3H), 2.76 - 2.65 (m, 1H), 2.30 - 2.21 (m, 2H), 2.21 - 2.08 (m, 3H), 2.06 - 1.97 (m, 1H), 1.96 - 1.81 (m, 2H), 1.74 - 1.57 (m, 1H), 1.11 (d, 6H).

**Example 8:**

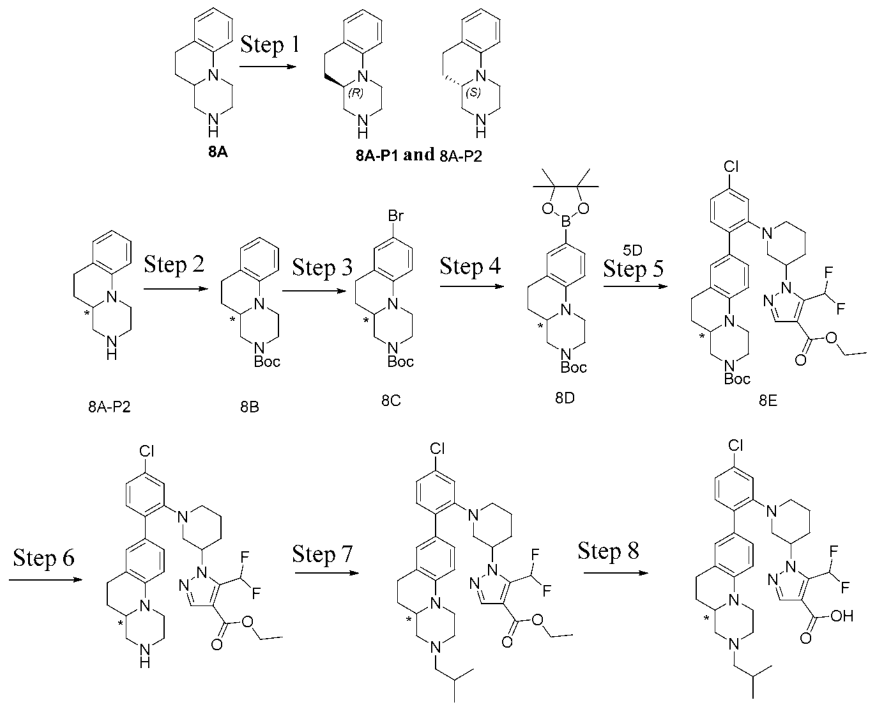


8A

8A-P1 and 8A-P2

8A-P2 8B 8C 8D 8E

8F 8G **Compound 8**

Step 1: Preparation of 8A-P1 and 8A-P2

Compound 8A (see DOI: 10.1021/acs.oprd.9b00364 for its synthesis method) was subjected to chiral resolution. The resolution method was as follows.

Instrument: SFC Prep 150 AP; chromatographic column: Daicel AD-H (19 mm × 250 mm).

The sample was dissolved in methanol and filtered through a 0.45 μm filter head to prepare a sample solution.

Preparative chromatography conditions: mobile phase A: CO2, mobile phase B: methanol; isocratic elution, mobile phase B: 25%; flow rate: 40 ml/min.

After preparative separation, fractions with the same retention time were combined and concentrated under reduced pressure to afford Compound 8A-P1 and Compound 8A-P2.

Compound 8A-P1: retention time under preparation method: 4.65 min; and

Compound 8A-P2: retention time under preparation method: 5.85 min.

Step 2: Preparation of 8B

Compound 8A-P2 (0.6 g, 3.19 mmol) was dissolved in dichloromethane (30 ml). Boc anhydride (1.39 g, 6.4 mmol) and 4-dimethylaminopyridine (0.39, 3.19 mmol) were added. The mixture was stirred at room temperature overnight. The reaction solution was washed with 0.5 *N* hydrochloric acid (50 mL), dried over anhydrous sodium sulfate, and then concentrated under reduced pressure. The residue was separated and purified by column chromatography on silica gel (ethyl acetate : petroleum ether (V : V) = 0 : 1 to 1 : 9) to afford Compound 8B (0.8 g, yield: 87%).

Step 3: Preparation of 8C

Compound 8B (0.78 g, 2.70 mmol) was dissolved in acetonitrile (20 ml). NBS (0.53 g, 2.97 mmol) was added, and the mixture was stirred for 1 hour. Then, the mixture was concentrated under reduced pressure. The residue was separated and purified by column chromatography on silica gel (ethyl acetate : petroleum ether (V : V) = 0 : 1 to 1 : 9) to afford Compound 8C (0.86 g, yield: 87%).
LCMS m/z = 367.5 $[M+1]^+$

Step 4: Preparation of 8D

8C (0.5 g, 1.36 mmol) and bis(pinacolato)diboron (1.69 g, 2.72 mmol) were dissolved in 1,4-dioxane (10 mL). [1,1'-Bis(diphenylphosphino)ferrocene]dichloropalladium (0.20 g, 0.27 mmol) and potassium acetate (0.40 g, 4.08 mmol)

were added. The mixture was purged with nitrogen three times, and heated to 95°C and stirred overnight under nitrogen atmosphere. The mixture was cooled to room temperature. An appropriate amount of silica gel was added, and the mixture was then concentrated under reduced pressure. The residue was purified by column chromatography on silica gel (petroleum ether/ethyl acetate (V/V) = 100/0 to 90/10) to afford Compound 8D (0.6 g, yield: 53%).

Step 5: Preparation of 8E

5D (0.39 g, 0.73 mmol) and 8D (0.45 g, 1.09 mmol) were dissolved in toluene (6 mL), ethanol (6 mL), and water (3 mL). Tetrakis(triphenylphosphine)palladium (0.17 g, 0.15 mmol) and sodium carbonate (0.23 g, 2.19 mmol) were added. The mixture was purged with nitrogen three times, and heated to 95°C and stirred overnight under nitrogen atmosphere. The mixture was cooled to room temperature. Ethyl acetate and water were added, and the mixture was stirred and then layered. An appropriate amount of silica gel was added to the organic phase, and the mixture was concentrated under reduced pressure. The residue was purified by column chromatography (mobile phase: petroleum ether/ethyl acetate (V/V) = 100/0 to 80/20) to afford Compound 8E (0.31 g, yield: 63%).

Step 6: Preparation of 8F

8E (0.31 g, 0.46 mmol) was dissolved in dichloromethane (3 mL). Trifluoroacetic acid (1 mL) was added, and the mixture was stirred at room temperature for 2 h. The mixture was concentrated under reduced pressure. Then, dichloromethane and saturated aqueous sodium bicarbonate solution were added, and the mixture was stirred and layered. The organic phase was dried over anhydrous sodium sulfate, and concentrated under reduced pressure to afford Compound 8F (0.24 g).
LCMS m/z = 570.4 $[M+H]^+$

Step 7: Preparation of 8G

8F (0.24 g, 0.42 mmol) and isobutyraldehyde (0.15 g, 2.10 mmol) were dissolved in DCM (5 mL). The mixture was stirred at room temperature for 1 hour. Sodium triacetoxyborohydride (0.27 g, 1.26 mmol) and glacial acetic acid (0.076 g, 1.26 mmol) were added, and the mixture was reacted at room temperature overnight. Dichloromethane and saturated aqueous sodium bicarbonate solution were added, and the mixture was stirred and then layered. An appropriate amount of silica gel was added to the organic phase, and the mixture was concentrated under reduced pressure. The residue was purified by column chromatography (mobile phase: petroleum ether/ethyl acetate (V/V) = 100/0 to 80/20) to afford Compound 8G (0.16 g, yield: 61%).
LCMS m/z = 626.3 $[M+H]^+$

Step 8: Preparation of Compound 8

Compound 8G (0.16 g, 0.26 mmol) was dissolved in a mixed solvent of 1,4-dioxane (3 mL) and water (1 mL). Lithium hydroxide monohydrate (0.11 g, 2.6 mmol) was added, and the mixture was stirred at room temperature overnight. Dichloromethane and water were added. The pH of the reaction solution was adjusted to 2-3 with 1 *N* aqueous hydrochloric acid, and the mixture was layered. An appropriate amount of silica gel was added to the organic layer, and the mixture was concentrated under reduced pressure. The residue was purified by column chromatography (dichloromethane/methanol (V/V) = 100/0 to 90/10) to afford Compound 8 (0.02 g, yield: 13%).
LCMS m/z = 598.5 $[M+1]^+$

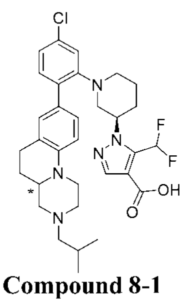
Compound 8-1

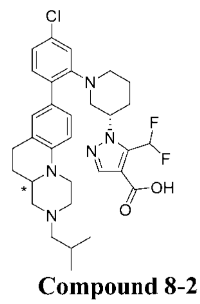
Compound 8-2

**Compound 8** (380 mg) was subjected to chiral separation and purification.

Preparative conditions: instrument: Waters 150 Prep-SFC; chromatographic column: chiral AD column; mobile phase: A for $CO_2$, B for isopropanol and acetonitrile (0.1% aqueous ammonia); gradient: B for 55%; flow rate: 100 ml/min; back pressure: 100 bar; column temperature: room temperature; detection wavelength: 220 nm; cycle: 4.0 min.

Analytical conditions: instrument: SHIMADZU LC-30AD SFC; chromatographic column: chiral AD column; mobile phase: A for $CO_2$, mobile phase: B for isopropanol (0.05% DEA); gradient: B for 5-40%; flow rate: 3 mL/min; column temperature: 35°C; detection wavelength: 220 nm.

After preparative separation, fractions with the same retention time were combined and concentrated under reduced pressure to afford **Compound 8-1** (161.7 mg) and **Compound 8-2** (189.9 mg).

**Compound 8-1:** retention time under analytical conditions: 1.841 min, LCMS m/z = 598.4 $[M+H]^+$

$^1H$ NMR (400 MHz, $CD_3OD$) δ 7.76 (s, 1H), 7.67 (t, 1H), 7.32 - 7.28 (m, 1H), 7.22 - 7.15 (m, 1H), 7.14 - 7.08 (m, 1H), 7.07 - 6.99 (m, 2H), 6.94 - 6.86 (m, 1H), 4.48 - 4.35 (m, 1H), 4.10 - 3.97 (m, 1H), 3.53 - 3.45 (m, 1H), 3.45 - 3.36 (m, 1H), 3.30 - 3.25 (m, 1H), 3.23 - 3.11 (m, 2H), 3.09 - 2.84 (m, 4H), 2.82 - 2.56 (m, 5H), 2.22 - 2.08 (m, 1H), 2.06 - 1.73 (m, 5H), 1.72 - 1.56 (m, 1H), 1.06 (d, 6H).

**Compound 8-2:** retention time under analytical conditions: 2.431 min, LCMS m/z = 598.6 $[M+H]^+$

$^1H$ NMR (400 MHz, $CD_3OD$) δ 7.76 (s, 1H), 7.66 (t, 1H), 7.36 - 7.28 (m, 1H), 7.23 - 7.14 (m, 1H), 7.14 - 7.08 (m, 1H), 7.07 - 6.97 (m, 2H), 6.96 - 6.87 (m, 1H), 4.51 - 4.36 (m, 1H), 4.11 - 3.96 (m, 1H), 3.53 - 3.45 (m, 1H), 3.43 - 3.34 (m, 1H), 3.26 - 3.18 (m, 2H), 3.18 - 3.10 (m, 1H), 3.10 - 3.01 (m, 1H), 3.00 - 2.83 (m, 3H), 2.81 - 2.56 (m, 5H), 2.19 - 2.08 (m, 1H), 2.06 - 1.90 (m, 3H), 1.89 - 1.74 (m, 2H), 1.72 - 1.56 (m, 1H), 1.06 (d, 6H).

**Example 9:**

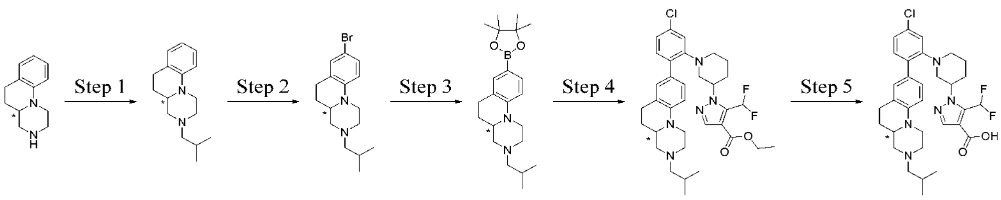


8A-P1 9A 9B 9C 9D **Compound 9**

Step 1: Preparation of **9A**

**8A**-**P1** (1.1 g, 5.86 mmol) and isobutyraldehyde (0.84 g, 11.68 mmol) were dissolved in DCM (20 mL). Glacial acetic acid (1.05 g, 17.52 mmol) was added, and the mixture was stirred at room temperature for 10 min. Sodium triacetoxyborohydride (4.95 g, 23.36 mmol) was added, and the mixture was reacted at room temperature overnight. Ethyl acetate and saturated aqueous sodium bicarbonate solution were added, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 2/1) to afford Compound **9A** (0.5 g, yield: 35%).
LCMS m/z = 245.1 $[M+H]^+$

Step 2: Preparation of **9B**

Compound **9A** (0.5 g, 2.05 mmol) was dissolved in acetonitrile (10 ml). NBS (0.36 g, 2.05 mmol) was added, and the mixture was stirred for 1 hour. Then, the reaction solution was concentrated under reduced pressure. The residue was separated and purified by column chromatography on silica gel (ethyl acetate : petroleum ether (V : V) = 0 : 1 to 1 : 9) to afford **9B** (0.53 g, yield: 79%).
LCMS m/z = 323.0 $[M+H]^+$

Step 3: Preparation of **9C**

Compound **9B** (0.53 g, 1.64 mmol) and bis(pinacolato)diboron (0.62 g, 2.46 mmol) were dissolved in 1,4-dioxane (10 mL). [1,1'-Bis(diphenylphosphino)ferrocene]dichloropalladium (0.24 g, 0.33 mmol) and potassium acetate (0.48 g, 4.92 mmol) were added. The mixture was purged with nitrogen three times, and heated to 100°C and stirred overnight under nitrogen atmosphere. The mixture was cooled to room temperature, and extracted with ethyl acetate and water. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 5/1) to afford Compound **9C** (0.45 g, yield: 74%).
LCMS m/z = 371.5 $[M+H]^+$

Step 4: Preparation of **9D**

**5D** (0.45 g, 0.85 mmol) and **9C** (0.47 g, 1.27 mmol) were dissolved in toluene (6 mL), ethanol (6 mL), and water (2 mL). Tetrakis(triphenylphosphine)palladium (0.2 g, 0.17 mmol) and sodium carbonate (0.27 g, 2.55 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **9D** (80 mg, yield: 15%).
LCMS m/z = 626.3 $[M+H]^+$

Step 5: Preparation of **Compound 9**

Compound **9D** (80 mg, 0.13 mmol) was dissolved in a mixed solvent of 1,4-dioxane (3 mL) and water (1.5 mL). Lithium hydroxide monohydrate (0.055 g, 1.3 mmol) was added, and the mixture was stirred at room temperature for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure, and concentrated under reduced pressure. The residue was purified by column chromatography (dichloromethane/methanol (V/V) = 1/0 to 10/1) to afford **Compound 9** (50 mg, yield: 65%).
LCMS m/z = 598.2 $[M+1]^+$

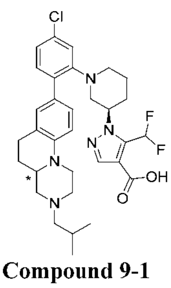
**Compound 9-1**

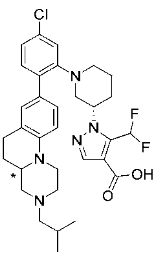
**Compound 9-2**

**Compound 9** (500 mg) was subjected to chiral separation and purification.

Preparative conditions: instrument: Waters 150 Prep-SFC; chromatographic column: chiral AD column; mobile phase: A for $CO_2$, B for isopropanol (0.1% aqueous ammonia); gradient: B for 50%; flow rate: 100 ml/min; back pressure: 100 bar; column temperature: room temperature; detection wavelength: 220 nm; cycle: 3.6 min.

Analytical conditions: instrument: SHIMADZU LC-30AD SFC; chromatographic column: chiral AD column; mobile phase: A for $CO_2$, mobile phase: B for isopropanol (0.05% DEA); gradient: B for 5-40%; flow rate: 3 mL/min; column temperature: 35°C; detection wavelength: 220 nm.

After preparative separation, fractions with the same retention time were combined and concentrated under reduced pressure to afford **Compound 9-1** (198.7 mg) and **Compound 9-2** (242.9 mg).

**Compound 9-1:** retention time under analytical conditions: 1.903 min, LCMS m/z = 598.3 $[M+H]^+$

$^1H$ NMR (400 MHz, $CD_3OD$) δ 7.80 - 7.74 (m, 1H), 7.64 (t, 1H), 7.35 - 7.29 (m, 1H), 7.21 - 7.15 (m, 1H), 7.14 - 7.08 (m, 1H), 7.07 - 6.99 (m, 2H), 6.95 - 6.87 (m, 1H), 4.49 - 4.39 (m, 1H), 4.12 - 4.01 (m, 1H), 3.55 - 3.46 (m, 1H), 3.45 - 3.36 (m, 1H), 3.28 - 3.19 (m, 2H), 3.18 - 3.11 (m, 1H), 3.10 - 2.85 (m, 4H), 2.84 - 2.57 (m, 5H), 2.20 - 2.08 (m, 1H), 2.07 - 1.89 (m, 3H), 1.89 - 1.74 (m, 2H), 1.72 - 1.57 (m, 1H), 1.06 (d, 6H).

**Compound 9-2:** retention time under analytical conditions: 2.468 min, LCMS m/z = 598.3 [M+H]+

$^1H$ NMR (400 MHz, $CD_3OD$) δ 7.76 (s, 1H), 7.68 (t, 1H), 7.35 - 7.26 (m, 1H), 7.22 - 7.15 (m, 1H), 7.14 - 7.08 (m, 1H), 7.07 - 6.98 (m, 2H), 6.95 - 6.85 (m, 1H), 4.47 - 4.33 (m, 1H), 4.11 - 3.94 (m, 1H), 3.53 - 3.45 (m, 1H), 3.44 - 3.36 (m, 1H), 3.30 - 3.24 (m, 1H), 3.24 - 3.10 (m, 2H), 3.09 - 2.83 (m, 4H), 2.82 - 2.53 (m, 5H), 2.21 - 2.08 (m, 1H), 2.07 - 1.71 (m, 5H), 1.71 - 1.56 (m, 1H), 1.06 (d, 6H).

**Example 10:**

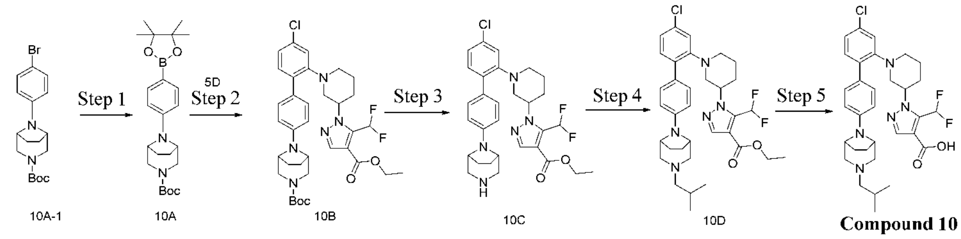


10A-1 10A 10B 10C 10D **Compound 10**

Step 1: Preparation of **10A**

**10A-1** (CAS: 2760247-54-7, see WO 2022029617 for its synthesis, 1 g, 2.72 mmol) and bis(pinacolato)diboron (1.38 g, 5.44 mmol) were dissolved in 1,4-dioxane (20 mL). [1,1'-Bis(diphenylphosphino)ferrocene]dichloropalladium (0.40 g, 0.54 mmol) and potassium acetate (0.80 g, 8.16 mmol) were added. The mixture was purged with nitrogen three times, and heated to 95°C and stirred overnight under nitrogen atmosphere. The mixture was cooled to room temperature. An appropriate amount of silica gel was added, and the mixture was concentrated under reduced pressure. The residue was purified by column chromatography on silica gel (petroleum ether/ethyl acetate (V/V) = 100/0 to 90/10) to afford Compound **10A** (0.6 g, yield: 53%).

Step 2: Preparation of **10B**

**5D** (0.50 g, 0.94 mmol) and **10A** (0.58 g, 1.41 mmol) were dissolved in toluene (6 mL), ethanol (6 mL), and water (3 mL). Tetrakis(triphenylphosphine)palladium (0.22 g, 0.19 mmol) and sodium carbonate (0.30 g, 2.83 mmol) were added. The mixture was purged with nitrogen three times, and heated to 95°C and stirred overnight under nitrogen atmosphere. The mixture was cooled to room temperature. Ethyl acetate and water were added, and the mixture was stirred and then layered. An appropriate amount of silica gel was added to the organic phase, and the mixture was concentrated under reduced pressure. The residue was purified by column chromatography (mobile phase: petroleum ether/ethyl acetate (V/V) = 100/0 to 80/20) to afford Compound **10B** (0.4 g, yield: 63%).

Step 3: Preparation of **10C**

**10B** (0.4 g, 0.60 mmol) was dissolved in dichloromethane (3 mL). Trifluoroacetic acid (1 mL) was added, and the mixture was stirred at room temperature for 2 h. The mixture was concentrated to dryness under reduced pressure. Dichloromethane and saturated aqueous sodium bicarbonate solution were added, and the mixture was stirred and layered. The organic phase was dried over anhydrous sodium sulfate, and concentrated under reduced pressure to afford Compound **10C** (0.3 g).
LCMS m/z = 570.3 [M+H]+

Step 5: Preparation of **10D**

**10C** (0.32 g, 0.56 mmol) and isobutyraldehyde (0.21 g, 2.85 mmol) were dissolved in DCM (5 mL). The mixture was stirred at room temperature for 1 hour. Sodium triacetoxyborohydride (0.36 g, 1.68 mmol) and glacial acetic acid (0.10 g, 1.68 mmol) were added, and the mixture was reacted at room temperature overnight. Dichloromethane and saturated aqueous sodium bicarbonate solution were added, and the mixture was stirred and then layered. An appropriate amount of silica gel was added to the organic phase, and the mixture was concentrated under reduced pressure. The residue was purified by column chromatography (mobile phase: petroleum ether/ethyl acetate (V/V) = 100/0 to 80/20) to afford Compound **10D** (0.3 g, yield: 79%).

LCMS m/z = 626.3 [M+H]+

Step 6: Preparation of **Compound 10**

Compound **10D** (0.17 g, 0.27 mmol) was dissolved in a mixed solvent of 1,4-dioxane (3 mL) and water (1 mL). Lithium hydroxide monohydrate (0.11 g, 2.7 mmol) was added, and the mixture was stirred at room temperature overnight. Dichloromethane and water were added. The pH of the reaction solution was adjusted to 2-3 with 1 *N* aqueous hydrochloric acid, and the mixture was layered. An appropriate amount of silica gel was added to the organic layer, and the mixture was concentrated under reduced pressure. The residue was purified by column chromatography (dichloromethane/methanol (V/V) = 100/0 to 90/10) to afford **Compound 10** (0.02 g, yield: 12%).
LCMS m/z = 598.3 [M+1]+

$^1$H NMR (400 MHz, DMSO-$d_6$) δ 7.96 (s, 1H), 7.56 (t, 1H), 7.40 (d, 2H), 7.17 (d, 1H), 7.12 - 6.99 (m, 2H), 6.85 (d, 2H), 4.63 - 4.50 (m, 1H), 4.34 - 4.16 (m, 2H), 3.35 - 3.30 (m, 2H), 3.11 - 2.96 (m, 2H), 2.49 - 2.42 (m, 2H), 2.37 - 2.28 (m, 2H), 2.07 - 1.79 (m, 8H), 1.77 - 1.63 (m, 2H), 1.58 - 1.39 (m, 1H), 0.84 (d, 6H).

**Example 11: Preparation of Compound 11**

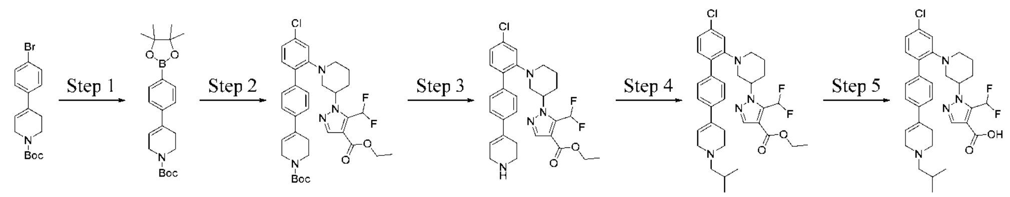


11A 11B 11C 11D 11E **Compound 11**

Step 1: Preparation of **11B**

Compound **11A** (3.5 g, 10.35 mmol) (see WO 2021255212 for its synthesis method) and bis(pinacolato)diboron (3.94 g, 15.52 mmol) were dissolved in DMSO (30 mL). [1,1'-Bis(diphenylphosphino)ferrocene]dichloropalladium (0.85 g, 1.03 mmol) and potassium acetate (3.05 g, 31.05 mmol) were added. The mixture was purged with nitrogen three times, and heated to 100°C and stirred overnight under nitrogen atmosphere. The mixture was cooled to room temperature, and extracted with ethyl acetate and water. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 10/1) to afford Compound **11B** (2.6 g, yield: 66%).

Step 2: Preparation of **11C**

**5D** (1.1 g, 2.07 mmol) and **11B** (1.2 g, 3.1 mmol) were dissolved in toluene (9 mL), ethanol (9 mL), and water (3 mL). Tetrakis(triphenylphosphine)palladium (0.48 g, 0.41 mmol) and sodium carbonate (0.66 g, 6.21 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **11C** (0.77 g, yield: 58%).
LCMS m/z = 641.3 [M+H]+

Step 3: Preparation of **11D**

**11C** (0.77 g, 1.2 mmol) was dissolved in 4 M HCl/1,4-dioxane (8 mL), and the mixture was stirred at room temperature for 1 h. The mixture was concentrated under reduced pressure. Ethyl acetate and saturated aqueous sodium bicarbonate solution were added, and the mixture was stirred and separated. The organic phase was washed with saturated brine, dried over anhydrous sodium sulfate, and concentrated under reduced pressure to afford Compound **11D** (0.61 g).
LCMS m/z = 541.3 [M+H]+

Step 5: Preparation of **11E**

**11D** (0.2 g, 0.37 mmol) and isobutyraldehyde (0.08 g, 1.11 mmol) were dissolved in DCM (5 mL). Glacial acetic acid (0.044 g, 0.74 mmol) was added, and the mixture was stirred at room temperature for 1 hour. Sodium triacetoxyborohydride (0.235 g, 1.11 mmol) was added, and the mixture was reacted at room temperature overnight. Ethyl acetate and saturated aqueous sodium bicarbonate solution were added, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 4/1) to afford Compound **11E** (0.157 g, yield: 71%).
LCMS m/z = 597.3 $[M+H]^+$

Step 6: Preparation of **Compound 11**

Compound **11E** (0.06 g, 0.1 mmol) was dissolved in a mixed solvent of 1,4-dioxane (3 mL) and water (1 mL). Lithium hydroxide monohydrate (0.042 g, 1 mmol) was added, and the mixture was stirred at 60°C for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified with a Flash column (acetonitrile/water (containing 0.1% trifluoroacetic acid) (V/V) = 0/100 to 60/40) to afford the trifluoroacetate salt of **Compound 11** (0.015 g).
LCMS m/z = 569.3 $[M+ H]^+$

$^1$H NMR (400 MHz, DMSO-$d_6$) δ 7.95 (s, 1H), 7.69 - 7.55 (m, 4H), 7.50 (t, 1H), 7.27 - 7.05 (m, 3H), 6.34 - 6.10 (m, 1H), 4.56 - 4.34 (m, 1H), 4.16 - 4.00 (m, 1H), 3.87 - 3.64 (m, 2H), 3.36 - 3.15 (m, 2H), 3.12 - 2.96 (m, 4H), 2.96 - 2.71 (m, 2H), 2.62 (t, 1H), 2.24 - 2.09 (m, 1H), 2.06 - 1.83 (m, 2H), 1.79 - 1.66 (m, 1H), 1.59 - 1.36 (m, 1H), 1.11 - 0.93 (m, 6H).

**Example 12: Preparation of Compound 12**

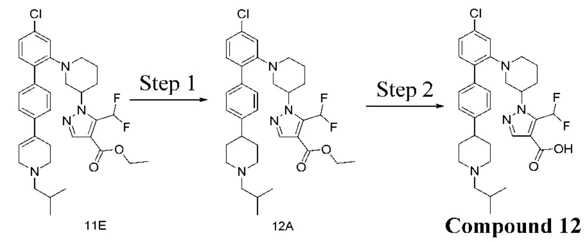


11E 12A **Compound 12**

Step 1: Preparation of **12A**

Compound **11E** (0.04 g, 0.067 mmol) was dissolved in methanol (3 mL). Tris(triphenylphosphine)rhodium chloride (6.2 mg, 0.0067 mmol) was added. The mixture was purged with hydrogen three times, heated to 50°C, and stirred overnight. The mixture was cooled to room temperature, filtered through celite, and concentrated under reduced pressure. The residue was purified by column chromatography on silica gel (petroleum ether/ethyl acetate (V/V) = 1/0 to 3/1) to afford Compound **12A** (22 mg, yield: 55%).
LCMS m/z = 599.1 $[M+H]^+$

Step 2: Preparation of **Compound 12**

Compound **12A** (22 mg, 0.037 mmol) was dissolved in a mixed solvent of 1,4-dioxane (1.5 mL) and water (0.5 mL). Lithium hydroxide monohydrate (15.53 mg, 0.37 mmol) was added, and the mixture was stirred at 60°C for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified with a Flash column (acetonitrile/water (containing 0.1% trifluoroacetic acid) (V/V) = 0/100 to 60/40) to afford the trifluoroacetate salt of **Compound 12** (5 mg).
LCMS m/z = 571.1 $[M+ H]^+$

$^1$H NMR (400 MHz, DMSO-$d_6$) δ 7.94 (s, 1H), 7.54 (d, 2H), 7.48 (t, 1H), 7.30 (d, 2H), 7.23 - 7.06 (m, 3H), 4.54 - 4.31 (m, 1H), 3.71 - 3.55 (m, 2H), 3.23 - 3.14 (m, 1H), 3.12 - 2.90 (m, 6H), 2.90-2.77 (m, 1H), 2.65 (t, 1H), 2.23 - 1.84 (m, 7H), 1.80 - 1.64 (m, 1H), 1.56 - 1.38 (m, 1H), 1.00 (d, 6H).

**Example 13:**

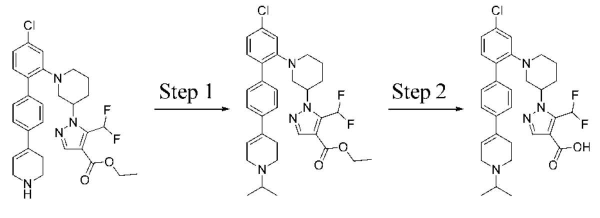


11D 13A **Compound 13**

Step 1: Preparation of **13A**

Compound **11D** (0.33 g, 0.61 mmol) and 2-iodopropane (0.31 g, 1.83 mmol) were dissolved in acetonitrile (10 mL). N,N-diisopropylethylamine (0.24 g, 1.83 mmol) was added. The mixture was heated to 60°C and stirred for 2 h. The mixture was cooled to room temperature, and extracted with ethyl acetate and water. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 4/1) to afford Compound **13A** (0.17 g, yield: 48%).
LCMS m/z = 583.1 $[M+H]^+$

Step 2: Preparation of **Compound 13**

Compound **13A** (0.05 g, 0.086 mmol) was dissolved in a mixed solvent of 1,4-dioxane (3 mL) and water (1 mL). Lithium hydroxide monohydrate (0.036 g, 0.86 mmol) was added, and the mixture was stirred at 60°C for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified with a Flash column (acetonitrile/water (containing 0.1% trifluoroacetic acid) (V/V) = 0/100 to 60/40) to afford the trifluoroacetate salt of **Compound 13** (0.008 g).
LCMS m/z = 555.3 $[M+H]^+$

$^1$H NMR (400 MHz, DMSO- $d_6$) δ 7.95 (s, 1H), 7.68 - 7.53 (m, 4H), 7.50 (t, 1H), 7.29 - 7.09 (m, 3H), 6.36 - 6.20 (m, 1H), 4.53 - 4.36 (m, 1H), 3.99 - 3.83 (m, 2H), 3.71 - 3.56 (m, 2H), 3.29 - 3.14 (m, 2H), 3.05 (t, 2H), 2.97 - 2.72 (m, 2H), 2.62 (t, 1H), 2.07 - 1.83 (m, 2H), 1.79 - 1.69 (m, 1H), 1.56 - 1.41 (m, 1H), 1.33 (d, 6H).

**Example 14: Preparation of Compound 14**

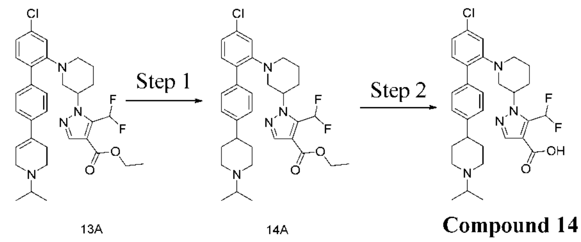


13A 14A **Compound 14**

Step 1: Preparation of **14A**

Compound **13A** (0.12 g, 0.21 mmol) was dissolved in methanol (5 mL). Tris(triphenylphosphine)rhodium chloride (19.43 mg, 0.021 mmol) was added. The mixture was purged with hydrogen three times, heated to 50°C, and stirred overnight. The mixture was cooled to room temperature, filtered through celite, and concentrated under reduced pressure. The residue was purified by column chromatography on silica gel (petroleum ether/ethyl acetate (V/V) = 1/0 to 3/1) to afford Compound **14A** (40 mg, yield: 33%).
LCMS m/z = 585.3 $[M+H]^+$

Step 2: Preparation of **Compound 14**

Compound **14A** (40 mg, 0.068 mmol) was dissolved in a mixed solvent of 1,4-dioxane (3 mL) and water (1 mL). Lithium hydroxide monohydrate (28.53 mg, 0.68 mmol) was added, and the mixture was stirred at 60°C for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified with a Flash column (acetonitrile/water (containing 0.1% trifluoroacetic acid) (V/V) = 0/100 to 60/40) to afford the trifluoroacetate salt of **Compound 14** (8 mg).
LCMS m/z = 557.3 $[M+H]^+$

$^1H$ NMR (400 MHz, DMSO- $d_6$) δ 7.93 (s, 1H), 7.54 (d, 2H), 7.47 (t, 1H), 7.29 (d, 2H), 7.22 - 7.09 (m, 3H), 4.48 - 4.28 (m, 1H), 3.55-3.45 (m, 2H), 3.24 - 2.82 (m, 7H), 2.66 (t, 1H), 2.18 - 1.82 (m, 6H), 1.81 - 1.64 (m, 1H), 1.59 - 1.41 (m, 1H), 1.30 (d, 6H).

**Example 15: Preparation of Compound 15**

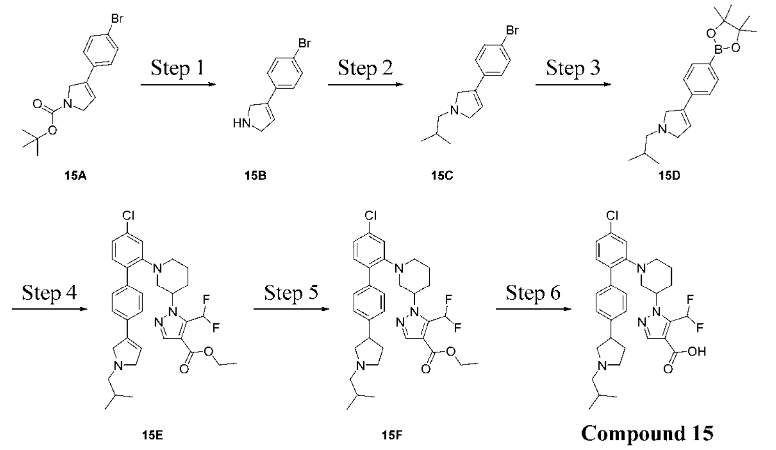


15A 15B 15C 15D 15E 15F **Compound 15**

Step 1: Preparation of **15B**

**15A** (1.9 g, 5.86 mmol) (see WO 2017181117 for its synthesis method) was dissolved in 4 M HCl/1,4-dioxane solution (10 mL), and the mixture was stirred at room temperature for 1 h. The mixture was concentrated under reduced pressure. Ethyl acetate and saturated aqueous sodium bicarbonate solution were added, and the mixture was stirred and separated. The organic phase was washed with saturated brine, dried over anhydrous sodium sulfate, and concentrated under reduced pressure to afford Compound **15B** (1 g).
LCMS m/z = 223.9 $[M+H]^+$

Step 2: Preparation of **15C**

**15B** (1 g, 4.46 mmol) and isobutyraldehyde (0.64 g, 8.92 mmol) were dissolved in DCM (10 mL). Glacial acetic acid (1.07 g, 17.84 mmol) was added, and the mixture was stirred at room temperature for 10 min. Sodium triacetoxyborohydride (2.84 g, 13.38 mmol) was added, and the mixture was reacted at room temperature overnight. Ethyl acetate and saturated aqueous sodium bicarbonate solution were added, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **15C** (0.84 g, yield: 67%).
LCMS m/z = 280.0 $[M+H]^+$

Step 3: Preparation of **15D**

Compound **15C** (0.84 g, 3.00 mmol) and bis(pinacolato)diboron (1.52 g, 6 mmol) were dissolved in 1,4-dioxane (10 mL). [1,1'-Bis(diphenylphosphino)ferrocene]dichloropalladium (0.22 g, 0.30 mmol) and potassium acetate (0.88 g, 9 mmol) were added. The mixture was purged with nitrogen three times, and heated to 100°C and stirred overnight under

nitrogen atmosphere. The mixture was cooled to room temperature, and extracted with ethyl acetate and water. The organic layer was washed with saturated brine, dried over anhydrous sodium sulfate, and concentrated under reduced pressure. The residue was purified by column chromatography on silica gel (petroleum ether/ethyl acetate (V/V) = 1/0 to 5/1) to afford Compound **15D** (0.16 g, yield: 16%).
LCMS m/z = 328.3 $[M+H]^+$

Step 4: Preparation of **15E**

**5D** (0.16 g, 0.30 mmol) and **15D** (0.15 g, 0.45 mmol) were dissolved in toluene (3 mL), ethanol (3 mL), and water (1 mL). Tetrakis(triphenylphosphine)palladium (0.069 g, 0.060 mmol) and sodium carbonate (0.095 g, 0.90 mmol) were added. The mixture was purged with nitrogen three times, and heated to 100°C and stirred overnight under nitrogen atmosphere. The mixture was cooled to room temperature and filtered through celite. Ethyl acetate and water were added to the filtrate, and the mixture was stirred and then layered. The organic phase was washed with saturated brine, dried over anhydrous sodium sulfate, and concentrated under reduced pressure. The residue was purified by column chromatography (mobile phase: petroleum ether/ethyl acetate (V/V) = 1/0 to 5/1) to afford Compound **15E** (50 mg, yield: 28%).
LCMS m/z = 583.3 $[M+H]^+$

Step 5: Preparation of **15F**

Compound **15E** (50 mg, 0.086 mmol) was dissolved in methanol (2 mL). 6 *N* hydrochloric acid (10 mL) and palladium on carbon (2.03 g, 1.90 mmol) were added. The mixture was purged with hydrogen three times, and reacted overnight under hydrogen atmosphere (balloon pressure). The reaction solution was filtered through celite and washed with methanol three times. The filtrate was concentrated under reduced pressure. The residue was purified by column chromatography (mobile phase: dichloromethane/methanol (V/V) = 1/0 to 10/1) to afford Compound **15F** (20 mg, yield: 39%).
LCMS m/z = 585.3 $[M+H]^+$

Step 6: Preparation of **Compound 15**

Compound **15F** (20 mg, 0.034 mmol) was dissolved in a mixed solvent of 1,4-dioxane (2 mL) and water (1 mL). Lithium hydroxide monohydrate (15 mg, 0.36 mmol) was added, and the mixture was stirred at room temperature for 3 h. The pH of the reaction solution was adjusted to 3-4 with 1 *N* aqueous hydrochloric acid. Ethyl acetate was added, and the organic phase was washed with saturated brine, dried over anhydrous sodium sulfate, and concentrated under reduced pressure. The residue was purified with a reversed-phase column (mobile phase: acetonitrile/water (0.1% TFA) (V/V) = 0/100 to 50/50) to afford the trifluoroacetate salt of **Compound 15** (3 mg).
LCMS m/z = 557.1 $[M+ H]^+$

Control Compound 1: 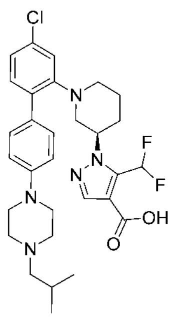 .

## Biological Assay Examples

### 1. cGMP Detection Experiment in CHO-KI/sGC Cells

Stably-transfected CHO-K1 cells stably expressing sGC $\alpha1/\beta1$ heterodimers were constructed and named CHO-KI/sGC. The CHO-KI/sGC cells were cultured in a complete medium (FK12+10% FBS+1% penicillin-streptomycin+0.5 mg/mL hygromycin+ 0.25 mg/mL G418). On the day of the detection, the cells were resuspended in EAB assay buffer (EBSS assay buffer+5 mL $MgCl_2$+10 mM HEPES+0.05% BSA) at a density of $2.25*10^5$ cells/mL. 0.5 mM IBMX was added to prevent cGMP degradation.

The cells were pre-incubated with 1 pM diethylenetriamine/nitric oxide (EDTA-NO) at room temperature for 30 minutes, and then different concentrations of the compounds were added. The mixture was further incubated at 37°C for 1 h. After the incubation was completed, the reaction was terminated, and intracellular cGMP levels were measured according to the instructions for the Cisbio kit (CisBio, 62GM2PEC). The maximum cGMP production relative to the positive compound was calculated according to Equation (1), where $RLU_{compound}$ represents the reading of the test compound, and $RLU_{reference}$ represents the maximum reading of the positive compound.

$$\text{Activion \%} = RLU_{compound}/RUL_{reference} * 100\% \text{ Equation (1)}$$

Conclusion: The compounds of the present invention, such as the example compounds, have good stimulatory effects on cGMP production in CHO-KI/sGC cells.

**2. In Vitro Detection Experiment for Soluble Guanylate Cyclase (sGC) Activity**

First, 100 nL of the compounds at different concentrations were transferred to a 384-well reaction plate (Greiner, Cat. No. 784075) using Echo 655 (LABCYTE, Cat # 655), with the final DMSO concentration of 1% in the reaction mixture; 2 μL of sGC (ICE, Cat. No. S2304F-H07SH2) was added to the 384-well reaction plate, and the mixture was centrifuged at 1000 rpm for 1 min; and then 1 μL of DETA NONOate was added, and the mixture was incubated at 37°C for 10 min. After the incubation was completed, 2 μL of GTP was added, and the mixture was centrifuged at 1000 rpm for 1 min and reacted at 37°C for 60 min. In the reaction system, the final concentrations of sGC, GTP, and DETA NONOate were 1.5 nM, 5 μM, and 100 μM, respectively. After the reaction was completed, 5 μL of the test mixture (PerkinElmer, Cat. No. 62GM2PEG) was added, and the mixture was incubated at room temperature for 60 min. The TR-FRET signal (Ratio: 665/620 nm) was read using a microplate reader (BMG, Cat. No. PHERAstar FSX). A nonlinear regression curve was fitted using GraphPad Prism software, and $EC_{50}$ values were calculated. Compound A (Example 1 in WO 2010065275) was used as a positive reference compound. The activation rate was calculated according to Equation 2-1, where Low control represents the TR-FRET signal value of 1 μM compound A, and High control represents the TR-FRET signal value in the DMSO well.

$$\text{Stimulation\%} = (\text{ave High control - cpd well})/(\text{ave High control - ave Low control}) * 100\% \text{ Equation 2-1}$$

Table 2-1 Results of enzymatic agonism and/or activation of test compounds on soluble guanylate cyclase (sGC).

| Compound No. | $EC_{50}$ (nM) | Compound No. | $EC_{50}$ (nM) |
|---|---|---|---|
| Compound 1 | A | Compound 7-2 | A |
| Compound 3-1 | A | Compound 8 | A |
| Compound 3-2 | A | Compound 8-1 | A |
| Trifluoroacetate salt of Compound 5 | A | Compound 8-2 | A |
| Compound 5-1 | A | Compound 9 | A |
| Compound 5-2 | A | Compound 9-1 | A |
| Compound 6-1 | A | Compound 10 | A |
| Compound 6-2 | A | Trifluoroacetate salt of Compound 11 | A |
| Compound 7-1 | A | Trifluoroacetate salt of Compound 12 | A |
| Note: A < 20 nM, 20 nM ≤ B < 50 nM, 50 nM ≤ C < 200 nM | | | |

Conclusion: The compounds of the present invention, such as the example compounds, have good agonistic and/or activating effects on the enzyme activity of soluble guanylate cyclase (sGC).

**Test example 3: Mouse Pharmacokinetic Assay**

**Experimental objective:** In this experiment, a single dose of test compounds was administered to ICR mice intravenously and intragastrically, the concentrations of the test compounds in the plasma of mice were measured, and the

pharmacokinetic characteristics and bioavailability of the test compounds in mice were evaluated.

**Experimental animals:** male ICR mice, 20-35 g, purchased from Chengdu Dossy Experimental Animals Co., Ltd.

**Experimental method:** On the day of the experiment, the ICR mice were randomly grouped according to their body weight. The animals were fasted but with water available overnight before the administration, and fed 4 h after the administration.

| Group | Administration information | | | | | | |
|---|---|---|---|---|---|---|---|
| | Test compound | Administration dosage* (mg/kg) | Administration concentration | Administration volume (mL/kg) | Collected sample | Administration mode | Vehicle |
| | | | (mg/mL) | | | | |
| G1 | Compound of the present invention | 2.5 | 0.5 | 5 | Plasma | Intravenous administration | 5% DMA+5% HS-15+90% NS or 10% DMA+10% HS-15+80% NS |
| G2 | | 10 | 1 | 10 | Plasma | Oral administration (intragastric administration) | 5% DMSO+5% Solutol+10% PEG 400+80% (20% SBE-β-CD) |
| *Dosage is calculated based on the free form. | | | | | | | |

Sampling: Blood was taken from the orbits at specified time points, and placed in an EDTAK2 centrifuge tube. The tube was centrifuged at 5000 rpm for 10 min, and the plasma was collected.

Time points for plasma collection in G1 group: 0, 5 min, 15 min, 30 min, 1 h, 2 h, 4 h, 7 h, and 24 h.

Time points for plasma collection in G2 group: 0, 5 min, 15 min, 30 min, 1 h, 2 h, 4 h, 7 h, and 24 h.

Before analysis and detection, all the samples were stored at -60°C or lower. The samples were analyzed quantitatively by LC-MS/MS.

Table 3-1 PK data of test compound (i.e., drug) in mice

| Compound No. | Mode of administration: i.g. | AUC ($h\cdot ng\cdot mL^{-1}$) |
|---|---|---|
| Compound 1-1 | 10 mg/kg | > 10000 |
| Compound 1-2 | 10 mg/kg | > 10000 |
| Compound 3-1 | 10 mg/kg | > 10000 |
| Compound 5-1 | 10 mg/kg | > 10000 |
| Compound 5-2 | 10 mg/kg | > 10000 |
| Compound 6-1 | 10 mg/kg | > 10000 |
| Compound 6-2 | 10 mg/kg | > 10000 |
| Compound 7-2 | 10 mg/kg | > 10000 |
| Compound 8-1 | 10 mg/kg | > 10000 |
| Compound 8-2 | 10 mg/kg | > 10000 |
| Compound 9-1 | 10 mg/kg | > 10000 |
| *Notes: i.g. (intragastric) administration of compounds. | | |

Conclusion: The compounds of the present invention, such as the example compounds, have good oral absorption performance in mice.

**Test example 4: Rat Pharmacokinetic Assay**

Experimental animals: male SD rats, 180-200 g, purchased from Chengdu Dossy Experimental Animals Co., Ltd.

Experimental design: On the day of the experiment, the SD rats were randomly grouped according to their body weight. The animals were fasted but with water available overnight before the administration, and fed 4 h after the administration.

| Group | Administration information | | | | | |
|---|---|---|---|---|---|---|
| | Test compound | Administratio n dosage (mg/kg) | Administratio n concentration (mg/mL) | Administratio n volume (mL/kg) | Collected sample | Administratio n mode |
| G1 | Compound of the present invention | 2.5 | 0.5 | 5 | Plasma | Intravenous administration |
| G2 | | 10 | 1 | 10 | Plasma | Intragastric administration |
| Note: vehicle for intravenous administration: 5% DMA+5% HS-15+90% NS or 10% DMA+10% HS-15+80% NS; vehicle for intragastric administration: 5% DMSO+5% Solutol+10% PEG 400+80% (20% SBE-β-CD). | | | | | | |

Blood was taken from the orbits at specified time points, and placed in an $EDTAK_2$ centrifuge tube. The tube was centrifuged at 5000 rpm for 10 min, and the plasma was collected.

Blood sampling time points for the intravenous administration group: 0, 5 min, 15 min, 30 min, 1 h, 2 h, 4 h, 7 h and 24 h; blood sampling time points for the intragastric administration group: 0, 5 min, 15 min, 30 min, 1 h, 2 h, 4 h, 7 h and 24 h. Before analysis and detection, all the samples were stored at -60°C or lower. The samples were analyzed quantitatively by LC-MS/MS.

Table 4-1 PK data of test compound (i.e., drug) in rats

| Compound No. | Mode of administration: i.g. | AUC ($h\cdot ng\cdot mL^{-1}$) |
|---|---|---|
| Compound 1-1 | 10 mg/kg | > 10000 |
| Compound 3-1 | 10 mg/kg | > 10000 |
| Compound 5-1 | 10 mg/kg | > 10000 |
| Compound 6-1 | 10 mg/kg | > 10000 |
| Compound 6-2 | 10 mg/kg | > 10000 |
| Compound 8-1 | 10 mg/kg | > 10000 |
| Compound 8-2 | 10 mg/kg | > 10000 |
| Compound 9-1 | 10 mg/kg | > 10000 |
| *Notes: i.g. (intragastric) administration of compounds. | | |

Conclusion: The compounds of the present invention, such as the example compounds, have good oral absorption performance in rats.

**5. hERG Potassium Channel Activity Assay**

**Experimental platform:** Manual electrophysiological patch-clamp system

**Cell line:** Chinese hamster ovary (CHO) cell line stably expressing hERG potassium channel

**Experimental method:** In Chinese Hamster Ovary (CHO) cells stably expressing hERG potassium channel, the whole-cell patch-clamp technique was used to record hERG potassium channel current at room temperature. The glass microelectrodes were pulled from glass electrode blanks (BF150-86-10, Sutter) by a glass microelectrode puller. The tip resistance of the microelectrodes after being filled with intrapipette solution was about 2-5 MΩ. The glass microelectrodes

were connected to the patch-clamp amplifier by inserting them into an amplifier probe. The clamping voltage and data recording were controlled and recorded by the pClamp 10 software through a computer, with a sampling frequency of 10 kHz and a filtering frequency of 2 kHz. After the whole cell records were obtained, the cells were clamped at -80 mV, and the step voltage that elicited the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was initiated after the hERG potassium current was confirmed to be stable (at least 1 minute). The compound was administered for at least 1 minute at each test concentration, and at least 2 cells ($n \geq 2$) were tested at each concentration.

**Data processing:** Data analysis and processing were performed using pClamp 10, GraphPad Prism 5, and Excel software. The degree of inhibition of hERG potassium current (peak value of hERG tail current elicited at -50 mV) at different compound concentrations was calculated according to the following formula:

$$\text{Inhibition\%} = [1 - (I / I\text{o})] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1 + 10\text{^}((\text{LogIC}_{50}\text{-X})\text{*HillSlope}))$$

where X represents the Log value of the tested concentration of the test compound, Y represents the percentage inhibition at the corresponding concentration, and Bottom and Top represent the minimum and maximum percentage inhibitions, respectively.

Conclusion: The compounds of the present invention, such as the example compounds, have no obvious inhibitory effect on the hERG potassium ion channel.

### 6. CYP450 Enzyme Activity Inhibition Assay

The objective of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the reaction was completed, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention, such as the example compounds, have no significant or only weak inhibitory effects on CYP450 enzymes.

### 7. Pharmacodynamic Study on Relaxation of Thoracic Aortic Vascular Rings in SD Rats

Male SD rats, 8 weeks old and of SPF grade, were purchased from Beijing Vital River Laboratory Animal Technology Co., Ltd. The animals were acclimatized for at least one week upon arrival at the animal facility. Prior to the experiment, the rats were weighed and randomly grouped according to their body weight. On the day of the experiment, the rats were deeply anesthetized with Zoletil 50 (20 mg/kg, i.p.) and Xylazine (8 mg/kg, i.p.). The thoracic cavity was quickly opened, and the aorta was carefully isolated. A segment of the descending aorta was excised and placed in a petri dish containing K-H solution saturated with carbogen (95% $O_2$ + 5% $CO_2$). After removing connective tissue surrounding the vessel, vascular rings of approximately 3-5 mm in length were prepared. The vascular rings were suspended using custom-made hooks in an isolated tissue perfusion bath containing 37°C constant-temperature K-H solution. The bath was aerated with carbogen, and connected to a force transducer (Chengdu Instrument Factory, JZ101H). The force transducer was connected to a multichannel electrophysiological signal recording system (Chengdu Instrument Factory, RM6240E). The vascular rings were rinsed with K-H solution and equilibrated for 90 minutes under a resting tension of 3 g. Subsequently, the vascular rings were pre-contracted with $10^{-6}$ M norepinephrine in the bath until a stable tension was achieved. The test compound was then added to the bath in a cumulative concentration-gradient manner (from low to high) to induce vasodilation, with a 5-minute interval between each addition. The changes in tension of the thoracic aortic vascular rings were observed. The percentage of vasodilation was calculated for each concentration.

Conclusion: The compounds of the present invention, such as the example compounds, have significant vasodilatory effects in rat thoracic aortic vascular rings.

### 8. SHR Rat Telemetric Blood Pressure Monitoring

Surgical implantation of blood pressure telemetry transmitter: One day prior to surgery, the DSI transmitter was sterilized by immersion in a 2% glutaraldehyde solution for 8-10 h; animals were weighed, and anesthetized with Xylazine (8 mg/kg, i.p.) and Zoletil 50 (20 mg/kg, i.p.); and the animals were fasted overnight before the procedure. The transmitter implantation procedure was performed on Day 1 of the study, as detailed below. The abdominal skin was aseptically disinfected, and a longitudinal incision was made. The abdominal organs were dissected to expose the abdominal aorta. The pressure-sensing catheter of the transmitter was inserted into the abdominal aorta. Hemostasis and closure of the vascular puncture site were achieved with bioadhesives, and then the transmitter body was secured to the abdominal wall. The muscle and skin layers were sutured, the surgical site was disinfected, and meloxicam was administered subcutaneously for analgesia. Following surgery, the animals were placed in a 37°C constant-temperature incubator to recover until resumption of voluntary motor activity, and then individually housed in their cages. For the first three postoperative days, the animals received daily subcutaneous injections of gentamicin sulfate (4-8 mg/kg) for infection prophylaxis and meloxicam for analgesia.

Blood pressure monitoring: After approximately 10 days of postoperative recovery, baseline blood pressure was recorded over a 24-hour period. Rats were assigned to groups based on baseline blood pressure. Following grouping, the rats were administered with the test compound. After a single administration (oral), blood pressure was monitored for 24 hours, during which changes in systolic blood pressure, diastolic blood pressure, mean arterial pressure, and heart rate were recorded. Raw data were used to calculate the mean systolic blood pressure, mean diastolic blood pressure, mean arterial pressure, and mean heart rate at regular intervals (conventionally set at 30 minutes). A p-value of less than 0.05 was considered statistically significant.

Conclusion: The compounds of the present invention, such as the example compounds, have significant blood pressure-lowering effects in rats.

### 9. Beagle Dog Pharmacokinetic Assay

**Experimental animals:** Male beagle dogs, about 8-11 kg, 5-6 dogs/compound.

**Experimental method:** On the day of the experiment, 5-6 beagle dogs/compound were randomly grouped according to their body weight. The animals were fasted with water available for 12-14 h one day before the administration, and were fed 4 h after the administration.

| Group | Number | Administration information | | | | | |
|---|---|---|---|---|---|---|---|
| | Male | Test compound | Administration dosage (mg/kg) | Administration concentrati | Administration volume (mL/kg) | Collected sample | Administration mode |
| | | | | on (mg/mL) | | | |
| G1 | 3 | Test compound | 1 | 0.5 | 2 | Plasma | Intravenous administration |
| G2 | 3 | | 5 | 1 | 5 | Plasma | Intragastric administration |
| Note: vehicle for intravenous administration: 5% DMA + 5% Solutol + 90% Saline; vehicle for intragastric administration: 0.5% MC. | | | | | | | |

(DMA: dimethylacetamide; Solutol: polyethylene glycol-15-hydroxystearate; Saline: physiological saline; 0.5% MC: 0.5% aqueous solution of methylcellulose.)

Before and after the administration, 1 ml of blood was taken from the jugular veins or limb veins and placed in an EDTAK2 centrifuge tube. The tube was centrifuged at 5000 rpm at 4°C for 10 min, and plasma was collected. Blood sampling time points for groups G1 and G2 (the intravenous group and intragastric administration group) were: 0, 5 min, 15 min, 30 min, 1 h, 2 h, 4 h, 6 h, 8 h, 10 h, 12 h, 24 h, and 48 h. Before analysis and detection, all samples were stored at - 80°C. The samples were analyzed quantitatively by LC-MS/MS.

**Conclusion:** The compounds of the present invention, such as the example compounds, have good oral absorption performance in beagle dogs.

### 10. Monkey Pharmacokinetic Assay

**Experimental animals:** Male cynomolgus monkeys, 3-5 kg, 3-6 years old, 4-6 monkeys/compound.
**Experimental method:** On the day of the experiment, 4-6 monkeys/compound were randomly grouped according to their body weight. The animals were fasted with water available for 14 to 18 h one day before the administration, and were fed 4 h after the administration.

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Administration mode |
| G1 | 2 | Compound | 1 | 1 | 1 | Plasma | Intravenous administration |
| G2 | 2 | | 10 | 2 | 5 | Plasma | Intragastric administration |
| G3 | 3 | Compound | 1 | 1 | 1 | Plasma | Intravenous administration |
| G4 | 3 | Compound | 10 | 2 | 5 | Plasma | Intragastric administration |
| Note: vehicle for intravenous administration: 5% DMA + 5% Solutol + 90% Saline; vehicle for intragastric administration: 0.5% MC. | | | | | | | |

(DMA: dimethylacetamide; Solutol: polyethylene glycol-15-hydroxystearate; Saline: physiological saline; 0.5% MC: 0.5% aqueous solution of methylcellulose.
Before and after the administration, 1.0 mL of blood was taken from the limb veins and placed in an EDTAK2 centrifuge tube. The tube was centrifuged at 5000 rpm at 4°C for 10 min, and the plasma was collected. Blood sampling time points for the intravenous group and intragastric administration group were: 0, 5 min, 15 min, 30 min, 1 h, 2 h, 4 h, 6 h, 8 h, 10 h, 12 h, and 24 h. Before analysis and detection, all samples were stored at -80°C, and the samples were quantitatively analyzed by LC-MS/MS.
Conclusion: The compounds of the present invention, such as the example compounds, have good oral absorption performance in monkeys.

### 11. Stability Test in Mouse Liver Microsomes

At 37°C, 1 μM of the test compound was incubated with mouse liver microsomes (0.5 mg/mL) supplemented with an NADPH regeneration system for 5, 10, 20, 30 and 60 minutes, and then the LC-MS/MS method was used to detect the concentration of the test compound in the resulting sample. The half-life ($T_{1/2}$) and intrinsic clearance ($CL_{int(mic)}$) of the compound in mouse liver microsome solution were obtained by calculating the remaining percentage of the compound at the corresponding time points.
Conclusion: The compounds of the present invention have good stability in mouse liver microsomes.

### 12. Stability Test in Rat Microsomes

At 37°C, 1 μM of the test compound was incubated with rat liver microsomes (0.5 mg/mL) supplemented with an NADPH regeneration system for 5, 10, 20, 30 and 60 minutes, and then the LC-MS/MS method was used to detect the concentration of the test compound in the resulting sample. The half-life ($T_{1/2}$) and intrinsic clearance ($CL_{int(mic)}$) of the compound in rat liver microsome solution were obtained by calculating the remaining percentage of the compound at the corresponding time points.
Biological assay results:

| Compound | $T_{1/2}$ (min) | $CL_{int(mic)}$ (μL/min/mg) |
|---|---|---|
| Compound 1-1 | 43.6 | 31.8 |
| Compound 5-1 | 140 | 9.87 |
| Compound 6-1 | 112 | 12.4 |
| Compound 6-2 | 117 | 11.8 |
| Compound 8-1 | 152 | 9.13 |
| Compound 8-2 | 98.1 | 14.1 |
| Compound 9-1 | 126 | 11.0 |
| Control compound 1 | 11.0 | 126 |

Conclusion: The compounds of the present invention have good stability in rat liver microsomes, and compared with Control Compound 1, the clearance rate of the compounds of the present invention in rat liver microsomes was significantly reduced, and the half-life was significantly prolonged.

**13. Stability Test in Monkey Microsomes**

At 37°C, 1 μM of the test compound was incubated with monkey liver microsomes (0.5 mg/mL) supplemented with an NADPH regeneration system for 5, 10, 20, 30 and 60 minutes, and then the LC-MS/MS method was used to detect the concentration of the test compound in the resulting sample. The half-life ($T_{1/2}$) and intrinsic clearance ($CL_{int(mic)}$) of the compound in monkey liver microsome solution were obtained by calculating the remaining percentage of the compound at the corresponding time points.
Conclusion: The compounds of the present invention have good stability in monkey liver microsomes.

**14. Stability Test in Canine Microsomes**

At 37°C, 1 μM of the test compound was incubated with canine liver microsomes (0.5 mg/mL) supplemented with an NADPH regeneration system for 5, 10, 20, 30 and 60 minutes, and then the LC-MS/MS method was used to detect the concentration of the test compound in the resulting sample. The half-life ($T_{1/2}$) and intrinsic clearance ($CL_{int(mic)}$) of the compound in canine liver microsome solution were obtained by calculating the remaining percentage of the compound at the corresponding time points.
Conclusion: The compounds of the present invention have good stability in canine liver microsomes.

**15. Stability Test in Human Microsomes**

At 37°C, 1 μM of the test compound was incubated with human liver microsomes (0.5 mg/mL) supplemented with an NADPH regeneration system for 5, 10, 20, 30 and 60 minutes, and then the LC-MS/MS method was used to detect the concentration of the test compound in the resulting sample. The half-life ($T_{1/2}$) and intrinsic clearance ($CL_{int(mic)}$) of the compound in human liver microsome solution were obtained by calculating the remaining percentage of the compound at the corresponding time points.
Conclusion: The compounds of the present invention have good stability in human liver microsomes.

**16. Caco2 Permeability Assay**

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (5 μM) was added to the administration well on the apical side or basolateral side. A DMSO-containing transport buffer solution was added to the corresponding receiving well. After incubation for 2 hours at 37°C ± 1°C, the cell plate was removed, and an appropriate amount of sample was taken from both the apical side and basolateral side and transferred to a new 96-well plate. Subsequently, acetonitrile containing an internal standard was added to precipitate the protein. The samples were analyzed using LC-MS/MS, and the concentrations of the compound of the present invention and the control compound were determined. Concentration data were used to calculate apparent permeability coefficients for transport from the apical side to the basal side, and from the basolateral side to the apical side of the cell monolayer, and thus to calculate the efflux ratio. The integrity of the cell monolayer after 2 hours of incubation was evaluated by the leakage of Lucifer Yellow.
Conclusion: The compounds of the present invention, such as the example compounds, have good Caco2

permeability.

**17. Toxicological Experiment in Rats**

Experimental animals: 120 SD rats were purchased, and 106 rats were expected to be used, with equal numbers of males and females; at the time of administration, the animals were aged 6-7 weeks, with the body weight of 180 g-220 g, and the individual's body weight within ± 20% of the mean body weight; and rats were acclimatized for at least 3 days before the experiment. In the experiment, a vehicle control group and compound dose groups (one dose constitutes one group) were set. For the main test groups, 10 rats were used per group (with 4 rats per group for the recovery period), and for the TK groups, 6 rats were used per group, with equal numbers of males and females.

Experimental procedure:

General clinical observations: On the day of administration, animals were closely observed within 0-6 h after the administration, and during the subsequent administration period and recovery period, observations were performed at least twice daily (once in the morning and once in the afternoon).

Body weight: Body weight was measured once before the administration, then once on Days 4, 7, 11, and 14 during the administration period, and once during the recovery period.

Food consumption: Food consumption was measured twice during the administration period and once during the recovery period. On Day 1, the amount of feed supplied to each cage was determined. On Day 2, at approximately the same time, the remaining feed was weighed. The difference between these two values represented the total 24-h feed intake of animals per cage. This value was then divided by the number of animals per cage to obtain the 24-h feed intake per animal.

Detection of serum biochemical parameters:
Test animals: Surviving animals in the main test groups.
Detection time: On the day of necropsy at the end of the administration period and at the end of the recovery period.
Blood sampling method: Animals were fasted for at least 8 h prior to blood collection. About 1.0 ml blood was collected from the jugular vein without anticoagulant. Blood samples were centrifuged to obtain serum. Serum biochemical parameters were determined using a Roche Cobas C311 full-automatic biochemical analyzer. The specific detection parameters were as follows:

| Parameter | Unit | Parameter | Unit |
|---|---|---|---|
| Alanine aminotransferase (ALT) | U/L | Creatinine (CRE) | μmol/L |
| Aspartate aminotransferase (AST) | U/L | Calcium (Ca) | mmol/L |
| Total protein (TP) | g/L | Phosphorus (P) | mmol/L |
| Albumin (ALB) | g/L | Total cholesterol (TCHO) | mmol/L |
| Globulin (GLB) | g/L | Triglyceride (TG) | mmol/L |
| Albumin/globulin ratio | Unitless | Total bilirubin (TBIL) | μmol/L |
| Alkaline phosphatase (ALP) | U/L | Sodium (Na) | mmol/L |
| γ-glutamyl transpeptidase (GGT) | U/L | Potassium (K) | mmol/L |
| Glucose (GLU) | mmol/L | Chlorine (Cl) | mmol/L |
| Urea (UREA) | mmol/L | Creatine kinase (CK) | U/L |
| Low density lipoprotein cholesterol (LDL-C) | mmol/L | Direct bilirubin (DBIL) | μmol/L |

Detection of hematological parameters:
Test animals: Surviving animals in the main test groups.
Detection time: On the day of necropsy at the end of the administration period and at the end of the recovery period.
Blood sampling method: About 1.0 ml of blood was collected from the jugular vein and anticoagulated with EDTA-K2. Hematological parameters were detected using a Siemens ADVIA 2120i hematology analyzer. The specific detection parameters were as follows:

| Hematological and coagulation parameters | Unit | Analytical method |
|---|---|---|
| Red blood cell count (RBC) | $10^{12}$/L | Instrument counting |
| Hemoglobin concentration (Hb) | g/L | Instrument counting |

(continued)

| Hematological and coagulation parameters | Unit | Analytical method |
|---|---|---|
| Hematocrit (HCT) | % | Instrument counting |
| Mean corpuscular volume (MCV) | fl | Instrument counting |
| Mean corpuscular hemoglobin (MCH) | Pg | Instrument counting |
| Mean corpuscular hemoglobin concentration (MCHC) | g/L | Instrument counting |
| Red blood cell distribution width (RDW) | % | Instrument counting |
| Mean platelet volume (MPV) | fl | Instrument counting |
| Platelet count (PLT) | $10^9$/L | Instrument counting |
| Total white blood cell count (WBC) | $10^9$/L | Instrument counting |
| White blood cell differential count (DC) | % | Instrument counting |

Toxicokinetic detection:
Animals in TK satellite groups were subjected to TK blood sampling on Days 1 and 14 of the experiment.
Blood sampling time points: Prior to administration, and at 0.25 h, 0.5 h, 1 h, 2 h, 4 h, 8 h, 24 h, and 48 h after the administration (48 h being for the final administration). The blood sampling time points for the HSK38966 BID administration group were: Prior to administration, and at 0.5 h, 1 h, 2 h, 4 h, 6.5 h, 7 h, 8 h, 24 h, 48 h after the first administration of the day (48 h being for the final administration).
Animals for sampling: All surviving animals in the TK groups of the administration groups
Sampling site: Jugular vein
Sampling volume: About 0.2 mL
Anticoagulant: EDTA-$K_2$
Blood sample processing: Whole blood samples were temporarily stored in an ice box prior to centrifugation. Samples were centrifuged at about 1800×g and 2-8°C for 10 minutes. The resulting samples were stored below -20°C until analysis.

Gross necropsy and organ weight:
Necropsy was performed on all animals that died accidentally and all animals scheduled for euthanasia in the main test groups.

Animals found dead were stored in a refrigerator at 2-8°C and necropsied as soon as possible within 24 hours.

At 24 h after the final administration (Day 15), following blood sampling from the jugular vein, animals were anesthetized with 20% urethane, euthanized by exsanguination from the abdominal aorta, and subjected to gross necropsy. Gross observation was performed during necropsy, including but not limited to abnormalities in the external surface, external orifices, cranial cavity, thoracic cavity, abdominal cavity, pelvic cavity and their contents. Any abnormalities should be promptly recorded with regard to the location, color, shape and size of the abnormal tissues or organs.

During necropsy, the weights of the required organs were collected, and the organ/body weight ratio and organ/brain weight ratio were calculated.

Histopathological examination:
The following tissues and/or organs were collected during necropsy, fixed in 10% formalin solution (testes/epididymides fixed in Davidson's fixative), and subjected to histopathological examination if necessary.

| Tissue/organ | Weighed |
|---|---|
| Abnormalities found on gross observation | |
| Adrenal gland | |
| Brain | √ |
| Epididymis | |
| Heart | √ |
| Kidney | √ |
| Large intestine (cecum, colon, rectum) | |
| Liver | √ |
| Lung (including trachea) | |
| Lymph node (mesenteric) | |
| Ovary and fallopian tube | |

(continued)

| Tissue/organ | Weighed |
|---|---|
| Pancreas | |
| Small intestine (duodenum, jejunum, ileum) | |
| Spleen | √ |
| Stomach | |
| Testis | |
| Thymus | √ |
| Uterus and cervix | |

Statistical methods:
SPSS 23 was used for statistical analysis. The mean and standard deviation (SD) were calculated for body weight, hematological parameters, serum biochemical parameters, organ weights and organ weight ratios in each group. Data were analyzed as follows: (1) Levene's test was used to evaluate homogeneity of variances. If variance was homogeneous ($P > 0.05$), one-way analysis of variance (ANOVA) was performed. If variance was heterogeneous ($P \leq 0.05$), Dunnett's T3 test was used for comparisons between groups (at the 0.05 and 0.01 significance levels). (2) If the result of analysis of variance showed a statistical difference ($P \leq 0.05$), Dunnett's t-test was further used for comparisons between groups (at the 0.05 and 0.01 significance levels). If the result of analysis of variance showed no statistical difference ($P > 0.05$), statistical analysis was terminated.

Experimental recording and preservation:
Study protocol, animal grouping record form, test article receipt record form, test article formulation record form, animal body weight and dosing operation record form, animal daily observation record form, organ weighing record form, and experimental animal necropsy record form.

Conclusion: The compounds of the present invention have a good safety profile in rats at set doses.

### 18. Assessment of Renal Function by Analysis of Proteinuria (Urine Protein-to-Creatine Ratio, uPCR) in Female Rats

Female RenTG/L-NAME-supplemented rats were treated with a placebo (set as 100%) or the compounds, respectively. Urine protein-to-creatinine ratio (uPCR) was determined and calculated.

Conclusion: The compounds of the present invention can result in a reduction in proteinuria in female RenTG/L-NAME-supplemented rats.

### 19. CYP3A4 Induction Activity Assay (PXR Activation)

Objective: The objective of this study was to evaluate the potential of the test compound to induce the activity of drug-metabolizing enzymes via in vitro activation of the activity of PXR.

1. Cell seeding

    1) DPX2 cells were cultured in growth medium containing 10% fetal bovine serum.
    2) DPX2 cells were cultured in a T-75 flask in an incubator at 37°C and 5% CO2 with 95% relative humidity. Cells were digested when they reached 80%-90% confluence.
    3) The cell monolayer in the T-75 flask was washed with 10 mL of PBS. PBS was aspirated, and then 3-5 mL of trypsin was added, followed by digestion at 37°C for 5 minutes or until the cells detached into suspension. The trypsinization was terminated by adding excess medium containing fetal bovine serum.
    4) The cell suspension was transferred to a conical-bottom centrifuge tube and centrifuged at 150 g for 5 minutes at room temperature. The supernatant was carefully aspirated, cells were resuspended in treatment medium, and the
    suspension was adjusted to a concentration of $3.2 \times 10^5$ cells/mL (incubation time: 24 hours, seeding density: $4.0 \times 10^5$ cells/mL). 25 μL of cell suspension was added per well in a 384-well cell culture plate. The cell plate was placed in an incubator at a temperature of 37°C and 5% CO2 with 95% humidity for 24 hours.

2. Compound formulation

1) 1000× stock solutions of test compounds, positive control (rifampicin), and negative control (propranolol) were prepared with DMSO. The final concentrations of the positive control (rifampicin) were 1 μM and 10 μM, and the final concentration of the negative control (propranolol) was 10 μM. The final concentrations of the test compounds were 10, 1, 0.1 uM or EC50 (30, 10, 3, 1, 0.3, or 0.1 uM). The final DMSO concentration was 0.1%.
2) The cell culture plate was removed from the incubator, and 25 nL of positive control, negative control, or test compound stock solution was directly added using an Echo system, with three replicates per concentration. The cell plate was returned to the incubator and incubated for another 48 hours (24 hours).
3) Prior to initiating the experiment with the substrate, cell morphology and monolayer integrity were examined to ensure that the monolayer was of acceptable quality for studies.

3. Quantitative PXR activation assay

1) After 48 hours (24 hours) of drug treatment, the cultures were subjected to quantitative PXR activation assay.
2) CellTiter-Fluor™ cell viability assay kit and One-Glo Luciferase assay reagent were equilibrated to room temperature. GF-AFC substrate (10 μL) was added to Assay Buffer (10 ml) to form a 2X reagent, which was then diluted with 10 ml of PBS to yield a 1X reagent. ONE-Glo Luciferase substrate was added to ONE-Glo Luciferase Assay Buffer.
3) The culture plate was removed from the incubator, the medium was discarded, and 1X CellTiter-Fluor™ reagent was poured into a loading slot. 25 μL of reagent was added to each well of the plate using a liquid handling workstation, followed by incubation for 30 minutes in the incubator.
4) The cell culture plate was removed from the incubator and allowed to cool slightly to room temperature, and fluorescence was measured using a fully automated quantitative microplate reader with excitation at 400 nm and emission at 505 nm.
5) ONE-Glo reagent was poured into a loading slot (25 μL/well), the plate was gently mixed and incubated at room temperature for 5 minutes, and luminescence was measured.

4. Data analysis
All data were calculated using Microsoft Excel.

1) Luciferase activity was expressed as RFU/RLU, where RLU was the average luminescence intensity of three replicates of each compound at each concentration, and RFU was the average fluorescence intensity of three replicates of each compound at each concentration.
The fold activation of mRNA was calculated using the following formula:

$$\text{Fold activation} = (\text{RLU test}/\text{RFU test}) / (\text{RLUvehicle}/\text{RFUvehicle})$$

2) The percentage of cell viability for the compounds was calculated according to the following formula:

$$\text{Cell viability \%} = (\text{RFUtest} / \text{RFUvehicle}) \times 100$$

3) The percentage relative to the positive control was calculated according to the following formula:

Percent of positive control (%) = (Fold activation test / Fold activation Positive control) × 100

Conclusion: The compounds of the present invention have no obvious induction effect on CYP3A4.

**20. CYP3A4 Induction Activity Assay (Enzyme Activity and mRNA)**

The objective of this study was to evaluate the effects of the test substance on the enzyme activity and gene expression levels of cytochrome P450 isozymes CYP1A2, CYP2B6, and CYP3A4 using an in vitro hepatocyte induction experiment.

Cryopreserved human hepatocytes from three donors were incubated with the test substance at various concentrations (≥ 5 concentration levels) at 37°C for 48 hours, with freshly prepared culture medium replaced every 24 hours. Lactate dehydrogenase release in the culture medium was measured 24 and 48 hours after cell incubation to assess potential cytotoxicity of the test substance. After the second administration, concentrations of the test substance in the culture medium were determined 0, 5, and 24 hours after incubation to evaluate the parent drug concentrations of the

test substance in human hepatocyte culture medium. After cryopreserved human hepatocytes from three donors were incubated with the test substance for 48 hours, the cell culture medium was removed, and the cells were washed with Hank's Balanced Salt Solution (HBSS) pre-warmed to 37°C. Enzyme-specific substrate was then added, followed by incubation at 37°C for 30 minutes. The amount of metabolites formed from each substrate was quantitatively analyzed by liquid chromatography-tandem mass spectrometry. Gene expression levels in the cells were assessed by the real-time fluorescent quantitative PCR method.

Conclusion: The compounds of the present invention have no obvious induction effect on CYP3A4.

**21. P-gp Transporter Inhibition Assay**

In this study, the MDR1-MDCK II monolayer cell model was used to evaluate the inhibitory effect of the test substance on the activity of P-glycoprotein transporter.

In the experiment, MDR1-MDCK II cells were seeded into a 96-well cell culture plate and cultured continuously for 7 days before being used in the transport experiment. Digoxin (a known P-gp substrate) was added bidirectionally in the presence and absence of the test substance, and co-incubated with the test substance at various concentrations (0-30 μM). After 150 minutes of incubation, samples from the receiving end were collected, and the digoxin concentration was determined by the liquid chromatography-tandem mass spectrometry (LC-MS/MS) method. By calculating the efflux ratio (ER) of digoxin with and without the test substance, the percentage of P-gp transport activity of MDR1-MDCK II cells at different test concentrations of the test substance (%VC (Vehicle Control, solvent control), the percentage of P-gp transport activity with and without the test substance) was obtained, and the half-maximal inhibitory concentration ($IC_{50}$) was calculated from this.

Conclusion: The compounds of the present invention have no obvious inhibitory effect on the P-gp transporter.

**22. BCRP Transporter Inhibition Assay**

The objective of this study was to evaluate the inhibitory effect of the test substance on the activity of breast cancer resistance protein transporter using the Caco-2 monolayer cell model.

In the experiment, Caco-2 cells were seeded into a 96-well cell culture plate and cultured continuously for 22 days before being used in the transport experiment. 5.00 μM of estrone 3-sulfate, a substrate of BCRP, was added bidirectionally with or without the test substance. The cells were co-incubated with the test substance at various concentrations (0-30 μM). After 120 minutes of incubation, samples from the receiving end were collected, and the content of estrone 3-sulfate was detected by the liquid chromatography-tandem mass spectrometry (LC-MS/MS) method. By calculating the efflux ratio (ER) of estrone 3-sulfate with and without the test substance, the percentage of BCRP transport activity of Caco-2 cells at different test concentrations of the test substance (%VC (Vehicle Control, solvent control), the percentage of BCRP transport activity with and without the test substance) was obtained, and the half-maximal inhibitory concentration ($IC_{50}$) was calculated from this.

Conclusion: The compounds of the present invention have no obvious inhibitory effect on the BCRP transporter.

**23. SLC Transporter Inhibition Assay**

The objective of this study was to evaluate the inhibitory effects of the test substance on the activities of the transporters OATP1B1, OATP1B3, OAT1, OAT3, OCT, MATE1 and MATE2-K.

HEK293-OATP1B1, OATP1B3, OAT1, OAT3, OCT, MATE1 or MATE2-K cells were incubated with and without the test substance (0-30 μM) for the corresponding time. Samples were then collected, and the substrate content in the samples was detected by liquid chromatography-tandem mass spectrometry (LC-MS/MS). By calculating the transport activity of the transporter with and without the test substance, the percentage of transporter activity of the transporter-expressing cells at different administration concentrations of the test substance (%VC (Vehicle Control, solvent control), the percentage of transport activity of the transporter with and without the test substance) was obtained, and the half-maximal inhibitory concentration ($IC_{50}$) was calculated from this.

Conclusion: The compounds of the present invention have no obvious inhibitory effect on the SLC transporters.

## Claims

1. A compound, or a stereoisomer, tautomer, deuterated compound, solvate, prodrug, metabolite, pharmaceutically acceptable salt, or co-crystal thereof, **characterized in that** the compound is a compound represented by general formula (I), wherein

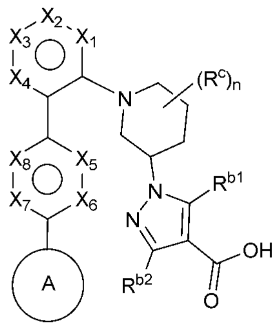

(I);

$X_1$ is selected from $CR^1$ or N, $X_2$ is selected from $CR^2$ or N, $X_3$ is selected from $CR^3$ or N, $X_4$ is selected from $CR^4$ or N, $X_5$ is selected from $CR^5$ or N, $X_6$ is selected from $CR^6$ or N, $X_7$ is selected from $CR^7$ or N, and $X_8$ is selected from $CR^8$ or N;

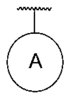

is selected from $C_{3-12}$ carbocyclyl or 4- to 12-membered heterocyclyl, wherein the carbocyclyl or heterocyclyl is optionally substituted with 1 to 4 $R^a$;
$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}\ alkyl)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl, or heterocyclyl is optionally substituted with 1 to 4 $R^k$;
or $R^7$ and $R^a$, together with the atoms to which they are attached, form a 4- to 8-membered carbocycle or a 4- to 8-membered heterocycle, wherein the carbocycle or heterocycle is optionally substituted with 1 to 4 $R^k$;
each $R^k$ is independently selected from deuterium, halogen, OH, =O, CN, $NH_2$, COOH, $CONH_2$, $C_{1-6}$ alkyl, $OC_{1-6}$ alkyl, $SC_{1-6}$ alkyl, $C_{2-6}$ alkenyl, $C_{2-6}$ alkynyl, $NHC_{1-6}$ alkyl, $N(C_{1-6}\ alkyl)_2$, -O-$C_{3-6}$ carbocyclyl, -O-3- to 7-membered heterocyclyl, -NH-$C_{3-6}$ carbocyclyl, -NH-3- to 7-membered heterocyclyl, -$C_{0-4}$ alkylene-$C_{3-6}$ carbocyclyl, or -$C_{0-4}$ alkylene-3- to 7-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$ alkoxy; and
n is selected from 0, 1, 2, 3, or 4;
provided that
1) $R^2$ is not Cl or $CF_3$;
or 2) at least one of $X_1$, $X_2$, $X_3$, $X_4$, $X_5$, and $X_8$ is N;
or 3) at least one of $R^1$, $R^3$, and $R^4$ is not H;
or 4)

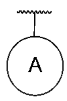

is not selected from

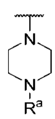 , 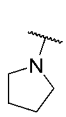 , 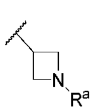 , or 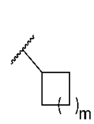 ,

wherein m is selected from 0, 1, 2, 3, or 4;
or 5) $R^7$ and $R^a$, together with the atoms to which they are attached, form a 4-to 8-membered carbocycle or a 4- to 8-membered heterocycle, wherein the carbocycle or heterocycle is optionally substituted with 1 to 4 $R^k$;
or 6) when $R^{b1}$ is $CHF_2$, $R^{b2}$ is H,

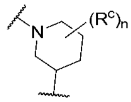

is

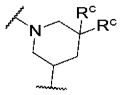,

each $R^c$ is independently selected from H or halogen,

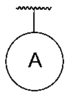

is selected from

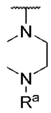, 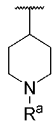, 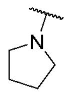,

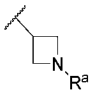, or 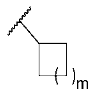,

wherein m is selected from 0, 1, 2, 3, or 4, $X_1$ is CH, $X_3$ is CH, $X_4$ is CH, $X_2$ is $CR^2$, wherein $R^2$ is selected from Cl or $CF_3$, $X_6$ or $X_7$ is selected from CH, CF, or N, $X_5$ is $CR^5$, and $X_8$ is $CR^8$, at least one of $R^5$ and $R^8$ is not H or $C_{1-4}$ alkyl; or 7) when

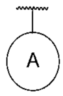

is

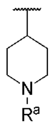,

$R^a$ is not $C_{1-4}$ alkylcarbonyl or optionally substituted $C_{3-6}$ cycloalkyl.

**2.** The compound, or the stereoisomer, tautomer, deuterated compound, solvate, prodrug, metabolite, pharmaceutically acceptable salt, or co-crystal thereof according to claim 1, **characterized in that**

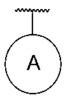

is selected from the following groups optionally substituted with 1 to 4 $R^a$: $C_{3-6}$ monocycloalkyl, $C_{3-6}$ monocycloalkenyl, $C_{5-11}$ spirocycloalkyl, $C_{4-10}$ fused cycloalkyl, $C_{5-10}$ bridged cycloalkyl, 4- to 6-membered monocyclic heterocycloalkyl, 4- to 6-membered monocyclic heterocycloalkenyl, $C_{5-11}$ spiroheterocycloalkyl, $C_{4-10}$ fused heterocycloalkyl, or $C_{5-10}$ bridged heterocycloalkyl;

$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, halogen, OH, CN, $NH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl, or heterocyclyl is optionally substituted with 1 to 4 $R^k$;

or $R^7$ and $R^a$, together with the atoms to which they are attached, form a 4- to 6-membered carbocycle or a 4- to 6-membered heterocycle, wherein the carbocycle or heterocycle is optionally substituted with 1 to 4 $R^k$; and

each $R^k$ is independently selected from deuterium, halogen, OH, =O, CN, $NH_2$, COOH, $CONH_2$, $C_{1-4}$ alkyl, $OC_{1-4}$ alkyl, $SC_{1-4}$ alkyl, $C_{2-4}$ alkenyl, $C_{2-4}$ alkynyl, $NHC_{1-4}$ alkyl, $N(C_{1-4}$ alkyl$)_2$, -O-$C_{3-6}$ carbocyclyl, -O-3- to 6-membered heterocyclyl, -NH-$C_{3-6}$ carbocyclyl, -NH-3- to 6-membered heterocyclyl, -$C_{0-2}$ alkylene-$C_{3-6}$ carbocyclyl, or -$C_{0-2}$ alkylene-3- to 6-membered heterocyclyl, wherein the alkyl, alkylene, alkenyl, alkynyl, carbocyclyl, or heterocyclyl is optionally substituted with 1 to 4 substituents selected from deuterium, halogen, =O, CN, OH, $NH_2$, $C_{1-6}$ alkyl, or $C_{1-6}$ alkoxy.

**3.** The compound, or the stereoisomer, tautomer, deuterated compound, solvate, prodrug, metabolite, pharmaceutically acceptable salt, or co-crystal thereof according to claim 2, **characterized in that**

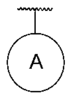

is selected from the following groups optionally substituted with 1 to 4 $R^a$: cyclopropyl, cyclobutyl, cyclopentyl, cyclohexyl, cyclohexenyl, azetidinyl, pyrrolidinyl, piperidinyl, azacyclohexenyl, piperazinyl, morpholinyl, oxetanyl, tetrahydrofuranyl, oxanyl,

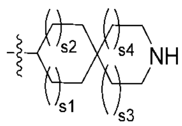, 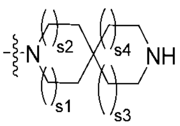, 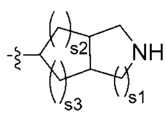,

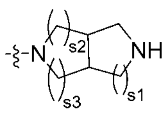, 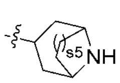, 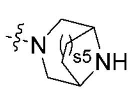, 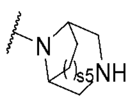, 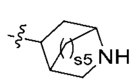, 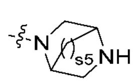,

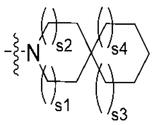, 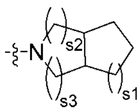, 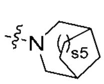, 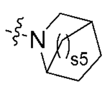, 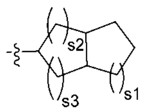, 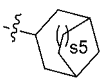, 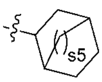,

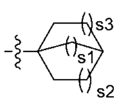, 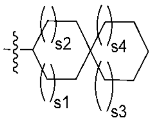, 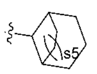, 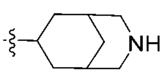, 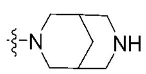, 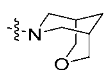,

or

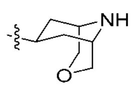;

s1, s2, s3, s4, and s5 are each independently selected from 0 or 1;

$R^1$, $R^2$, $R^3$, $R^4$, $R^5$, $R^6$, $R^7$, $R^8$, $R^a$, $R^{b1}$, $R^{b2}$, and $R^c$ are each independently selected from H, deuterium, F, Cl, Br, I, OH, CN, $NH_2$, $NHCH_3$, $NHCH_3$,

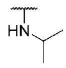,

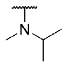,

or the following groups optionally substituted with 1 to 4 $R^k$: methyl, ethyl, propyl, isopropyl, methoxy, ethoxy, isopropoxy, ethenyl, ethynyl, methylthio, cyclopropyl, cyclobutyl, cyclopentyl, cyclohexyl, azetidinyl, oxetanyl, pyrrolidyl, piperidyl, morpholinyl, or phenyl;

or $R^7$ and $R^a$, together with the atoms to which they are attached, form a 5- to 6-membered carbocycle or a 5- to 6-membered heterocycle, wherein the carbocycle or heterocycle is optionally substituted with 1 to 4 $R^k$; and

each $R^k$ is independently selected from deuterium, F, Cl, Br, I, OH, =O, CN, $NH_2$, COOH, $CONH_2$, $NHCH_3$, $N(CH_3)_2$, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl, wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, or phenyl is optionally substituted with 1 to 4 substituents selected from deuterium, F, Cl, Br, I, =O, CN, OH, $NH_2$, $C_{1-4}$ alkyl, or $C_{1-4}$ alkoxy.

4. The compound, or the stereoisomer, tautomer, deuterated compound, solvate, prodrug, metabolite, pharmaceutically acceptable salt, or co-crystal thereof according to claim 3, **characterized in that**

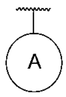

is selected from the following groups optionally substituted with 1 to 4 $R^a$: cyclopropyl, cyclobutyl, cyclopentyl, cyclohexyl,

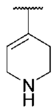, 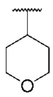, 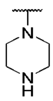, 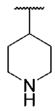, 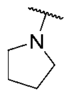,

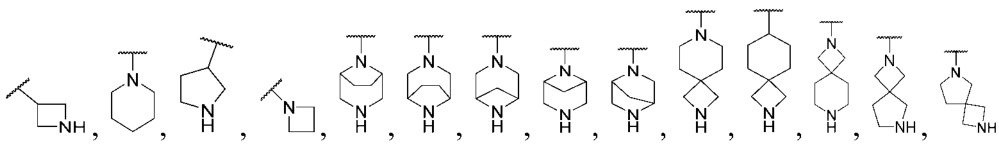 , , , , , , , , , , , , , , , ,

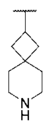, 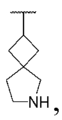, 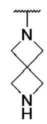, or 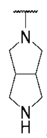;

or $R^7$ and $R^a$, together with the atoms to which they are attached, form 5- to 6-membered heterocyclyl.

5. The compound, or the stereoisomer, tautomer, deuterated compound, solvate, prodrug, metabolite, pharmaceutically acceptable salt, or co-crystal thereof according to claim 4, **characterized in that** the compound represented by general formula (I) is selected from a compound represented by general formula (II-a), (II-b), (II-c), (II-d), (II-e), (II-f), (II-g), (II-h), or (II-i),

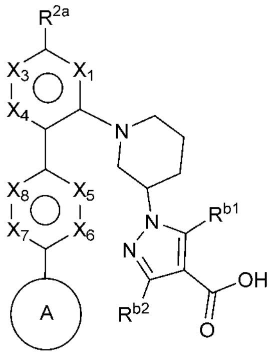

(II-a),

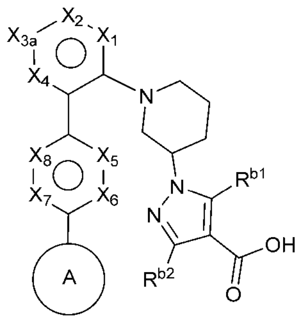

(II-b),

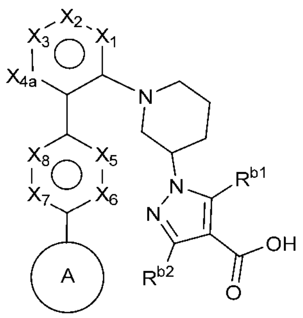

(II-c),

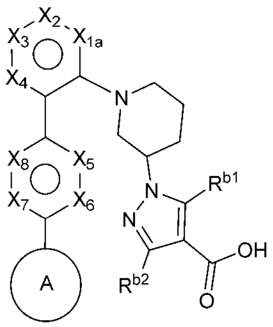

(II-d),

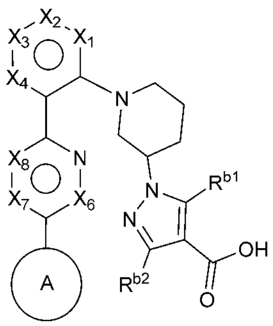

(II-e),

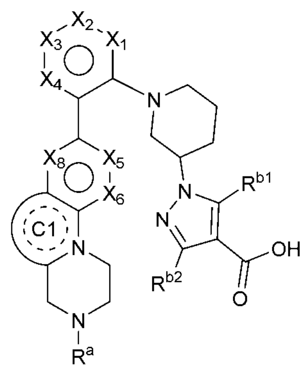

(II-f),

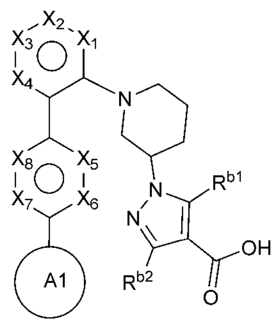

(II-g),

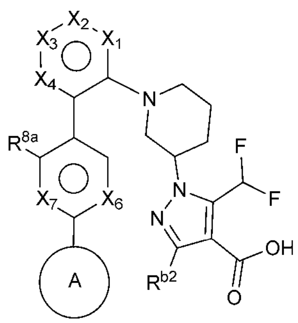

(II-h),

or

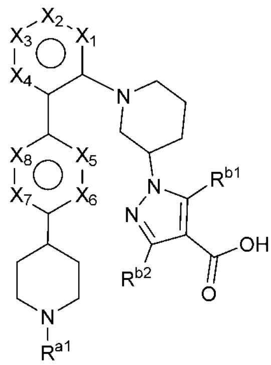

(II-i);

$R^{2a}$ is selected from H, deuterium, F, Br, $CHF_2$, methyl, ethyl, isopropyl, or cyclopropyl;
$x^{1a}$ is selected from N or $CR^{1a}$;
$X^{3a}$ is selected from N or $CR^{3a}$;
$X^{4a}$ is selected from N or $CR^{4a}$;
$R^{1a}$, $R^{3a}$, and $R^{4a}$ are each independently selected from deuterium, F, Cl, Br, $CHF_2$, methyl, ethyl, isopropyl, or cyclopropyl;

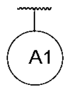

is selected from the following groups optionally substituted with 1 to 4 $R^a$:

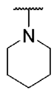, 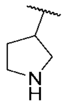, 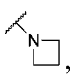, 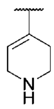, 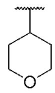, 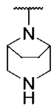, 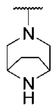, 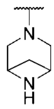, 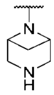, 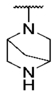, 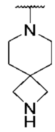, 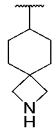, 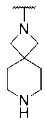, 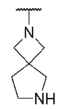,

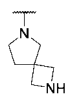, 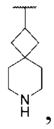, 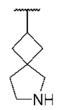, 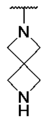, or 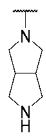;

$R^a$ is selected from H, deuterium, F, Cl, Br, I, OH, CN, $NH_2$, $NHCH_3$, $NHCH_3$,

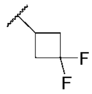, 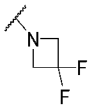, 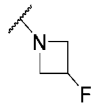, 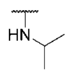, 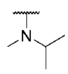, 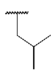, 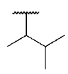,

methyl, methyl, ethyl, propyl, isopropyl, butyl, cyclopropyl, or $-CH_2$-cyclopropyl;
$R^{a1}$ is selected from H, deuterium, F, Cl, Br, I, OH, CN, $NH_2$, $NHCH_3$, $NHCH_3$,

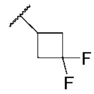, 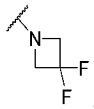, 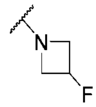, 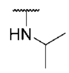, 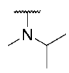, 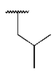, 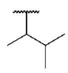,

methyl, methyl, ethyl, propyl, isopropyl, butyl, or cyclopropyl;
$R^{8a}$ is selected from deuterium, F, Br, $CF_3$, $CHF_2$, or cyclopropyl;

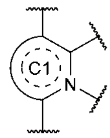 ;

is selected from

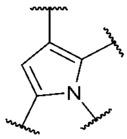 , 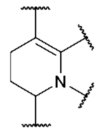 , or 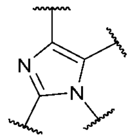 ;

and
the remaining definitions are the same as those in claim 4.

**6.** The compound, or the stereoisomer, tautomer, deuterated compound, solvate, prodrug, metabolite, pharmaceutically acceptable salt, or co-crystal thereof according to claim 1, **characterized in that** the compound has a structure selected from one of those in Table E-1.

**7.** A pharmaceutical composition comprising the compound, or the stereoisomer, tautomer, deuterated compound, solvate, prodrug, metabolite, pharmaceutically acceptable salt, or co-crystal thereof according to any one of claims 1-6, and a pharmaceutically acceptable carrier, **characterized in that** preferably, the pharmaceutical composition comprises 0.01-1500 mg of the compound, or the stereoisomer, tautomer, deuterated compound, solvate, prodrug, metabolite, pharmaceutically acceptable salt, or co-crystal thereof according to any one of claims 1-6.

**8.** Use of the compound, or the stereoisomer, tautomer, deuterated compound, solvate, prodrug, metabolite, pharmaceutically acceptable salt, or co-crystal thereof according to any one of claims 1-6, or the pharmaceutical composition according to claim 7 in the preparation of a medicament for treating a disease associated with sGC.

**9.** The use according to claim 8, **characterized in that** the disease is selected from a cardiovascular disease, a kidney disease, or a respiratory disease, preferably pulmonary arterial hypertension, pulmonary hypertension, chronic obstructive pulmonary disease, chronic kidney disease, or non-proliferative diabetic retinopathy.

**10.** A method for treating a disease in a mammal, comprising administering to the subject a therapeutically effective amount of the compound, or the stereoisomer, deuterated compound, solvate, prodrug, metabolite, pharmaceutically acceptable salt, or co-crystal thereof according to any one of claims 1-6, or the pharmaceutical composition according to claim 7, **characterized in that** the therapeutically effective amount is preferably 0.01-1500 mg, and the disease is preferably a cardiovascular disease, a kidney disease, or a respiratory disease.

**INTERNATIONAL SEARCH REPORT**

International application No.
**PCT/CN2024/121712**

**A. CLASSIFICATION OF SUBJECT MATTER**

C07D401/04(2006.01)i； A61K31/496(2006.01)i； A61P9/10(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, DWPI, CNKI, Caplus (STN), Registry (STN): 海思科制药, 吡唑, 羧酸, 苯, 哌嗪, 鸟苷酸环化酶, 心血管疾病, 结构检索, structure search, pyrazole, carboxylic acid, benzene, piperazine, sGC, cardiovascular disease

**C. DOCUMENTS CONSIDERED TO BE RELEVANT**

| Category* | Citation of document, with indication, where appropriate, of the relevant passages | Relevant to claim No. |
|---|---|---|
| A | CN 114981257 A (BAYER AG) 30 August 2022 (2022-08-30)<br>abstract, claims 1-8, and embodiment 2 | 1-10 |
| A | CN 115175681 A (BAYER AG) 11 October 2022 (2022-10-11)<br>abstract, claims 1-15, and embodiment 2 | 1-10 |
| A | CN 116710439 A (BAYER AG) 05 September 2023 (2023-09-05)<br>entire document | 1-10 |
| A | WO 2022122913 A1 (BAYER AG) 16 June 2022 (2022-06-16)<br>entire document | 1-10 |
| A | WO 2022122916 A1 (BAYER AG) 16 June 2022 (2022-06-16)<br>entire document | 1-10 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 December 2024** | **31 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/121712**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 relates to a method for diagnosing and treating diseases (PCT Rule 39.1(iv)). The following search is carried out on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/121712**

| Patent document cited in search report | Publication date (day/month/year) | Patent family member(s) | Publication date (day/month/year) |
|---|---|---|---|
| CN 114981257 A | 30 August 2022 | CR 20230238 A | 19 July 2023 |
| | | CL 2023001633 A1 | 10 November 2023 |
| | | US 2023265072 A1 | 24 August 2023 |
| | | MX 2023006904 A | 26 June 2023 |
| | | EP 4259615 A1 | 18 October 2023 |
| | | US 2022274958 A1 | 01 September 2022 |
| | | CA 3204491 A1 | 16 June 2022 |
| | | GEP 20247635 B | 25 June 2024 |
| | | AR 124196 A1 | 22 February 2023 |
| | | PE 20231502 A1 | 26 September 2023 |
| | | EP 4011873 A1 | 15 June 2022 |
| | | AU 2021393987 A1 | 22 June 2023 |
| | | DOP 2023000114 A | 09 July 2023 |
| | | WO 2022122910 A1 | 16 June 2022 |
| | | ECSP 23041151 A | 31 July 2023 |
| | | KR 20230118145 A | 10 August 2023 |
| | | CO 2023007334 A2 | 09 June 2023 |
| | | TW 202237588 A | 01 October 2022 |
| | | JP 2023543646 A | 18 October 2023 |
| | | JP 7451700 B2 | 18 March 2024 |
| | | IL 303299 A | 01 July 2023 |
| CN 115175681 A | 11 October 2022 | TW 202237105 A | 01 October 2022 |
| | | US 2022241273 A1 | 04 August 2022 |
| | | MX 2023006902 A | 26 June 2023 |
| | | IL 303297 A | 01 July 2023 |
| | | WO 2022122917 A1 | 16 June 2022 |
| | | JP 2023514928 A | 12 April 2023 |
| | | JP 7458683 B2 | 01 April 2024 |
| | | AU 2021398486 A1 | 22 June 2023 |
| | | AU 2021398486 A9 | 08 February 2024 |
| | | CA 3204596 A1 | 16 June 2022 |
| | | EP 4259140 A1 | 18 October 2023 |
| | | US 2023346777 A1 | 02 November 2023 |
| | | KR 20230118143 A | 10 August 2023 |
| | | JP 2024073585 A | 29 May 2024 |
| | | CL 2023001574 A1 | 17 November 2023 |
| CN 116710439 A | 05 September 2023 | GEP 20247649 B | 25 July 2024 |
| | | AR 124297 A1 | 15 March 2023 |
| | | EP 4259617 A1 | 18 October 2023 |
| | | EP 4259617 B1 | 23 October 2024 |
| | | AU 2021393988 A1 | 22 June 2023 |
| | | AU 2021393988 A9 | 19 September 2024 |
| | | DOP 2023000115 A | 16 July 2023 |
| | | KR 20230118144 A | 10 August 2023 |
| | | JP 2023552583 A | 18 December 2023 |
| | | FI 4259617 T3 | 27 November 2024 |
| | | TW 202237589 A | 01 October 2022 |
| | | WO 2022122914 A1 | 16 June 2022 |
| | | CO 2023007335 A2 | 09 June 2023 |
| | | ECSP 23040828 A | 31 July 2023 |

Form PCT/ISA/210 (patent family annex) (July 2022)

**INTERNATIONAL SEARCH REPORT**
**Information on patent family members**

International application No.
**PCT/CN2024/121712**

| Patent document cited in search report | Publication date (day/month/year) | Patent family member(s) | Publication date (day/month/year) |
|---|---|---|---|
| | | CL 2023001632 A1 | 10 November 2023 |
| | | CA 3204495 A1 | 16 June 2022 |
| | | MX 2023006903 A | 26 June 2023 |
| | | PE 20231501 A1 | 26 September 2023 |
| | | IL 303296 A | 01 July 2023 |
| | | US 2024051935 A1 | 15 February 2024 |
| | | CR 20230242 A | 19 July 2023 |
| WO 2022122913 A1 | 16 June 2022 | CA 3204494 A1 | 16 June 2022 |
| | | US 2024101528 A1 | 28 March 2024 |
| | | EP 4259616 A1 | 18 October 2023 |
| | | EP 4011874 A1 | 15 June 2022 |
| WO 2022122916 A1 | 16 June 2022 | CA 3204595 A1 | 16 June 2022 |
| | | EP 4259618 A1 | 18 October 2023 |
| | | US 2024109864 A1 | 04 April 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022122910 A
- WO 2009050236 A
- WO 2022029617 A
- WO 2021255212 A
- WO 2017181117 A
- WO 2010065275 A


### Non-patent literature cited in the description


- *CHEMICAL ABSTRACTS*, 2781965-82-8
- *CHEMICAL ABSTRACTS*, 60748-47-2
- *CHEMICAL ABSTRACTS*, 2760247-54-7